# EUROPEAN PATENT SPECIFICATION

(11) **EP 1 548 122 B1**
(45) Date of publication and mention of the grant of the patent: **15.08.2007**
(21) Application number: 04028191.7
(22) Date of filing: 26.11.2004
(51) Int. Cl.: C12P 21/02

(54) **Process for producing dipeptides**
Verfahren zur Herstellung von Dipeptiden
Procédé de préparation de dipeptides

(30) Priority: 27.11.2003 JP 2003397104; 25.06.2004 JP 2004189009
(43) Date of publication of application: 29.06.2005
(73) Proprietor: KYOWA HAKKO KOGYO CO., LTD., Chiyoda-ku, Tokyo 100-8185 (JP)
(72) Inventor: Hashimoto, Shin-ichi c/o Technical Research Laboratories, Hofu-shi Yamaguchi 747-8522 (JP); Tabata, Kazuhiko c/o Technical Research Laboratories, Machida-shi Tokyo 194-8533 (JP); Noguchi, Ayako c/o Healthcare Research Laboratories, Tsukuba-shi Ibaraki 305-0841 (JP)
(74) Representative: Vossius & Partner

(56) References cited:
- EP-A- 0 321 722
- WO-A-03/048189

### SEQUENCE LISTING

<110> KYOWA HAKNO KOGYO CO., LTD.
<120> Process for producing dipeptides
<130> 11634
<150> JP2003-397104
   <151> 2003-11-27
<150> JP2004-189009
   <151> 2004-06-25
<160> 37
<170> PatentIn Ver. 2.1
<210> 1
   <211> 472
   <212> PRT
   <213> Bacillus subtilis 168
<400> 1
   Met Glu Arg Lys Thr Val Leu Val Ile Ala Asp Leu Gly Gly Cys Pro
   1 5 10 15
   Pro His Met Phe Tyr Lys Ser Ala Ala Glu Lys Tyr Asn Leu Val Ser
   20 25 30
   Phe Ile Pro Arg Pro Phe Ala Ile Thr Ala Ser His Ala Ala Leu Ile
   35 40 45
   Glu Lys Tyr Ser Val Ala Val Ile Lys Asp Lys Asp Tyr Phe Lys Ser
   50 55 60
   Leu Ala Asp Phe Glu His Pro Asp Ser Ile Tyr Trp Ala His Glu Asp
   65 70 75 80
   His Asn Lys Pro Glu Glu Glu Val Val Glu Gln Ile Val Lys Val Ala
   85 90 95
   Glu Met Phe Gly Ala Asp Ala Ile Thr Thr Asn Asn Glu Leu Phe Ile
   100 105 110
   Ala Pro Met Ala Lys Ala Cys Glu Arg Leu Gly Leu Arg Gly Ala Gly
   115 120 125
   Val Gln Ala Ala Glu Asn Ala Arg Asp Lys Asn Lys Met Arg Asp Ala
   130 135 140
   Phe Asn Lys Ala Gly Val Lys Ser Ile Lys Asn Lys Arg Val Thr Thr
   145 150 155 160
   Leu Glu Asp Phe Arg Ala Ala Leu Glu Glu Ile Gly Thr Pro Leu Ile
   165 170 175
   Leu Lys Pro Thr Tyr Leu Ala Ser Ser Ile Gly Val Thr Leu Ile Thr
   180 185 190
   Asp Thr Glu Thr Ala Glu Asp Glu Phe Asn Arg Val Asn Asp Tyr Leu
   195 200 205
   Lys Ser Ile Asn Val Pro Lys Ala Val Thr Phe Glu Ala Pro Phe Ile
   210 215 220
   Ala Glu Glu Phe Leu Gln Gly Glu Tyr Gly Asp Trp Tyr Gln Thr Glu
   225 230 235 240
   Gly Tyr Ser Asp Tyr Ile Ser Ile Glu Gly Ile Met Ala Asp Gly Glu
   245 250 255
   Tyr Phe Pro Ile Ala Ile His Asp Lys Thr Pro Gln Ile Gly Phe Thr
   260 265 270
   Glu Thr Ser His Ile Thr Pro Ser Ile Leu Asp Glu Glu Ala Lys Lys
   275 280 285
   Lys Ile Val Glu Ala Ala Lys Lys Ala Asn Glu Gly Leu Gly Leu Gln
   290 295 300
   Asn Cys Ala Thr His Thr Glu Ile Lys Leu Met Lys Asn Arg Glu Pro
   305 310 315 320
   Gly Leu Ile Glu Ser Ala Ala Arg Phe Ala Gly Trp Asn Met Ile Pro
   325 330 335
   Asn Ile Lys Lys Val Phe Gly Leu Asp Met Ala Gln Leu Leu Leu Asp
   340 345 350
   Val Leu Cys Phe Gly Lys Asp Ala Asp Leu Pro Asp Gly Leu Leu Asp
   355 360 365
   Gln Glu Pro Tyr Tyr Val Ala Asp Cys His Leu Tyr Pro Gln His Phe
   370 375 380
   Lys Gln Asn Gly Gln Ile Pro Glu Thr Ala Glu Asp Leu Val Ile Glu
   385 390 395 400
   Ala Ile Asp Ile Pro Asp Gly Leu Leu Lys Gly Asp Thr Glu Ile Val
   405 410 415
   Ser Phe Ser Ala Ala Ala Pro Gly Thr Ser Val Asp Leu Thr Leu Phe
   420 425 430
   Glu Ala Phe Asn Ser Ile Ala Ala Phe Glu Leu Lys Gly Ser Asn Ser
   435 440 445
   Gln Asp Val Ala Glu Ser Ile Arg Gln Ile Gln Gln His Ala Lys Leu
   450 455 460
   Thr Ala Lys Tyr Val Leu Pro Val
   465 470
<210> 2
   <211> 472
   <212> PRT
   <213> Bacillus subtilis ATCC6633
<210> 3
   <211> 472
   <212> PRT
   <213> Bacillus subtilis IAM1213
<400> 3
   Met Glu Arg Lys Thr Val Leu Val Ile Ala Asp Leu Gly Gly Cys Pro
   1 5 10 15
   Pro His Met Phe Tyr Lys Ser Ala Ala Glu Lys Tyr Asn Leu Val Ser
   20 25 30
   Phe Ile Pro Arg Pro Phe Ala Ile Thr Ala Ser His Ala Ala Leu Ile
   35 40 45
   Glu Lys Tyr Ser Val Ala Val Ile Lys Asp Lys Asp Tyr Phe Lys Ser
   50 55 60
   Leu Ala Asp Phe Glu His Pro Asp Ser Ile Tyr Trp Ala His Glu Asp
   65 70 75 80
   His Asn Lys Pro Glu Glu Glu Val Val Glu Gln Ile Val Lys Val Ala
   85 90 95
   Glu Met Phe Gly Ala Asp Ala Ile Thr Thr Asn Asn Glu Leu Phe Ile
   100 105 110
   Ala Pro Met Ala Lys Ala Cys Glu Arg Leu Gly Leu Arg Gly Ala Gly
   115 120 125
   Val Gln Ala Ala Glu Asn Ala Arg Asp Lys Asn Lys Met Arg Asp Ala
   130 135 140
   Phe Asn Lys Ala Gly Val Lys Ser Ile Lys Asn Lys Arg Val Thr Thr
   145 150 155 160
   Leu Glu Asp Phe Arg Ala Ala Leu Glu Glu Ile Gly Thr Pro Leu Ile
   165 170 175
   Leu Lys Pro Thr Tyr Leu Ala Ser Ser Ile Gly Val Thr Leu Ile Thr
   180 185 190
   Asp Thr Glu Thr Ala Glu Asp Glu Phe Asn Arg Val Asn Asp Tyr Leu
   195 200 205
   Lys Ser Ile Asn Val Pro Lys Ala Val Thr Phe Glu Ala Pro Phe Ile
   210 215 220
   Ala Glu Glu Phe Leu Gln Gly Glu Tyr Gly Asp Trp Tyr Gln Thr Glu
   225 230 235 240
   Gly Tyr Ser Asp Tyr Ile Ser Ile Glu Gly Ile Met Ala Asp Gly Glu
   245 250 255
   Tyr Phe Pro Ile Ala Ile His Asp Lys Thr Pro Gln Ile Gly Phe Thr
   260 265 270
   Glu Thr Ser His Ile Thr Pro Ser Ile Leu Asp Glu Glu Ala Lys Lys
   275 280 285
   Lys Ile Val Glu Ala Ala Lys Lys Ala Asn Glu Gly Leu Gly Leu Gln
   290 295 300
   Asn Cys Ala Thr His Thr Glu Ile Lys Leu Met Lys Asn Arg Glu Pro
   305 310 315 320
   Gly Leu Ile Glu Ser Ala Ala Arg Phe Ala Gly Trp Asn Met Ile Pro
   325 330 335
   Asn Ile Lys Lys Val Phe Gly Leu Asp Met Ala Gln Leu Leu Leu Asp
   340 345 350
   Val Leu Cys Phe Gly Lys Asp Ala Asp Leu Pro Asp Gly Leu Leu Asp
   355 360 365
   Gln Glu Pro Tyr Tyr Val Ala Asp Cys His Leu Tyr Pro Gln His Phe
   370 375 380
   Lys Gln Asn Gly Gln Ile Pro Glu Thr Ala Glu Asp Leu Val Ile Glu
   385 390 395 400
   Ala Ile Asp Leu Pro Asp Gly Leu Leu Lys Gly Asp Thr Glu Ile Val
   405 410 415
   Ser Phe Ser Ala Ala Ala Pro Gly Thr Ser Val Asp Leu Thr Leu Phe
   420 425 430
   Glu Ala Phe Asn Ser Ile Ala Ala Phe Glu Leu Lys Gly Ser Asn Ser
   435 440 445
   Gln Asp Val Ala Glu Ser Ile Arg Gln Ile Gln Gln His Ala Lys Leu
   450 455 460
   Thr Ala Lys Tyr Val Leu Pro Val
   465 470
<210> 4
   <211> 472
   <212> PRT
   <213> Bacillus subtilis IAM1107
<400> 4
   Met Glu Arg Lys Thr Val Leu Val Ile Ala Asp Leu Gly Gly Cys Pro
   1 5 10 15
   Pro His Met Phe Tyr Lys Ser Ala Ala Glu Lys Tyr Asn Leu Val Ser
   20 25 30
   Phe Ile Pro Arg Pro Phe Ala Ile Thr Ala Ser His Ala Ala Leu Ile
   35 40 45
   Glu Lys Tyr Ser Val Ala Val Val Lys Asp Lys Asp Tyr Phe Lys Ser
   50 55 60
   Leu Ala Asp Phe Glu His Pro Asp Ser Ile Tyr Trp Ala His Glu Asp
   65 70 75 80
   His Asn Lys Pro Glu Glu Glu Val Val Glu Gln Ile Val Lys Val Ala
   85 90 95
   Glu Met Phe Gly Ala Asp Ala Ile Thr Thr Asn Asn Glu Leu Phe Ile
   100 105 110
   Ala Pro Met Ala Lys Ala Cys Glu Arg Leu Gly Leu Arg Gly Ala Gly
   115 120 125
   Val Gln Ala Ala Glu Asn Ala Arg Asp Lys Asn Lys Met Arg Asp Ala
   130 135 140
   Phe Asn Lys Ala Gly Val Lys Ser Ile Lys Asn Lys Arg Val Thr Thr
   145 150 155 160
   Leu Glu Asp Phe Arg Ala Ala Leu Glu Glu Ile Gly Thr Pro Leu Ile
   165 170 175
   Leu Lys Pro Thr Tyr Leu Ala Ser Ser Ile Gly Val Thr Leu Ile Thr
   180 185 190
   Asp Thr Glu Thr Ala Glu Asp Glu Phe Asn Arg Val Asn Asp Tyr Leu
   195 200 205
   Lys Ser Ile Asn Val Pro Lys Ala Val Thr Phe Glu Ala Pro Phe Ile
   210 215 220
   Ala Glu Glu Phe Leu Gln Gly Glu Tyr Gly Asp Trp Tyr Gln Thr Glu
   225 230 235 240
   Gly Tyr Ser Asp Tyr Ile Ser Ile Glu Gly Ile Met Ala Asp Gly Glu
   245 250 255
   Tyr Phe Pro Ile Ala Ile His Asp Lys Thr Pro Gln Ile Gly Phe Thr
   260 265 270
   Glu Thr Ser His Ile Thr Pro Ser Ile Leu Asp Glu Glu Ala Lys Lys
   275 280 285
   Lys Ile Val Glu Ala Ala Lys Lys Ala Asn Glu Gly Leu Gly Leu Gln
   290 295 300
   Asn Cys Ala Thr His Thr Glu Val Lys Leu Met Lys Asn Arg Glu Pro
   305 310 315 320
   Gly Leu Ile Glu Ser Ala Ala Arg Phe Ala Gly Trp Asn Met Ile Pro
   325 330 335
   Asn Ile Lys Lys Val Phe Gly Leu Asp Met Ala Gln Leu Leu Leu Asp
   340 345 350
   Val Leu Cys Phe Gly Lys Asp Ala Asp Leu Pro Asp Gly Leu Leu Asp
   355 360 365
   Gln Glu Pro Tyr Tyr Val Ala Asp Cys His Leu Tyr Pro Gln His Phe
   370 375 380
   Lys Gln Asn Gly Gln Ile Pro Glu Thr Ala Glu Asp Leu Val Ile Glu
   385 390 395 400
   Ala Ile Asp Ile Pro Asp Gly Leu Leu Lys Gly Asp Thr Glu Ile Phe
   405 410 415
   Ser Phe Ser Ala Ala Ala Pro Gly Thr Ser Val Asp Leu Thr Leu Phe
   420 425 430
   Glu Ala Phe Asn Ser Ile Ala Ala Phe Glu Leu Lys Gly Ser Asn Ser
   435 440 445
   Gln Asp Val Ala Glu Ser Ile Arg Gln Ile Gln Gln His Ala Lys Leu
   450 455 460
   Thr Ala Lys Tyr Val Leu Pro Val
   465 470
<210> 5
   <211> 472
   <212> PRT
   <213> Bacillus subtilis IAM1214
<400> 5
   Met Glu Arg Lys Thr Val Leu Val Ile Ala Asp Leu Gly Gly Cys Pro
   1 5 10 15
   Pro His Met Phe Tyr Lys Ser Ala Ala Glu Lys Tyr Asn Leu Val Ser
   20 25 30
   Phe Ile Pro Arg Pro Phe Ala Ile Thr Ala Ser His Ala Ala Leu Ile
   35 40 45
   Glu Lys Tyr Ser Val Ala Val Ile Lys Asp Lys Asp Tyr Phe Gln Ser
   50 55 60
   Leu Ala Asp Phe Glu His Pro Asp Ser Ile Tyr Trp Ala His Glu Asp
   65 70 75 80
   His Asp Lys Pro Glu Glu Glu Val Val Glu Gln Ile Val Lys Val Ala
   85 90 95
   Gln Met Phe Glu Ala Asp Ala Ile Thr Thr Asn Asn Glu Leu Phe Ile
   100 105 110
   Ala Pro Met Ala Lys Ala Cys Glu Arg Leu Gly Leu Arg Gly Ala Gly
   115 120 125
   Val Gln Ala Ala Glu Asn Ala Arg Asp Lys Asn Lys Met Arg Asp Ala
   130 135 140
   Phe Asn Lys Ala Gly Val Lys Ser Ile Lys Asn Lys Arg Val Thr Thr
   145 150 155 160
   Leu Glu Asp Phe Arg Ala Ala Leu Glu Glu Ile Gly Thr Pro Leu Ile
   165 170 175
   Leu Lys Pro Thr Tyr Leu Ala Ser Ser Ile Gly Val Thr Leu Ile Thr
   180 185 190
   Asp Thr Glu Thr Ala Glu Asp Glu Phe Asn Arg Val Asn Asp Tyr Leu
   195 200 205
   Lys Ser Ile Asn Val Pro Lys Ala Val Thr Phe Glu Ala Pro Phe Ile
   210 215 220
   Ala Glu Glu Phe Leu Gln Gly Glu Tyr Gly Asp Trp Tyr Gln Thr Glu
   225 230 235 240
   Gly Tyr Ser Asp Tyr Ile Ser Ile Glu Gly Ile Met Ala Asp Gly Glu
   245 250 255
   Tyr Phe Pro Ile Ala Ile His Asp Lys Thr Pro Gln Ile Gly Phe Thr
   260 265 270
   Glu Thr Ser His Ile Thr Pro Ser Ile Leu Asp Glu Glu Ala Lys Lys
   275 280 285
   Lys Ile Val Glu Ala Ala Lys Lys Ala Asn Glu Gly Leu Gly Leu Gln
   290 295 300
   Asn Cys Ala Thr His Thr Glu Ile Lys Leu Met Lys Asn Arg Glu Pro
   305 310 315 320
   Gly Leu Ile Glu Ser Ala Ala Arg Phe Ala Gly Trp Asn Met Ile Pro
   325 330 335
   Asn Ile Lys Lys Val Phe Gly Leu Asp Met Ala Gln Leu Leu Leu Asp
   340 345 350
   Val Leu Cys Phe Gly Lys Asp Ala Asp Leu Pro Asp Gly Leu Leu Asp
   355 360 365
   Gln Glu Pro Tyr Tyr Val Ala Asp Cys His Leu Tyr Pro Gln His Phe
   370 375 380
   Lys Gln Asn Gly Gln Ile Pro Glu Thr Ala Glu Asp Leu Val Ile Glu
   385 390 395 400
   Ala Ile Asp Ile Pro Asp Gly Leu Leu Lys Gly Asp Thr Glu Ile Val
   405 410 415
   Thr Phe Ser Ala Ala Ala Pro Gly Thr Ser Val Asp Leu Thr Leu Phe
   420 425 430
   Glu Ala Phe Asn Ser Ile Ala Ala Phe Glu Leu Lys Gly Ser Asn Ser
   435 440 445
   Gln Asp Val Ala Glu Ser Ile Arg Gln Ile Gln Gln His Ala Lys Leu
   450 455 460
   Thr Ala Lys Tyr Val Leu Pro Val
   465 470
<210> 6
   <211> 472
   <212> PRT
   <213> Bacillus subtilis ATCC21555
<400> 6
   Met Glu Arg Lys Thr Val Leu Val Ile Ala Asp Leu Gly Gly Cys Pro
   1 5 10 15
   Pro His Met Phe Tyr Lys Ser Ala Ala Glu Lys Tyr Asn Leu Val Ser
   20 25 30
   Phe Ile Pro Arg Pro Phe Ala Ile Thr Ala Ser His Ala Ala Leu Ile
   35 40 45
   Glu Lys Tyr Ser Ile Ala Val Ile Lys Asp Lys Asp Tyr Phe Lys Ser
   50 55 60
   Leu Ala Asp Phe Glu His Pro Asp Ser Ile Tyr Trp Ala His Glu Asp
   65 70 75 80
   His Asp Lys Pro Glu Glu Glu Val Val Glu Glu Ile Val Lys Val Ala
   85 90 95
   Asp Met Phe Gly Val Asp Ala Ile Thr Thr Asn Asn Glu Leu Phe Ile
   100 105 110
   Ala Pro Met Ala Lys Ala Cys Lys Arg Leu Gly Leu Arg Gly Ala Gly
   115 120 125
   Val Gln Ala Ala Glu Asn Ala Arg Asp Lys Asn Lys Met Arg Ala Ala
   130 135 140
   Phe Asn Arg Ala Gly Val Lys Ser Ile Lys Asn Lys Arg Val Thr Thr
   145 150 155 160
   Leu Glu Asp Phe Arg Ala Ala Leu Gln Glu Ile Gly Thr Pro Leu Ile
   165 170 175
   Leu Lys Pro Thr Tyr Leu Ala Ser Ser Ile Gly Val Thr Leu Ile Lys
   180 185 190
   Glu Met Glu Thr Ala Glu Ala Glu Phe Asn Arg Val Asn Glu Tyr Leu
   195 200 205
   Lys Ser Ile Asn Val Pro Lys Ala Val Thr Phe Glu Ala Pro Phe Ile
   210 215 220
   Ala Glu Glu Phe Leu Gln Gly Glu Tyr Asp Asp Trp Tyr Glu Thr Ser
   225 230 235 240
   Gly Tyr Ser Asp Tyr Ile Ser Ile Glu Gly Ile Met Ala Asp Gly Glu
   245 250 255
   Tyr Phe Pro Val Ala Ile His Asp Lys Thr Pro Gln Ile Gly Phe Thr
   260 265 270
   Glu Thr Ala His Ile Thr Pro Ser Ile Leu Asp Asp Asp Ala Lys Arg
   275 280 285
   Lys Ile Val Glu Ala Ala Lys Lys Ala Asn Glu Gly Leu Gly Leu Glu
   290 295 300
   Asn Cys Ala Thr His Thr Glu Ile Lys Leu Met Lys Asn Arg Glu Ala
   305 310 315 320
   Gly Leu Ile Glu Ser Ala Ala Arg Phe Ala Gly Trp Asn Met Ile Pro
   325 330 335
   Asn Ile Lys Lys Val Phe Gly Val Asp Met Ala Gln Leu Leu Leu Asp
   340 345 350
   Val Leu Cys Tyr Gly Lys Glu Ala Asp Leu Pro Lys Gly Leu Leu Glu
   355 360 365
   Gln Glu Pro Cys Tyr Val Ala Asp Cys His Leu Tyr Pro Gln His Phe
   370 375 380
   Lys Glu Asn Gly Gln Leu Pro Glu Thr Val Val Asp Phe Val Ile Glu
   385 390 395 400
   Ser Ile Glu Ile Pro Asp Gly Val Leu Lys Gly Asp Thr Glu Leu Val
   405 410 415
   Ser Phe Ser Ala Ala Glu Ala Gly Thr Ser Val Asp Leu Arg Leu Phe
   420 425 430
   Glu Ala Phe Asn Ser Ile Ala Ala Phe Glu Leu Lys Gly Ser Asn Ser
   435 440 445
   Asn Asp Val Ala Glu Ser Ile Lys Gln Ile Gln Gln Gln Ala Lys Leu
   450 455 460
   Thr Ala Lys Tyr Ala Leu Ser Val
   465 470
<210> 7
   <211> 472
   <212> PRT
   <213> Bacillus amyloliquefaciens IFO3022
<400> 7
   Met Glu Arg Lys Thr Val Leu Val Ile Ala Asp Leu Gly Gly Cys Pro
   1 5 10 15
   Pro His Met Phe Tyr Lys Ser Ala Ala Glu Lys Tyr Asn Leu Val Ser
   20 25 30
   Phe Ile Pro Arg Pro Phe Ala Ile Thr Ala Ser His Ala Ala Leu Ile
   35 40 45
   Glu Lys Tyr Ser Val Ala Val Ile Lys Asp Lys Asp Tyr Phe Lys Ser
   50 55 60
   Leu Ala Asp Phe Glu His Pro Asp Ser Ile Tyr Trp Ala His Glu Asp
   65 70 75 80
   His Asp Lys Pro Glu Glu Glu Val Val Glu Glu Ile Val Lys Val Ala
   85 90 95
   Gly Met Phe Ala Val Asp Ala Ile Thr Thr Asn Asn Glu Leu Phe Ile
   100 105 110
   Ala Pro Met Ala Lys Ala Cys Glu Arg Leu Gly Leu Arg Gly Ala Gly
   115 120 125
   Val Gln Ala Ala Glu Asn Ala Arg Asp Lys Asn Lys Met Arg Ala Ala
   130 135 140
   Phe Asn Arg Ala Gly Val Lys Ser Ile Lys Asn Arg Arg Val Thr Thr
   145 150 155 160
   Leu Glu Asp Phe Arg Ala Ala Leu Gln Glu Ile Gly Thr Pro Leu Ile
   165 170 175
   Leu Lys Pro Thr Tyr Leu Ala Ser Ser Ile Gly Val Thr Leu Ile Lys
   180 185 190
   Glu Arg Glu Thr Ala Glu Ala Glu Phe Asn Arg Val Asn Glu Tyr Leu
   195 200 205
   Lys Ser Ile Asn Val Pro Lys Ala Val Thr Phe Glu Ala Pro Phe Ile
   210 215 220
   Ala Glu Glu Phe Leu Gln Gly Glu Tyr Asp Asp Trp Tyr Glu Thr Ser
   225 230 235 240
   Gly Tyr Ser Asp Tyr Ile Ser Ile Glu Gly Ile Met Ala Asp Gly Glu
   245 250 255
   Tyr Phe Pro Val Ala Ile His Asp Lys Thr Pro Gln Ile Gly Phe Thr
   260 265 270
   Glu Thr Ser His Ile Thr Pro Ser Ile Leu Asp Asp Asp Ala Lys Arg
   275 280 285
   Lys Ile Val Glu Ala Ala Lys Lys Ala Asn Glu Gly Leu Gly Leu Glu
   290 295 300
   Asn Cys Ala Thr His Thr Glu Ile Lys Leu Met Lys Asn Arg Glu Ala
   305 310 315 320
   Gly Leu Ile Glu Ser Ala Ala Arg Phe Ala Gly Trp Asn Met Ile Pro
   325 330 335
   Asn Ile Lys Lys Val Phe Gly Val Asp Met Ala Gln Leu Leu Leu Asp
   340 345 350
   Val Leu Cys Phe Gly Lys Glu Ala Asp Leu Pro Lys Gly Leu Leu Glu
   355 360 365
   Gln Glu Pro Cys Tyr Val Ala Asp Cys His Leu Tyr Pro Gln His Phe
   370 375 380
   Lys Glu Asn Gly Gln Leu Pro Glu Thr Ala Val Asp Phe Val Ile Glu
   385 390 395 400
   Ser Ile Asp Ile Pro Asp Gly Val Leu Lys Gly Asp Thr Glu Ile Val
   405 410 415
   Ser Phe Ser Ala Ala Glu Ala Gly Thr Ser Val Asp Leu Arg Leu Phe
   420 425 430
   Glu Ala Phe Asn Ser Ile Ala Ala Phe Glu Leu Lys Gly Ser Asn Ser
   435 440 445
   Gly Asp Val Ala Glu Ser Ile Lys Gln Ile Gln Gln Gln Ala Lys Leu
   450 455 460
   Thr Ala Lys Tyr Ala Leu Pro Val
   465 470
<210> 8
   <211> 1416
   <212> DNA
   <213> Bacillus subtilis 168
<400> 8
   atg gag aga aaa aca gta ttg gtc atc gct gat ctt gga ggc tgc ccg 48
   Met Glu Arg Lys Thr Val Leu Val Ile Ala Asp Leu Gly Gly Cys Pro
   1 5 10 15
   ccg cac atg ttt tat aaa agc gct gct gaa aaa tat aac ctg gtc agc 96
   Pro His Met Phe Tyr Lys Ser Ala Ala Glu Lys Tyr Asn Leu Val Ser
   20 25 30
   ttt att cca aga cct ttt gca att aca gcc tcc cat gca gca ttg att 144
   Phe Ile Pro Arg Pro Phe Ala Ile Thr Ala Ser His Ala Ala Leu Ile
   35 40 45
   gaa aaa tac tcg gtc gcg gtc ata aaa gat aaa gac tat ttt aag agt   192
   Glu Lys Tyr Ser Val Ala Val Ile Lys Asp Lys Asp Tyr Phe Lys Ser
        50                  55                  60
   tta gct gat ttt gaa cac cct gat tcc att tat tgg gcg cat gaa gat   240
   Leu Ala Asp Phe Glu His Pro Asp Ser Ile Tyr Trp Ala His Glu Asp
    65                  70                  75                  80
   cat aac aag cct gag gaa gag gtc gtc gag caa atc gtc aag gtt gcc   288
   His Asn Lys Pro Glu Glu Glu Val Val Glu Gln Ile Val Lys Val Ala
                    85                  90                  95
   gaa atg ttt ggg gcg gat gcc atc aca aca aac aat gaa tta ttc att   336
   Glu Met Phe Gly Ala Asp Ala Ile Thr Thr Asn Asn Glu Leu Phe Ile
               100                 105                 110
   gct ccg atg gcg aaa gcc tgt gaa cgt ctg ggc ttg aga ggt gcc ggc   384
   Ala Pro Met Ala Lys Ala Cys Glu Arg Leu Gly Leu Arg Gly Ala Gly
           115                 120                 125
   gtg cag gca gcc gaa aat gcc aga gat aaa aat aaa atg agg gac gct   432
   Val Gln Ala Ala Glu Asn Ala Arg Asp Lys Asn Lys Met Arg Asp Ala
       130                 135                 140
   ttt aat aag gcc gga gtc aaa tcg atc aaa aac aaa cga gtc aca act   480
   Phe Asn Lys Ala Gly Val Lys Ser Ile Lys Asn Lys Arg Val Thr Thr
   145                 150                 155                 160
   ctt gaa gat ttc cgt gct gct ctt gaa gag atc ggc aca cct ctt atc   528
   Leu Glu Asp Phe Arg Ala Ala Leu Glu Glu Ile Gly Thr Pro Leu Ile
                   165                 170                 175
   tta aag cct aca tac tta gcg agt tct atc ggt gta acg ctg att acg   576
   Leu Lys Pro Thr Tyr Leu Ala Ser Ser Ile Gly Val Thr Leu Ile Thr
               180                 185                 190
   gac act gag acg gca gaa gat gaa ttt aac aga gtc aat gac tat ctg   624
   Asp Thr Glu Thr Ala Glu Asp Glu Phe Asn Arg Val Asn Asp Tyr Leu
           195                 200                 205
   aaa tca att aac gtg cca aag gcg gtt acg ttt gaa gcg ccg ttt atc   672
   Lys Ser Ile Asn Val Pro Lys Ala Val Thr Phe Glu Ala Pro Phe Ile
               210                     215                     220
   gct gaa gaa ttt tta cag ggt gag tac gga gac tgg tat caa aca gaa      720
   Ala Glu Glu Phe Leu Gln Gly Glu Tyr Gly Asp Trp Tyr Gln Thr Glu
   225                 230                 235                 240
   ggg tac tcc gac tat atc agt ata gaa ggc atc atg gct gac ggt gag      768
   Gly Tyr Ser Asp Tyr Ile Ser Ile Glu Gly Ile Met Ala Asp Gly Glu
                   245                 250                 255
   tat ttc ccg atc gcc att cat gat aaa acg ccg caa atc ggg ttt aca      816
   Tyr Phe Pro Ile Ala Ile His Asp Lys Thr Pro Gln Ile Gly Phe Thr
               260                 265                 270
   gag aca tcc cac att acg ccg tcc att ctg gat gaa gag gca aaa aag      864
   Glu Thr Ser His Ile Thr Pro Ser Ile Leu Asp Glu Glu Ala Lys Lys
           275                 280                 285
   aaa att gtc gaa gct gcc aaa aag gca aat gaa ggg ctt gga ctg caa      912
   Lys Ile Val Glu Ala Ala Lys Lys Ala Asn Glu Gly Leu Gly Leu Gln
       290                 295                 300
   aat tgc gca aca cat aca gag atc aag cta atg aaa aac aga gaa ccg      960
   Asn Cys Ala Thr His Thr Glu Ile Lys Leu Met Lys Asn Arg Glu Pro
   305                 310                 315                 320
   ggt tta ata gag tcg gca gcc aga ttt gcc ggc tgg aat atg atc ccc      1008
   Gly Leu Ile Glu Ser Ala Ala Arg Phe Ala Gly Trp Asn Met Ile Pro
                   325                 330                 335
   aat att aaa aag gtc ttt ggc ctt gat atg gcg caa tta tta tta gat      1056
   Asn Ile Lys Lys Val Phe Gly Leu Asp Met Ala Gln Leu Leu Leu Asp
               340                 345                 350
   gtc ctc tgt ttc gga aaa gac gcc gat ctg ccg gac gga tta ttg gat      1104
   Val Leu Cys Phe Gly Lys Asp Ala Asp Leu Pro Asp Gly Leu Leu Asp
           355                 360                 365
   caa gag cct tat tat gtt gcc gac tgc cat ttg tac ccg cag cat ttc      1152
   Gln Glu Pro Tyr Tyr Val Ala Asp Cys His Leu Tyr Pro Gln His Phe
       370                 375                 380
   aaa caa aat ggc caa att cct gaa act gct gag gat ttg gtc att gaa 1200
   Lys Gln Asn Gly Gln Ile Pro Glu Thr Ala Glu Asp Leu Val Ile Glu
   385 390 395 400
   gcg atc gat att ccg gac ggg ctt tta aaa ggg gat act gaa atc gtt 1248
   Ala Ile Asp Ile Pro Asp Gly Leu Leu Lys Gly Asp Thr Glu Ile Val
   405 410 415
   tct ttt tcg gcc gca gca cca ggc act tca gtt gat ttg aca ttg ttt 1296
   Ser Phe Ser Ala Ala Ala Pro Gly Thr Ser Val Asp Leu Thr Leu Phe
   420 425 430
   gaa gct ttc aat tcc att gct gca ttt gaa ctg aaa ggc agt aat tca 1344
   Glu Ala Phe Asn Ser Ile Ala Ala Phe Glu Leu Lys Gly Ser Asn Ser
   435 440 445
   cag gat gtg gct gaa tca atc aga caa att cag cag cat gcg aag ctg 1392
   Gln Asp Val Ala Glu Ser Ile Arg Gln Ile Gln Gln His Ala Lys Leu
   450 455 460
   acg gca aag tat gtg ctg cca gta 1416
   Thr Ala Lys Tyr Val Leu Pro Val
   465 470
<210> 9
   <211> 1416
   <212> DNA
   <213> Bacillus subtilis ATCC6633
<400> 9
   atg gag aga aaa aca gta ttg gtc atc gct gat ctt gga ggc tgc ccg 48
   Met Glu Arg Lys Thr Val Leu Val Ile Ala Asp Leu Gly Gly Cys Pro
   1 5 10 15
   ccg cac atg ttt tat aaa agc gct gct gaa aaa tat aac ctg gtt agc 96
   Pro His Met Phe Tyr Lys Ser Ala Ala Glu Lys Tyr Asn Leu Val Ser
   20 25 30
   ttt att ccg aga cct ttt gca ata aca gcc tcc cat gca gca ctg att 144
   Phe Ile Pro Arg Pro Phe Ala Ile Thr Ala Ser His Ala Ala Leu Ile
   35 40 45
   gaa aaa tac tcg gtc gcg gtc ata aaa gat aaa gac tat ttt cag agc    192
   Glu Lys Tyr Ser Val Ala Val Ile Lys Asp Lys Asp Tyr Phe Gln Ser
        50                  55                  60
   tta gct gat ttt gag cat ccc gat tca att tat tgg gcg cat gag gat    240
   Leu Ala Asp Phe Glu His Pro Asp Ser Ile Tyr Trp Ala His Glu Asp
   65                  70                  75                  80
   cat gac aag cct gaa gaa gag gtt gtc gag caa atc gtc aag gtt gcc    288
   His Asp Lys Pro Glu Glu Glu Val Val Glu Gln Ile Val Lys Val Ala
                   85                  90                  95
   caa atg ttt gag gcg gac gcc atc aca aca aac aat gaa tta ttc att    336
   Gln Met Phe Glu Ala Asp Ala Ile Thr Thr Asn Asn Glu Leu Phe Ile
               100                 105                 110
   gcc ccg atg gcg aaa gcc tgt gaa cgc ctt ggc ctg agg ggc gcc gga    384
   Ala Pro Met Ala Lys Ala Cys Glu Arg Leu Gly Leu Arg Gly Ala Gly
           115                 120                 125
   gtg cag gca gcg gaa aat gcc aga gat aaa aat aaa atg agg gac gct    432
   Val Gln Ala Ala Glu Asn Ala Arg Asp Lys Asn Lys Met Arg Asp Ala
       130                 135                 140
   ttt aat aag gcg gga gtc aaa tcg atc aaa aac aaa cga gtc aca act    480
   Phe Asn Lys Ala Gly Val Lys Ser Ile Lys Asn Lys Arg Val Thr Thr
   145                 150                 155                 160
   ctt gag gat ttt cgt gct gca ctt gaa gag atc ggc aca cct cta atc    528
   Leu Glu Asp Phe Arg Ala Ala Leu Glu Glu Ile Gly Thr Pro Leu Ile
                   165                 170                 175
   tta aag cct aca tac tta gcg agt tca atc ggc gta acg ctg att acc    576
   Leu Lys Pro Thr Tyr Leu Ala Ser Ser Ile Gly Val Thr Leu Ile Thr
               180                 185                 190
   gac acg gag acg gca gaa gat gaa ttt aac aga gtc aat gac tac ctg    624
   Asp Thr Glu Thr Ala Glu Asp Glu Phe Asn Arg Val Asn Asp Tyr Leu
           195                 200                 205
   aaa tcg att aac gtg ccg aag gcg gtc aca ttt gaa gca ccg ttt att    672
   Lys Ser Ile Asn Val Pro Lys Ala Val Thr Phe Glu Ala Pro Phe Ile
       210                 215                 220
   gct gag gaa ttt tta cag ggt gag tac gga gac tgg tat caa aca gaa     720
   Ala Glu Glu Phe Leu Gln Gly Glu Tyr Gly Asp Trp Tyr Gln Thr Glu
   225                 230                 235                 240
   ggg tac tcc gac tat atc agc ata gaa ggc att atg gca gat ggt gag     768
   Gly Tyr Ser Asp Tyr Ile Ser Ile Glu Gly Ile Met Ala Asp Gly Glu
                   245                 250                 255
   tat ttt ccg atc gcc att cat gac aaa acg ccg caa att gga ttt aca     816
   Tyr Phe Pro Ile Ala Ile His Asp Lys Thr Pro Gln Ile Gly Phe Thr
               260                 265                 270
   gag aca tca cat att acg cca tcc att ctg gat gaa gag gcg aaa aag     864
   Glu Thr Ser His Ile Thr Pro Ser Ile Leu Asp Glu Glu Ala Lys Lys
           275                 280                 285
   aaa att gtc gaa gcg gct aaa aag gca aat gaa ggg ctt gga ctg caa     912
   Lys Ile Val Glu Ala Ala Lys Lys Ala Asn Glu Gly Leu Gly Leu Gln
       290                 295                 300
   aat tgc gca aca cat aca gaa atc aag cta atg aaa aac aga gaa ccg     960
   Asn Cys Ala Thr His Thr Glu Ile Lys Leu Met Lys Asn Arg Glu Pro
   305                 310                 315                 320
   ggt tta ata gag tcg gct gcc aga ttc gca ggc tgg aat atg att cct     1008
   Gly Leu Ile Glu Ser Ala Ala Arg Phe Ala Gly Trp Asn Met Ile Pro
                   325                 330                 335
   aac att aaa aag gtt ttc ggc ctt gat atg gcg caa tta tta tta gat     1056
   Asn Ile Lys Lys Val Phe Gly Leu Asp Met Ala Gln Leu Leu Leu Asp
               340                 345                 350
   gtt ctc tgt ttc gga aaa gat gct gat ctg ccg gac ggg tta ttg gat     1104
   Val Leu Cys Phe Gly Lys Asp Ala Asp Leu Pro Asp Gly Leu Leu Asp
           355                 360                 365
   caa gag cct tac tat gtt gct gac tgc cat ctg tac cct cag cat ttc     1152
   Gln Glu Pro Tyr Tyr Val Ala Asp Cys His Leu Tyr Pro Gln His Phe
       370                 375                 380
   aaa caa aat ggc cag atc cct gaa act gcc gag gat ttg gta atc gaa    1200
   Lys Gln Asn Gly Gln Ile Pro Glu Thr Ala Glu Asp Leu Val Ile Glu
   385                 390                 395                 400
   gcg atc gat att ccg gat ggg ctt ttg aag ggt gat aca gaa atc gtt    1248
   Ala Ile Asp Ile Pro Asp Gly Leu Leu Lys Gly Asp Thr Glu Ile Val
                   405                 410                 415
   act ttt tcg gct gcg gca cca gga aca tca gtt gat ttg aca ctg ttt    1296
   Thr Phe Ser Ala Ala Ala Pro Gly Thr Ser Val Asp Leu Thr Leu Phe
               420                 425                 430
   gaa gcc ttc aac tcc att gct gca ttt gaa ctg aaa ggc agc aat tca    1344
   Glu Ala Phe Asn Ser Ile Ala Ala Phe Glu Leu Lys Gly Ser Asn Ser
           435                 440                 445
   cag gat gtg gct gaa tca atc aga caa att cag cag cat gcg aag ctg    1392
   Gln Asp Val Ala Glu Ser Ile Arg Gln Ile Gln Gln His Ala Lys Leu
       450                 455                 460
   acg gca aag tat gtg ctg cca gta                                    1416
   Thr Ala Lys Tyr Val Leu Pro Val
   465                 470
<210> 10
   <211> 1416
   <212> DNA
   <213> Bacillus subtilis IAM1213
<400> 10
   atg gag aga aaa aca gta ttg gtc atc gct gat ctt gga ggc tgc ccg    48
   Met Glu Arg Lys Thr Val Leu Val Ile Ala Asp Leu Gly Gly Cys Pro
    1               5                  10                  15
   ccg cac atg ttt tat aaa agc gct gct gaa aaa tat aac ctg gtc agc    96
   Pro His Met Phe Tyr Lys Ser Ala Ala Glu Lys Tyr Asn Leu Val Ser
               20                  25                  30
   ttt att cca aga cct ttt gca att aca gcc tcc cat gca gca ttg att    144
   Phe Ile Pro Arg Pro Phe Ala Ile Thr Ala Ser His Ala Ala Leu Ile
               35                  40                  45
   gaa aaa tac tcg gtc gcg gtc ata aaa gat aaa gac tat ttt aag agt    192
   Glu Lys Tyr Ser Val Ala Val Ile Lys Asp Lys Asp Tyr Phe Lys Ser
           50                  55                  60
   tta gct gat ttt gag cat cct gac tcc att tat tgg gcg cat gag gat    240
   Leu Ala Asp Phe Glu His Pro Asp Ser Ile Tyr Trp Ala His Glu Asp
   65                  70                  75                  80
   cat aac aag cct gag gaa gag gtc gtc gag caa atc gtc aag gtt gcc    288
   His Asn Lys Pro Glu Glu Glu Val Val Glu Gln Ile Val Lys Val Ala
                   85                  90                  95
   gaa atg ttt ggg gcg gat gcc atc aca aca aac aat gaa tta ttc att    336
   Glu Met Phe Gly Ala Asp Ala Ile Thr Thr Asn Asn Glu Leu Phe Ile
               100                 105                 110
   gct ccg atg gcg aaa gcc tgt gaa cgt ctg ggc ctg aga ggt gcc ggc    384
   Ala Pro Met Ala Lys Ala Cys Glu Arg Leu Gly Leu Arg Gly Ala Gly
           115                 120                 125
   gtg cag gca gcc gaa aat gcc aga gat aaa aat aaa atg agg gac gct    432
   Val Gln Ala Ala Glu Asn Ala Arg Asp Lys Asn Lys Met Arg Asp Ala
       130                 135                 140
   ttt aat aag gcc gga gtc aaa tcg atc aaa aac aaa cga gtc aca act    480
   Phe Asn Lys Ala Gly Val Lys Ser Ile Lys Asn Lys Arg Val Thr Thr
   145                 150                 155                 160
   ctc gaa gat ttc cgt gct gct ctt gaa gag atc ggc aca cct ctt atc    528
   Leu Glu Asp Phe Arg Ala Ala Leu Glu Glu Ile Gly Thr Pro Leu Ile
                   165                 170                 175
   tta aag cct aca tac tta gcg agt tca atc ggt gta acg ctg att acg    576
   Leu Lys Pro Thr Tyr Leu Ala Ser Ser Ile Gly Val Thr Leu Ile Thr
               180                 185                 190
   gac act gag acg gca gaa gat gaa ttt aac aga gtc aat gac tat ctg    624
   Asp Thr Glu Thr Ala Glu Asp Glu Phe Asn Arg Val Asn Asp Tyr Leu
           195                 200                 205
   aaa tca att aac gtg cca aag gcg gtt acg ttt gaa gcg ccg ttt atc     672
   Lys Ser Ile Asn Val Pro Lys Ala Val Thr Phe Glu Ala Pro Phe Ile
       210                 215                 220
   gct gaa gaa ttt tta cag ggt gag tac gga gac tgg tat caa aca gaa     720
   Ala Glu Glu Phe Leu Gln Gly Glu Tyr Gly Asp Trp Tyr Gln Thr Glu
   225                 230                 235                 240
   ggg tac tcc gac tat atc agt ata gaa ggc atc atg gct gac ggt gag     768
   Gly Tyr Ser Asp Tyr Ile Ser Ile Glu Gly Ile Met Ala Asp Gly Glu
                   245                 250                 255
   tat ttc ccg atc gcc att cat gat aaa acg ccg caa atc ggg ttt aca     816
   Tyr Phe Pro Ile Ala Ile His Asp Lys Thr Pro Gln Ile Gly Phe Thr
               260                 265                 270
   gag aca tcc cac att acg ccg tcc att ctg gat gaa gag gca aaa aag     864
   Glu Thr Ser His Ile Thr Pro Ser Ile Leu Asp Glu Glu Ala Lys Lys
           275                 280                 285
   aaa att gtc gaa gct gcc aaa aag gca aat gaa ggt ctt ggc ctg caa     912
   Lys Ile Val Glu Ala Ala Lys Lys Ala Asn Glu Gly Leu Gly Leu Gln
       290                 295                 300
   aat tgc gca aca cat aca gag atc aag cta atg aaa aat aga gaa ccg     960
   Asn Cys Ala Thr His Thr Glu Ile Lys Leu Met Lys Asn Arg Glu Pro
   305                 310                 315                 320
   ggt tta att gaa tcg gca gcc aga ttc gcc ggc tgg aat atg atc ccc     1008
   Gly Leu Ile Glu Ser Ala Ala Arg Phe Ala Gly Trp Asn Met Ile Pro
                   325                 330                 335
   aat att aaa aag gtc ttt ggc ctt gat atg gcg caa tta tta tta gat     1056
   Asn Ile Lys Lys Val Phe Gly Leu Asp Met Ala Gln Leu Leu Leu Asp
               340                 345                 350
   gtc ctt tgt ttc gga aaa gac gcc gat ctg ccg gac gga tta ttg gat     1104
   Val Leu Cys Phe Gly Lys Asp Ala Asp Leu Pro Asp Gly Leu Leu Asp
           355                 360                 365
   caa gag cct tat tat gtt gcc gac tgc cat ttg tac ccg caa cat ttc     1152
   Gln Glu Pro Tyr Tyr Val Ala Asp Cys His Leu Tyr Pro Gln His Phe
   370 375 380
   aaa caa aat ggc cag att cca gaa act gct gag gat ttg gtc att gaa 1200
   Lys Gln Asn Gly Gln Ile Pro Glu Thr Ala Glu Asp Leu Val Ile Glu
   385 390 395 400
   gcg atc gat ctg cct gac ggg ctt tta aaa ggg gat act gag atc gtt 1248
   Ala Ile Asp Leu Pro Asp Gly Leu Leu Lys Gly Asp Thr Glu Ile Val
   405 410 415
   tct ttt tcg gcc gca gca cca gga act tca gtt gat ttg aca ttg ttt 1296
   Ser Phe Ser Ala Ala Ala Pro Gly Thr Ser Val Asp Leu Thr Leu Phe
   420 425 430
   gaa gct ttc aat tcc att gct gca ttt gaa ctg aaa ggc agt aat tca 1344
   Glu Ala Phe Asn Ser Ile Ala Ala Phe Glu Leu Lys Gly Ser Asn Ser
   435 440 445
   cag gat gtg gct gaa tca atc aga caa att cag cag cat gcg aag ctg 1392
   Gln Asp Val Ala Glu Ser Ile Arg Gln Ile Gln Gln His Ala Lys Leu
   450 455 460
   acg gca aag tat gtg ctg cca gta 1416
   Thr Ala Lys Tyr Val Leu Pro Val
   465 470
<210> 11
   <211> 1416
   <212> DNA
   <213> Bacillus subtilis IAM1107
<400> 11
   atg gag aga aaa aca gta ttg gtc atc gct gat ctt gga ggc tgc ccg 48
   Met Glu Arg Lys Thr Val Leu Val Ile Ala Asp Leu Gly Gly Cys Pro
   1 5 10 15
   ccg cac atg ttt tat aaa agc gct gct gaa aaa tat aac ctg gtc agc 96
   Pro His Met Phe Tyr Lys Ser Ala Ala Glu Lys Tyr Asn Leu Val Ser
   20 25 30
   ttt att cca aga cct ttt gca att aca gcc tcc cat gca gca ttg att 144
   Phe Ile Pro Arg Pro Phe Ala Ile Thr Ala Ser His Ala Ala Leu Ile
            35                  40                  45
   gaa aaa tac tcg gtc gcg gtc gta aaa gat aaa gac tat ttt aag agt     192
   Glu Lys Tyr Ser Val Ala Val Val Lys Asp Lys Asp Tyr Phe Lys Ser
        50                  55                  60
   tta gct gat ttt gag cat cct gac tcc att tat tgg gcg cat gag gat     240
   Leu Ala Asp Phe Glu His Pro Asp Ser Ile Tyr Trp Ala His Glu Asp
    65                  70                  75                  80
   cat aac aag cct gag gaa gag gtc gtc gag caa atc gtc aag gtt gcc     288
   His Asn Lys Pro Glu Glu Glu Val Val Glu Gln Ile Val Lys Val Ala
                    85                  90                  95
   gaa atg ttc ggg gcg gat gcc atc aca aca aac aat gaa tta ttc att     336
   Glu Met Phe Gly Ala Asp Ala Ile Thr Thr Asn Asn Glu Leu Phe Ile
               100                 105                 110
   gct ccg atg gcg aaa gcc tgt gaa cgt ctg ggc ttg aga ggt gcc ggc     384
   Ala Pro Met Ala Lys Ala Cys Glu Arg Leu Gly Leu Arg Gly Ala Gly
           115                 120                 125
   gtg cag gca gcc gaa aat gcc aga gat aaa aat aaa atg agg gac gct     432
   Val Gln Ala Ala Glu Asn Ala Arg Asp Lys Asn Lys Met Arg Asp Ala
       130                 135                 140
   ttt aat aag gcc gga gtc aaa tcg atc aaa aac aaa cga gtc aca act     480
   Phe Asn Lys Ala Gly Val Lys Ser Ile Lys Asn Lys Arg Val Thr Thr
   145                 150                 155                 160
   ctt gaa gat ttc cgt gct gct ctt gaa gag atc ggc aca cct ctt atc     528
   Leu Glu Asp Phe Arg Ala Ala Leu Glu Glu Ile Gly Thr Pro Leu Ile
                   165                 170                 175
   tta aag cct aca tac tta gcg agt tct atc ggt gta acg ctg att acg     576
   Leu Lys Pro Thr Tyr Leu Ala Ser Ser Ile Gly Val Thr Leu Ile Thr
               180                 185                 190
   gac act gag acg gca gaa gat gaa ttt aac aga gtc aat gac tat ctg     624
   Asp Thr Glu Thr Ala Glu Asp Glu Phe Asn Arg Val Asn Asp Tyr Leu
           195                 200                 205
   aaa tca att aac gtg cca aag gcg gtt acg ttt gaa gcg ccg ttt atc 672
   Lys Ser Ile Asn Val Pro Lys Ala Val Thr Phe Glu Ala Pro Phe Ile
   210 215 220
   gct gaa gaa ttt tta cag ggt gag tac gga gac tgg tat caa aca gaa 720
   Ala Glu Glu Phe Leu Gln Gly Glu Tyr Gly Asp Trp Tyr Gln Thr Glu
   225 230 235 240
   ggg tac tcc gac tat atc agt ata gaa ggc atc atg gct gac ggt gag 768
   Gly Tyr Ser Asp Tyr Ile Ser Ile Glu Gly Ile Met Ala Asp Gly Glu
   245 250 255
   tat ttc ccg atc gcc att cat gat aaa acg ccg caa atc ggg ttt aca 816
   Tyr Phe Pro Ile Ala Ile His Asp Lys Thr Pro Gln Ile Gly Phe Thr
   260 265 270
   gag aca tcc cac att acg ccg tcc att ctg gat gaa gag gca aaa aag 864
   Glu Thr Ser His Ile Thr Pro Ser Ile Leu Asp Glu Glu Ala Lys Lys
   275 280 285
   aaa att gtc gaa gct gcc aaa aag gca aat gaa ggg ctt ggc ctg caa 912
   Lys Ile Val Glu Ala Ala Lys Lys Ala Asn Glu Gly Leu Gly Leu Gln
   290 295 300
   aat tgc gca aca cat aca gag gtc aag cta atg aaa aac aga gaa ccg 960
   Asn Cys Ala Thr His Thr Glu Val Lys Leu Met Lys Asn Arg Glu Pro
   305 310 315 320
   ggt tta att gaa tcg gca gcc aga ttt gcc ggc tgg aat atg atc cct 1008
   Gly Leu Ile Glu Ser Ala Ala Arg Phe Ala Gly Trp Asn Met Ile Pro
   325 330 335
   aac att aaa aag gtt ttc ggc ctt gat atg gcg caa tta tta tta gat 1056
   Asn Ile Lys Lys Val Phe Gly Leu Asp Met Ala Gln Leu Leu Leu Asp
   340 345 350
   gtc ctc tgt ttc gga aaa gat gcc gat ctg ccg gac gga tta ttg gat 1104
   Val Leu Cys Phe Gly Lys Asp Ala Asp Leu Pro Asp Gly Leu Leu Asp
   355 360 365
   caa gag cct tac tat gtc gcc gac tgc cat ttg tac ccg cag cat ttc 1152
   Gln Glu Pro Tyr Tyr Val Ala Asp Cys His Leu Tyr Pro Gln His Phe
       370                 375                 380
   aaa caa aat ggc cag att cca gaa acc gct gag gat ttg gtc att gaa    1200
   Lys Gln Asn Gly Gln Ile Pro Glu Thr Ala Glu Asp Leu Val Ile Glu
   385                 390                 395                 400
   gcg atc gat att ccg gac ggg ctt tta aaa ggg gat act gaa atc ttt    1248
   Ala Ile Asp Ile Pro Asp Gly Leu Leu Lys Gly Asp Thr Glu Ile Phe
                   405                 410                 415
   tct ttt tcg gcc gca gca cca ggc act tca gtt gat ttg aca ttg ttt    1296
   Ser Phe Ser Ala Ala Ala Pro Gly Thr Ser Val Asp Leu Thr Leu Phe
               420                 425                 430
   gaa gct ttc aat tcc att gct gca ttt gaa ctg aaa ggc agt aat tca    1344
   Glu Ala Phe Asn Ser Ile Ala Ala Phe Glu Leu Lys Gly Ser Asn Ser
           435                 440                 445
   cag gat gtg gct gaa tca atc aga caa att cag cag cat gcg aag ctg    1392
   Gln Asp Val Ala Glu Ser Ile Arg Gln Ile Gln Gln His Ala Lys Leu
       450                 455                 460
   acg gca aag tat gtg ctg cca gta                                    1416
   Thr Ala Lys Tyr Val Leu Pro Val
   465                 470
<210> 12
   <211> 1416
   <212> DNA
   <213> Bacillus subtilis IAM1214
<400> 12
   atg gag aga aaa aca gta ttg gtc atc gct gat ctt gga ggc tgc ccg    48
   Met Glu Arg Lys Thr Val Leu Val Ile Ala Asp Leu Gly Gly Cys Pro
     1               5                  10                  15
   ccg cac atg ttt tat aaa agc gct gct gaa aaa tat aac ctg gtt agc    96
   Pro His Met Phe Tyr Lys Ser Ala Ala Glu Lys Tyr Asn Leu Val Ser
                   20                  25                  30
   ttt att ccg aga cct ttt gca ata aca gcc tcc cat gca gca ctg att    144
   Phe Ile Pro Arg Pro Phe Ala Ile Thr Ala Ser His Ala Ala Leu Ile
           35                  40                  45
   gaa aaa tac tcg gtc gcg gtc ata aaa gat aaa gac tat ttt cag agc    192
   Glu Lys Tyr Ser Val Ala Val Ile Lys Asp Lys Asp Tyr Phe Gln Ser
       50                  55                  60
   tta gct gat ttt gag cat ccc gat tca att tat tgg gcg cat gag gat    240
   Leu Ala Asp Phe Glu His Pro Asp Ser Ile Tyr Trp Ala His Glu Asp
   65                  70                  75                  80
   cat gac aag cct gaa gaa gag gtt gtc gag caa atc gtc aag gtt gcc    288
   His Asp Lys Pro Glu Glu Glu Val Val Glu Gln Ile Val Lys Val Ala
                   85                  90                  95
   caa atg ttt gag gcg gac gcc atc aca aca aac aat gaa tta ttc att    336
   Gln Met Phe Glu Ala Asp Ala Ile Thr Thr Asn Asn Glu Leu Phe Ile
               100                 105                 110
   gcc ccg atg gcg aaa gcc tgt gaa cgc ctt ggc ctg agg ggc gcc gga    384
   Ala Pro Met Ala Lys Ala Cys Glu Arg Leu Gly Leu Arg Gly Ala Gly
           115                 120                 125
   gtg cag gca gcg gaa aat gcc aga gat aaa aat aaa atg agg gac gct    432
   Val Gln Ala Ala Glu Asn Ala Arg Asp Lys Asn Lys Met Arg Asp Ala
       130                 135                 140
   ttt aat aag gcg gga gtc aaa tcg atc aaa aac aaa cga gtc aca act    480
   Phe Asn Lys Ala Gly Val Lys Ser Ile Lys Asn Lys Arg Val Thr Thr
   145                 150                 155                 160
   ctt gag gat ttt cgt gct gca ctt gaa gag atc ggc aca cct cta atc    528
   Leu Glu Asp Phe Arg Ala Ala Leu Glu Glu Ile Gly Thr Pro Leu Ile
                   165                 170                 175
   tta aag cct aca tac tta gcg agt tca atc ggc gta acg ctg att acc    576
   Leu Lys Pro Thr Tyr Leu Ala Ser Ser Ile Gly Val Thr Leu Ile Thr
               180                 185                 190
   gac acg gag acg gca gaa gat gaa ttt aac aga gtc aat gac tac ctg    624
   Asp Thr Glu Thr Ala Glu Asp Glu Phe Asn Arg Val Asn Asp Tyr Leu
           185                 200                 205
   aaa tcg att aac gtg ccg aag gcg gtc aca ttt gaa gca ccg ttt att    672
   Lys Ser Ile Asn Val Pro Lys Ala Val Thr Phe Glu Ala Pro Phe Ile
       210                 215                 220
   gct gag gaa ttt tta cag ggt gag tac gga gac tgg tat caa aca gaa    720
   Ala Glu Glu Phe Leu Gln Gly Glu Tyr Gly Asp Trp Tyr Gln Thr Glu
   225                 230                 235                 240
   ggg tac tcc gac tat atc agc ata gaa ggc att atg gca gat ggt gag    768
   Gly Tyr Ser Asp Tyr Ile Ser Ile Glu Gly Ile Met Ala Asp Gly Glu
                   245                 250                 255
   tat ttt ccg atc gcc att cat gac aaa acg ccg caa att gga ttt aca    816
   Tyr Phe Pro Ile Ala Ile His Asp Lys Thr Pro Gln Ile Gly Phe Thr
               260                 265                 270
   gag aca tca cat att acg cca tcc att ctg gat gaa gag gcg aaa aag    864
   Glu Thr Ser His Ile Thr Pro Ser Ile Leu Asp Glu Glu Ala Lys Lys
           275                 280                 285
   aaa att gtc gaa gcg gct aaa aag gca aat gaa ggg ctt gga ctg caa    912
   Lys Ile Val Glu Ala Ala Lys Lys Ala Asn Glu Gly Leu Gly Leu Gln
       290                 295                 300
   aat tgc gca aca cat aca gaa atc aag cta atg aaa aac aga gaa ccg    960
   Asn Cys Ala Thr His Thr Glu Ile Lys Leu Met Lys Asn Arg Glu Pro
   305                 310                 315                 320
   ggt tta ata gag tcg gct gcc aga ttc gca ggc tgg aat atg att cct   1008
   Gly Leu Ile Glu Ser Ala Ala Arg Phe Ala Gly Trp Asn Met Ile Pro
                   325                 330                 335
   aac att aaa aag gtt ttc ggc ctt gat atg gcg caa tta tta tta gat   1056
   Asn Ile Lys Lys Val Phe Gly Leu Asp Met Ala Gln Leu Leu Leu Asp
               340                 345                 350
   gtt ctc tgt ttc gga aaa gat gct gat ctg ccg gac ggg tta ttg gat   1104
   Val Leu Cys Phe Gly Lys Asp Ala Asp Leu Pro Asp Gly Leu Leu Asp
           355                 360                 365
   caa gag cct tac tat gtt gct gac tgc cat ctg tac cct cag cat ttc   1152
   Gln Glu Pro Tyr Tyr Val Ala Asp Cys His Leu Tyr Pro Gln His Phe
       370                 375                 380
   aaa caa aat ggc cag atc cct gaa act gcc gag gat ttg gta atc gaa   1200
   Lys Gln Asn Gly Gln Ile Pro Glu Thr Ala Glu Asp Leu Val Ile Glu
   385                 390                 395                 400
   gcg atc gat att ccg gat ggg ctt ttg aag ggt gat aca gaa atc gtt   1248
   Ala Ile Asp Ile Pro Asp Gly Leu Leu Lys Gly Asp Thr Glu Ile Val
                   405                 410                 415
   act ttt tcg gct gcg gca cca gga aca tca gtt gat ttg aca ctg ttt   1296
   Thr Phe Ser Ala Ala Ala Pro Gly Thr Ser Val Asp Leu Thr Leu Phe
               420                 425                 430
   gaa gcc ttc aac tcc att gct gca ttt gaa ctg aaa ggc agc aat tca   1344
   Glu Ala Phe Asn Ser Ile Ala Ala Phe Glu Leu Lys Gly Ser Asn Ser
           435                 440                 445
   cag gat gtg gct gaa tca atc aga caa att cag cag cat gcg aag ctg   1392
   Gln Asp Val Ala Glu Ser Ile Arg Gln Ile Gln Gln His Ala Lys Leu
       450                 455                 460
   acg gca aag tat gtg ctg cca gta                                   1416
   Thr Ala Lys Tyr Val Leu Pro Val
   465                 470
<210> 13
   <211> 1416
   <212> DNA
   <213> Bacillus subtilis ATCC21555
<400> 13
   atg gag aga aaa aca gta ttg gtt atc gct gat ctt ggg ggc tgc ccg   48
   Met Glu Arg Lys Thr Val Leu Val Ile Ala Asp Leu Gly Gly Cys Pro
     1               5                  10                  15
   ccg cat atg ttt tac aaa agc gca gcc gaa aaa tac aac ctc gtc agc   96
   Pro His Met Phe Tyr Lys Ser Ala Ala Glu Lys Tyr Asn Leu Val Ser
                20                  25                  30
   ttt att ccg aga ccc ttt gca att aca gcc tct cat gcg gcc tta att   144
   Phe Ile Pro Arg Pro Phe Ala Ile Thr Ala Ser His Ala Ala Leu Ile
            35                  40                  45
   gaa aaa tac tcg att gcg gtc att aaa gat aaa gac tat ttt aag agt   192
   Glu Lys Tyr Ser Ile Ala Val Ile Lys Asp Lys Asp Tyr Phe Lys Ser
        50                  55                  60
   ctg gct gat ttt gaa cat ccc gat tcg att tat tgg gct cat gaa gat   240
   Leu Ala Asp Phe Glu His Pro Asp Ser Ile Tyr Trp Ala His Glu Asp
    65                  70                  75                  80
   cat gac aaa cct gag gaa gaa gtc gtc gaa gaa atc gtg aaa gtg gcc   288
   His Asp Lys Pro Glu Glu Glu Val Val Glu Glu Ile Val Lys Val Ala
                    85                  90                  95
   gac atg ttt ggg gtt gac gcc att acg acc aac aat gaa ctg ttt atc   336
   Asp Met Phe Gly Val Asp Ala Ile Thr Thr Asn Asn Glu Leu Phe Ile
               100                 105                 110
   gct ccg atg gca aaa gcg tgt aaa cgt ctc ggc ctg cgg gga gcg ggc   384
   Ala Pro Met Ala Lys Ala Cys Lys Arg Leu Gly Leu Arg Gly Ala Gly
           115                 120                 125
   gta cag gcc gct gaa aac gcc aga gat aaa aat aaa atg aga gcc gcc   432
   Val Gln Ala Ala Glu Asn Ala Arg Asp Lys Asn Lys Met Arg Ala Ala
       130                 135                 140
   ttc aac cgg gcc ggc gtc aaa tcc atc aaa aac aaa cgg gtg acg acc   480
   Phe Asn Arg Ala Gly Val Lys Ser Ile Lys Asn Lys Arg Val Thr Thr
   145                 150                 155                 160
   ctg gaa gat ttc cgc gcc gcg ctt cag gaa atc gga acg ccg ctt att   528
   Leu Glu Asp Phe Arg Ala Ala Leu Gln Glu Ile Gly Thr Pro Leu Ile
                   165                 170                 175
   ctg aag cct aca tat ctg gca agc tcg atc ggc gtg acg ctt att aaa   576
   Leu Lys Pro Thr Tyr Leu Ala Ser Ser Ile Gly Val Thr Leu Ile Lys
               180                 185                 190
   gag atg gaa acg gcc gaa gct gaa ttc aac aga gtc aat gag tac ttg   624
   Glu Met Glu Thr Ala Glu Ala Glu Phe Asn Arg Val Asn Glu Tyr Leu
           195                 200                 205
   aaa tcg att aat gta ccg aaa gcg gtg acg ttt gaa gcg ccg ttt atc     672
   Lys Ser Ile Asn Val Pro Lys Ala Val Thr Phe Glu Ala Pro Phe Ile
       210                 215                 220
   gcg gaa gaa ttc ttg cag ggc gag tat gat gac tgg tac gaa aca agc     720
   Ala Glu Glu Phe Leu Gln Gly Glu Tyr Asp Asp Trp Tyr Glu Thr Ser
   225                 230                 235                 240
   ggt tat tcc gac tat atc agc atc gaa ggc atc atg gcc gac gga gaa     768
   Gly Tyr Ser Asp Tyr Ile Ser Ile Glu Gly Ile Met Ala Asp Gly Glu
                   245                 250                 255
   tac ttc ccc gtt gcg atc cat gat aaa aca ccg caa atc gga ttc acg     816
   Tyr Phe Pro Val Ala Ile His Asp Lys Thr Pro Gln Ile Gly Phe Thr
               260                 265                 270
   gag aca gcg cat att acg ccg tcc atc ctg gat gat gac gcc aag cgg     864
   Glu Thr Ala His Ile Thr Pro Ser Ile Leu Asp Asp Asp Ala Lys Arg
           275                 280                 285
   aaa atc gtc gaa gct gcc aag aag gcg aat gaa gga ctc ggc ctc gaa     912
   Lys Ile Val Glu Ala Ala Lys Lys Ala Asn Glu Gly Leu Gly Leu Glu
       290                 295                 300
   aac tgt gca acg cat aca gaa ata aaa tta atg aaa aac cgg gaa gcc     960
   Asn Cys Ala Thr His Thr Glu Ile Lys Leu Met Lys Asn Arg Glu Ala
   305                 310                 315                 320
   gga ctg att gag tca gcg gcc aga ttc gcg gga tgg aat atg att ccg    1008
   Gly Leu Ile Glu Ser Ala Ala Arg Phe Ala Gly Trp Asn Met Ile Pro
                   325                 330                 335
   aat att aaa aag gtc ttc ggc gtt gat atg gcg cag cta tta ttg gat    1056
   Asn Ile Lys Lys Val Phe Gly Val Asp Met Ala Gln Leu Leu Leu Asp
               340                 345                 350
   gtt ctc tgt tac gga aaa gaa gct gat ctg ccg aaa gga tta ttg gag    1104
   Val Leu Cys Tyr Gly Lys Glu Ala Asp Leu Pro Lys Gly Leu Leu Glu
           355                 360                 365
   cag gag cca tgc tat gtc gca gac tgc cac ttg tat cct cag cat ttc    1152
   Gln Glu Pro Cys Tyr Val Ala Asp Cys His Leu Tyr Pro Gln His Phe
       370                 375                 380
   aaa gag aac ggc cag ctg cct gag acg gtt gtc gat ttc gtc att gaa    1200
   Lys Glu Asn Gly Gln Leu Pro Glu Thr Val Val Asp Phe Val Ile Glu
   385                 390                 395                 400
   agc att gaa att cct gac ggc gtc tta aag gga gac act gaa ctc gtt    1248
   Ser Ile Glu Ile Pro Asp Gly Val Leu Lys Gly Asp Thr Glu Leu Val
                   405                 410                 415
   tct ttc tca gcg gct gag gcg ggt acg tca gtg gat ctg cgg ctg ttc    1296
   Ser Phe Ser Ala Ala Glu Ala Gly Thr Ser Val Asp Leu Arg Leu Phe
               420                 425                 430
   gaa gcg ttc aac agc att gcg gcg ttt gag ctg aaa gga agc aat tcg    1344
   Glu Ala Phe Asn Ser Ile Ala Ala Phe Glu Leu Lys Gly Ser Asn Ser
           435                 440                 445
   aac gac gtg gcc gaa tca atc aaa caa att cag cag cag gcg aag ctg    1392
   Asn Asp Val Ala Glu Ser Ile Lys Gln Ile Gln Gln Gln Ala Lys Leu
       450                 455                 460
   act gca aag tat gcg tta tcg gta                                    1416
   Thr Ala Lys Tyr Ala Leu Ser Val
<210> 14
   <211> 1416
   <212> DNA
   <213> Bacillus amyloliquefaciens IFO3022
<400> 14
   atg gag aga aaa aca gta ttg gtt atc gct gac ctt ggg gga tgc ccg    48
   Met Glu Arg Lys Thr Val Leu Val Ile Ala Asp Leu Gly Gly Cys Pro
   1               5                   10                  15
   ccg cat atg ttt tac aaa agc gca gcc gaa aaa tac aac ctc gtc agc    96
   Pro His Met Phe Tyr Lys Ser Ala Ala Glu Lys Tyr Asn Leu Val Ser
               20                  25                  30
   ttt att ccg aga cct ttt gca att aca gcc tct cat gcg gca tta att   144
   Phe Ile Pro Arg Pro Phe Ala Ile Thr Ala Ser His Ala Ala Leu Ile
           35                  40                  45
   gaa aaa tac tcg gtc gcg gtc ata aaa gat aaa gac tat ttt aag agt   192
   Glu Lys Tyr Ser Val Ala Val Ile Lys Asp Lys Asp Tyr Phe Lys Ser
       50                  55                  60
   ctg gct gat ttt gag cat ccc gat tcg att tac tgg gct cat gaa gat   240
   Leu Ala Asp Phe Glu His Pro Asp Ser Ile Tyr Trp Ala His Glu Asp
   65                  70                  75                  80
   cat gac aaa cct gag gaa gaa gta gtc gaa gaa atc gtc aag gtg gcc   288
   His Asp Lys Pro Glu Glu Glu Val Val Glu Glu Ile Val Lys Val Ala
                   85                  90                  95
   ggc atg ttc gcg gtt gac gcc att acg acc aac aat gaa ctg ttt atc   336
   Gly Met Phe Ala Val Asp Ala Ile Thr Thr Asn Asn Glu Leu Phe Ile
               100                 105                 110
   gct ccg atg gca aaa gcg tgt gaa cgt ctc ggc ctg cgg gga gcg ggc   384
   Ala Pro Met Ala Lys Ala Cys Glu Arg Leu Gly Leu Arg Gly Ala Gly
           115                 120                 125
   gta cag gcc gct gaa aat gcc aga gat aaa aac aaa atg aga gcc gct   432
   Val Gln Ala Ala Glu Asn Ala Arg Asp Lys Asn Lys Met Arg Ala Ala
       130                 135                 140
   ttc aac cgg gcc ggc gtc aag tct atc aaa aac aga cgg gtg acg acg   480
   Phe Asn Arg Ala Gly Val Lys Ser Ile Lys Asn Arg Arg Val Thr Thr
   145                 150                 155                 160
   ctg gaa gat ttc cgc gcc gcg ctt cag gaa atc gga acg ccg ctc att   528
   Leu Glu Asp Phe Arg Ala Ala Leu Gln Glu Ile Gly Thr Pro Leu Ile
                   165                 170                 175
   ctg aag cct aca tat ctg gcg agc tcc atc ggc gtg acg ctc atc aaa   576
   Leu Lys Pro Thr Tyr Leu Ala Ser Ser Ile Gly Val Thr Leu Ile Lys
               180                 185                 190
   gag agg gaa acg gcc gaa gcc gaa ttt aac aga gtc aat gaa tac ctg   624
   Glu Arg Glu Thr Ala Glu Ala Glu Phe Asn Arg Val Asn Glu Tyr Leu
   195 200 205
   aag tcg atc aac gta ccg aaa gcg gtc acg ttt gaa gcg ccg ttt atc 672
   Lys Ser Ile Asn Val Pro Lys Ala Val Thr Phe Glu Ala Pro Phe Ile
   210 215 220
   gcg gaa gaa ttt ttg cag ggc gag tat gac gac tgg tac gaa aca agc 720
   Ala Glu Glu Phe Leu Gln Gly Glu Tyr Asp Asp Trp Tyr Glu Thr Ser
   225 230 235 240
   ggt tat tcc gac tat atc agc ata gaa ggc atc atg gcc gac gga gaa 768
   Gly Tyr Ser Asp Tyr Ile Ser Ile Glu Gly Ile Met Ala Asp Gly Glu
   245 250 255
   tac ttc cct gtc gca att cat gat aaa aca ccg caa atc gga ttc acg 816
   Tyr Phe Pro Val Ala Ile His Asp Lys Thr Pro Gln Ile Gly Phe Thr
   260 265 270
   gag aca tcg cat att acg ccg tcc atc ctg gat gat gac gcg aag cgg 864
   Glu Thr Ser His Ile Thr Pro Ser Ile Leu Asp Asp Asp Ala Lys Arg
   275 280 285
   aaa atc gtc gaa gca gcc aaa aag gcg aat gaa gga ctc ggc ctc gaa 912
   Lys Ile Val Glu Ala Ala Lys Lys Ala Asn Glu Gly Leu Gly Leu Glu
   290 295 300
   aac tgc gca acc cat aca gag att aaa tta atg aaa aac cgg gaa gcc 960
   Asn Cys Ala Thr His Thr Glu Ile Lys Leu Met Lys Asn Arg Glu Ala
   305 310 315 320
   gga ctg att gaa tca gcg gca cga ttt gcg ggc tgg aac atg att ccg 1008
   Gly Leu Ile Glu Ser Ala Ala Arg Phe Ala Gly Trp Asn Met Ile Pro
   325 330 335
   aat att aaa aag gtc ttc ggc gtc gat atg gcg cag ctg tta ttg gat 1056
   Asn Ile Lys Lys Val Phe Gly Val Asp Met Ala Gln Leu Leu Leu Asp
   340 345 350
   gtt ctc tgt ttc gga aaa gaa gcc gat ctg ccg aaa gga tta ttg gag 1104
   Val Leu Cys Phe Gly Lys Glu Ala Asp Leu Pro Lys Gly Leu Leu Glu
   355 360 365
   cag gag ccg tgc tat gtc gcc gac tgc cac ttg tat cct cag cat ttc 1152
   Gln Glu Pro Cys Tyr Val Ala Asp Cys His Leu Tyr Pro Gln His Phe
   370 375 380
   aaa gag aac ggc cag ctg cct gag acg gct gtc gat ttc gtc att gaa 1200
   Lys Glu Asn Gly Gln Leu Pro Glu Thr Ala Val Asp Phe Val Ile Glu
   385 390 395 400
   agc att gac att ccc gac ggc gtc tta aag gga gac acc gaa atc gtt 1248
   Ser Ile Asp Ile Pro Asp Gly Val Leu Lys Gly Asp Thr Glu Ile Val
   405 410 415
   tct ttc tcg gcg gcc gag gcg ggt aca tcc gtg gat ctg cgg ctg ttc 1296
   Ser Phe Ser Ala Ala Glu Ala Gly Thr Ser Val Asp Leu Arg Leu Phe
   420 425 430
   gaa gcg ttc aac agc att gcg gcg ttc gag ctg aaa gga agc aat tcg 1344
   Glu Ala Phe Asn Ser Ile Ala Ala Phe Glu Leu Lys Gly Ser Asn Ser
   435 440 445
   ggt gac gtg gcc gaa tca atc aaa caa att cag cag cag gcg aag ctg 1392
   Gly Asp Val Ala Glu Ser Ile Lys Gln Ile Gln Gln Gln Ala Lys Leu
   450 455 460
   act gca aag tat gcg tta ccg gta 1416
   Thr Ala Lys Tyr Ala Leu Pro Val
<210> 15
   <211> 93
   <212> PRT
   <213> Bacillus subtilis 168
<400> 15
   Gly Ala Gly Val Gln Ala Ala Glu Asn Ala Arg Asp Lys Asn Lys Met
   1 5 10 15
   Arg Asp Ala Phe Asn Lys Ala Gly Val Lys Ser Ile Lys Asn Lys Arg
   20 25 30
   Val Thr Thr Leu Glu Asp Phe Arg Ala Ala Leu Glu Glu Ile Gly Thr
   35 40 45
   Pro Leu Ile Leu Lys Pro Thr Tyr Leu Ala Ser Ser Ile Gly Val Thr
   50 55 60
   Leu Ile Thr Asp Thr Glu Thr Ala Glu Asp Glu Phe Asn Arg Val Asn
   65 70 75 80
   Asp Tyr Leu Lys Ser Ile Asn Val Pro Lys Ala Val Thr
   85 90
<210> 16
   (211) 279
   <212> DNA
   <213> Bacillus subtilis 168
<400> 16
<210> 17
   <211> 413
   <212> PRT
   <213> Corynebacterium glutamicum
<400> 17
   Met Thr Lys Thr Leu Gly Ser Leu Gln Leu Glu Glu Ile Thr Leu Thr
   1 5 10 15
   Leu Pro Leu Thr Glu Asp Val Ala Asp Glu Arg Thr Ile Asp Val Phe
   20 25 30
   Ala Arg Ile Ala Thr Arg Val Gly Gly Glu Asp Leu Pro Tyr Leu Val
   35 40 45
   Phe Leu Gln Gly Gly Pro Gly Asn Glu Ala Pro Arg Pro Ser Leu Asn
   50 55 60
   Pro Leu Asn Pro Asn Trp Leu Gly Val Ala Leu Glu Glu Tyr Arg Val
   65 70 75 80
   Val Met Leu Asp Gln Arg Gly Thr Gly Arg Ser Thr Pro Val Gly Asn
   85 90 95
   Asp Ile Leu Glu Lys Pro Thr Ala Glu Val Val Glu Tyr Leu Ser His
   100 105 110
   Leu Arg Ala Asp Gly Ile Val Arg Asp Ala Glu Ala Leu Arg Lys His
   115 120 125
   Leu Gly Val Asn Gln Trp Asn Leu Leu Gly Gln Ser Phe Gly Gly Phe
   130 135 140
   Thr Thr Leu His Tyr Leu Ser Arg His Ala Asp Ser Leu Asp Asn Val
   145 150 155 160
   Phe Ile Thr Gly Gly Leu Ser Ala Ile Asp Arg Pro Ala Glu Asp Val
   165 170 175
   Tyr Ala Asn Cys Tyr Asn Arg Met Arg Arg Asn Ser Glu Glu Phe Tyr
   180 185 190
   Arg Arg Phe Pro Gln Leu Arg Glu Thr Phe Arg Gly Leu Val Asn Arg
   195 200 205
   Ala Arg Ala Gly Glu Ile Val Leu Pro Thr Gly Glu Val Val Ser Glu
   210 215 220
   Thr Arg Leu Arg Ser Leu Gly His Leu Leu Gly Ser Asn Asp Gly Trp
   225 230 235 240
   Phe Asp Leu Tyr Asn Leu Leu Glu Leu Asp Pro Thr Ser Asn Ala Phe
   245 250 255
   Val His Asp Leu Ala Gly Leu Leu Pro Phe Gly Asn Arg Asn Pro Ile
   260 265 270
   Tyr Tyr Val Leu His Glu Ser Ser Tyr Ala Asp Gly Val Val Thr Asn
   275 280 285
   Trp Ala Ala Glu Arg Val Leu Pro Glu Asp Phe Arg Glu Asp Pro Thr
   290 295 300
   Leu Leu Thr Gly Glu His Val Phe Gln Glu Trp Thr Asp Thr Val Pro
   305 310 315 320
   Ser Leu Lys Pro Trp Lys Asp Val Ala Leu Ala Leu Ala Gln Gln Glu
   325 330 335
   Trp Pro Lys Leu Tyr Asp Ala Lys Ala Leu Glu Asn Ser Gln Ala Lys
   340 345 350
   Gly Ala Ala Ala Val Tyr Xaa Asn Asp Val Phe Val Pro Val Asp Tyr
   355 360 365
   Ser Leu Glu Thr Ala Gln His Leu Pro Gly Val Gln Leu Phe Ile Thr
   370 375 380
   Ser Gln His Glu His Asn Gly Leu Arg Ala Ser Ser Gly Ala Val Leu
   385 390 395 400
   Xaa His Leu Phe Asp Leu Ala His Gly Arg Glu Val Arg
   405 410
<210> 18
   <211> 337
   <212> PRT
   <213> Pseudomonas putida
<400> 18
   Met Met Pro Asn Gly Ser Gln Tyr Pro His Thr Glu Cys Ala Met Gln
   1 5 10 15
   Thr Leu Tyr Pro Gln Ile Lys Pro Tyr Ala Arg His Asp Leu Ala Val
   20 25 30
   Glu Ala Pro His Val Leu Tyr Val Asp Glu Ser Gly Ser Pro Glu Gly
   35 40 45
   Leu Pro Val Val Phe Ile His Gly Gly Pro Gly Ala Gly Cys Asp Ala
   50 55 60
   Gln Ser Arg Cys Tyr Phe Asp Pro Asn Leu Tyr Arg Ile Ile Thr Phe
   65 70 75 80
   Asp Gln Arg Gly Cys Gly Arg Ser Thr Pro His Ala Ser Leu Glu Asn
   85 90 95
   Asn Thr Thr Trp His Leu Val Glu Asp Leu Glu Arg Ile Arg Glu His
   100 105 110
   Leu Gly Ile Asp Lys Trp Val Leu Phe Gly Gly Ser Trp Gly Ser Thr
   115 120 125
   Leu Ala Leu Ala Tyr Ala Gln Thr His Pro Glu Arg Val His Gly Leu
   130 135 140
   Ile Leu Arg Gly Ile Phe Leu Cys Arg Pro Gln Glu Ile Glu Trp Phe
   145 150 155 160
   Tyr Gln Glu Gly Ala Ser Arg Leu Phe Pro Asp Tyr Trp Gln Asp Tyr
   165 170 175
   Ile Ala Pro Ile Pro Pro Glu Glu Arg Gly Asp Leu Val Lys Ala Phe
   180 185 190
   His Lys Arg Leu Thr Gly Asn Asp Gln Ile Ala Gln Met His Ala Ala
   195 200 205
   Lys Ala Trp Ser Thr Trp Glu Gly Arg Thr Ala Thr Leu Arg Pro Asn
   210 215 220
   Pro Leu Val Val Asp Arg Phe Ser Glu Pro Gln Arg Ala Leu Ser Ile
   225 230 235 240
   Ala Arg Ile Glu Cys His Tyr Phe Met Asn Asn Ala Phe Leu Glu Pro
   245 250 255
   Asp Gln Leu Ile Arg Asp Leu Pro Lys Ile Ala His Leu Pro Ala Val
   260 265 270
   Ile Val His Gly Arg Tyr Asp Val Ile Cys Pro Leu Asp Asn Ala Trp
   275 280 285
   Ala Leu His Gln Ala Trp Pro Asn Ser Glu Leu Lys Val Ile Arg Asp
   290 295 300
   Ala Gly His Ala Ala Ser Glu Pro Gly Ile Thr Asp Ala Leu Val Arg
   305 310 315 320
   Ala Ala Asp Gln Met Ala Arg Arg Leu Leu Asp Leu Pro Leu Glu Glu
   325 330 335
   Ala
<210> 19 .
   <211> 323
   <212> PRT
   <213> Pseudomonas putida
<400> 19
   Met Gln Thr Leu Tyr Pro Gln Ile Lys Pro Tyr Val Arg His Asp Leu
   1 5 10 15
   Ala Val Asp Glu Thr His Thr Leu Tyr Val Asp Glu Ser Gly Ser Pro
   20 25 30
   Gln Gly Leu Pro Val Val Phe Ile His Gly Gly Pro Gly Ala Gly Cys
   35 40 45
   Asp Ala Asn Ser Arg Cys Tyr Phe Asp Pro Asn Leu Tyr Arg Ile Val
   50 55 60
   Thr Phe Asp Gln Arg Gly Cys Gly Arg Ser Thr Pro Arg Ala Ser Leu
   65 70 75 80
   Glu Asn Asn Thr Thr Trp Asp Leu Val Ala Asp Leu Glu Arg Ile Arg
   85 90 95
   Glu His Leu Gly Ile Glu Lys Trp Val Leu Phe Gly Gly Ser Trp Gly
   100 105 110
   Ser Thr Leu Ala Leu Ala Tyr Ala Gln Thr His Pro Asp Arg Val Leu
   115 120 125
   Gly Leu Ile Val Arg Gly Ile Phe Leu Ala Arg Pro Gln Asp Ile Gln
   130 135 140
   Trp Phe Tyr Gln Ala Gly Ala Ser Arg Leu Phe Pro Asp Tyr Trp Gln
   145 150 155 160
   Asp Tyr Ile Ala Pro Ile Pro Ala Glu Glu Arg His Asp Met Ile Ser
   165 170 175
   Ala Tyr His Lys Arg Leu Thr Gly Asn Asp Gln Ile Ala Gln Met His
   180 185 190
   Ala Ala Lys Ala Trp Ser Thr Trp Glu Gly Arg Met Leu Gly Leu Cys
   195 200 205
   Pro Ser Pro Gln Leu Ile Glu Arg Phe Ser Glu Pro Gln Arg Ala Leu
   210 215 220
   Ser Ile Ala Arg Ile Glu Cys His Tyr Phe Thr Asn Asn Ser Phe Leu
   225 230 235 240
   Glu Pro Asn Gln Leu Ile Arg Asp Met His Lys Ile Ala His Leu Pro
   245                 250                 255
   Gly Ile Ile Val His Gly Arg Tyr Asp Met Ile Cys Pro Leu Asp Asn
           260                 265                 270
   Ala Trp Glu Leu His Gln Ala Trp Pro Asn Ser Glu Leu Gln Val Ile
       275                 280                 285
   Arg Glu Ala Gly His Ala Ala Ser Glu Pro Gly Ile Thr Asp Ala Leu
       290                 295                 300
   Val Arg Ala Ala Gly Asp Met Ala Arg Arg Leu Leu Asp Leu Pro Pro
   305                 310                 315                 320
   Glu Glu Ala
<210> 20
   <211> 1239
   <212> DNA
   <213> Corynebacterium glutamicum
<400> 20
   atg act aaa aca ctt ggt tcc ctt caa ctt gaa gaa att acc ttg acg    48
   Met Thr Lys Thr Leu Gly Ser Leu Gln Leu Glu Glu Ile Thr Leu Thr
    1               5                  10                  15
   ctc cct ctg act gaa gat gtg gcc gat gaa cgc acc att gat gtg ttc    96
   Leu Pro Leu Thr Glu Asp Val Ala Asp Glu Arg Thr Ile Asp Val Phe
               20                  25                  30
   gca cgc att gcc aca cgc gtc ggt ggg gaa gac ctt cca tat tta gta   144
   Ala Arg Ile Ala Thr Arg Val Gly Gly Glu Asp Leu Pro Tyr Leu Val
           35                  40                  45
   ttc ctg cag ggt ggg cct ggc aat gaa gct cca cgt cca agc ctt aat   192
   Phe Leu Gln Gly Gly Pro Gly Asn Glu Ala Pro Arg Pro Ser Leu Asn
       50                  55                  60
   ccc ctc aac ccc aat tgg ttg ggc gtg gcc ttg gag gaa tac cgc gtg   240
   Pro Leu Asn Pro Asn Trp Leu Gly Val Ala Leu Glu Glu Tyr Arg Val
   65                  70                  75                  80
   gtc atg ttg gat caa cgt ggc acc ggc cgt tcc acc cca gtg ggt aat    288
   Val Met Leu Asp Gln Arg Gly Thr Gly Arg Ser Thr Pro Val Gly Asn
                    85                  90                  95
   gat att ttg gaa aaa ccc aca gca gaa gta gtg gag tac tta tcc cac    336
   Asp Ile Leu Glu Lys Pro Thr Ala Glu Val Val Glu Tyr Leu Ser His
               100                 105                 110
   ctg cgc gca gat ggc att gtg cga gat gct gaa gcc ctg cgt aag cat    384
   Leu Arg Ala Asp Gly Ile Val Arg Asp Ala Glu Ala Leu Arg Lys His
           115                 120                 125
   ttg ggt gtg aat cag tgg aac ctt tta ggc cag tcc ttc gga ggt ttc    432
   Leu Gly Val Asn Gln Trp Asn Leu Leu Gly Gln Ser Phe Gly Gly Phe
       130                 135                 140
   acc acc ctg cat tac ttg tcc cgg cac gcc gat tcc ttg gac aac gtg    480
   Thr Thr Leu His Tyr Leu Ser Arg His Ala Asp Ser Leu Asp Asn Val
   145                 150                 155                 160
   ttt att acc ggc ggt ctc agc gct att gat cgc cca gca gaa gac gtg    528
   Phe Ile Thr Gly Gly Leu Ser Ala Ile Asp Arg Pro Ala Glu Asp Val
                   165                 170                 175
   tat gcc aac tgt tac aac cgc atg cgc cga aac tct gag gaa ttc tac    576
   Tyr Ala Asn Cys Tyr Asn Arg Met Arg Arg Asn Ser Glu Glu Phe Tyr
               180                 185                 190
   cgt cgc ttc ccg caa tta cgg gaa act ttc cga ggg ttg gtt aat cgt    624
   Arg Arg Phe Pro Gln Leu Arg Glu Thr Phe Arg Gly Leu Val Asn Arg
           195                 200                 205
   gct cgc gcc ggg gag att gtg ctt ccc acc ggc gaa gtt gtg tca gaa    672
   Ala Arg Ala Gly Glu Ile Val Leu Pro Thr Gly Glu Val Val Ser Glu
       210                 215                 220
   acc agg ctg cga tcc ctt ggt cac ttg ttg ggt agc aat gac ggc tgg    720
   Thr Arg Leu Arg Ser Leu Gly His Leu Leu Gly Ser Asn Asp Gly Trp
   225                 230                 235                 240
   ttt gat ctg tac aac ctg ctg gaa tta gat ccc acc tcc aac gct ttt    768
   Phe Asp Leu Tyr Asn Leu Leu Glu Leu Asp Pro Thr Ser Asn Ala Phe
                   245                 250                 255
   gtc cat gac ctg gca gga ctt ttg cct ttc ggc aac cgc aac cca att      816
   Val His Asp Leu Ala Gly Leu Leu Pro Phe Gly Asn Arg Asn Pro Ile
               260                 265                 270
   tat tac gtg ctc cat gag tcc tct tac gcc gac ggt gtg gtg aca aat      864
   Tyr Tyr Val Leu His Glu Ser Ser Tyr Ala Asp Gly Val Val Thr Asn
           275                 280                 285
   tgg gca gca gag cgt gtg ctt cca gag gat ttc cgc gag gat cca aca      912
   Trp Ala Ala Glu Arg Val Leu Pro Glu Asp Phe Arg Glu Asp Pro Thr
       290                 295                 300
   ctg ctc acc ggt gag cac gtg ttc cag gag tgg aca gac acc gtg ccg      960
   Leu Leu Thr Gly Glu His Val Phe Gln Glu Trp Thr Asp Thr Val Pro
   305                 310                 315                 320
   tcg ctc aag ccg tgg aag gac gtt gcc ctg gca ttg gct cag cag gaa     1008
   Ser Leu Lys Pro Trp Lys Asp Val Ala Leu Ala Leu Ala Gln Gln Glu
                   325                 330                 335
   tgg ccc aag ctt tat gat gcg aag gca ttg gaa aac tca cag gcc aag     1056
   Trp Pro Lys Leu Tyr Asp Ala Lys Ala Leu Glu Asn Ser Gln Ala Lys
               340                 345                 350
   ggc gct gca gca gtg tat ghc aat gac gtt ttc gtc cca gtg gat tac     1104
   Gly Ala Ala Ala Val Tyr Xaa Asn Asp Val Phe Val Pro Val Asp Tyr
           355                 360                 365
   tct ctg gaa acc gca caa cac ctg ccc ggt gtg cag ctg ttt atc acc     1152
   Ser Leu Glu Thr Ala Gln His Leu Pro Gly Val Gln Leu Phe Ile Thr
       370                 375                 380
   agc cag cat gaa cac aat gga ctt cgt gcc agc tca ggc gca gta ctg     1200
   Ser Gln His Glu His Asn Gly Leu Arg Ala Ser Ser Gly Ala Val Leu
   385                 390                 395                 400
   rag cac ctt ttc gat ctg gcc cac ggc cga gag gta cgc                 1239
   Xaa His Leu Phe Asp Leu Ala His Gly Arg Glu Val Arg
                   405                 410
<210> 21
   <211> 1011
   <212> DNA
   <213> Pseudomonas putida
<400> 21
   atg atg ccc aac ggc agt caa tat cct cac acg gag tgc gca atg cag    48
   Met Met Pro Asn Gly Ser Gln Tyr Pro His Thr Glu Cys Ala Met Gln
    1               5                  10                  15
   acc ctc tac ccg cag atc aaa ccc tac gcc cgg cac gat ctg gcc gtg    96
   Thr Leu Tyr Pro Gln Ile Lys Pro Tyr Ala Arg His Asp Leu Ala Val
               20                  25                  30
   gaa gcg ccg cat gtg ctg tat gtc gat gaa agc ggc tcg ccg gaa ggt    144
   Glu Ala Pro His Val Leu Tyr Val Asp Glu Ser Gly Ser Pro Glu Gly
           35                  40                  45
   ctg ccc gtg gta ttc atc cac ggt ggc ccg ggt gct ggc tgc gac gcc    192
   Leu Pro Val Val Phe Ile His Gly Gly Pro Gly Ala Gly Cys Asp Ala
       50                  55                  60
   cag agc cgt tgc tac ttt gac ccc aac ctg tac cgc atc atc acc ttc    240
   Gln Ser Arg Cys Tyr Phe Asp Pro Asn Leu Tyr Arg Ile Ile Thr Phe
   65                  70                  75                  80
   gac cag cgc ggc tgt ggc cgc tcc acg ccc cat gcc agc ctg gag aac    288
   Asp Gln Arg Gly Cys Gly Arg Ser Thr Pro His Ala Ser Leu Glu Asn
                   85                  90                  95
   aac aca acc tgg cac ctg gtc gag gac ctg gag cgc atc cgc gag cac    336
   Asn Thr Thr Trp His Leu Val Glu Asp Leu Glu Arg Ile Arg Glu His
               100                 105                 110
   ctg ggc atc gac aaa tgg gtg ctg ttc ggc ggc tcg tgg ggt tcg acc    384
   Leu Gly Ile Asp Lys Trp Val Leu Phe Gly Gly Ser Trp Gly Ser Thr
           115                 120                 125
   ctg gcc ctg gcc tac gcc cag acc cac ccc gag cgt gtg cat ggg ctg    432
   Leu Ala Leu Ala Tyr Ala Gln Thr His Pro Glu Arg Val His Gly Leu
   130 135 140
   atc ctg cgg ggc atc ttc ctg tgc cgg ccg cag gag atc gag tgg ttc 480
   Ile Leu Arg Gly Ile Phe Leu Cys Arg Pro Gln Glu Ile Glu Trp Phe
   145 150 155 160
   tac cag gag ggc gcc agc cgc ctg ttc ccc gac tac tgg cag gac tac 528
   Tyr Gln Glu Gly Ala Ser Arg Leu Phe Pro Asp Tyr Trp Gln Asp Tyr
   165 170 175
   atc gcg ccg att ccg ccg gaa gaa cgc ggc gac ctg gtc aag gcc ttc 576
   Ile Ala Pro Ile Pro Pro Glu Glu Arg Gly Asp Leu Val Lys Ala Phe
   180 185 190
   cac aag cgc ctc acc ggt aac gat cag att gcc cag atg cac gcc gcc 624
   His Lys Arg Leu Thr Gly Asn Asp Gln Ile Ala Gln Met His Ala Ala
   195 200 205
   aag gcg tgg tct acc tgg gag ggc cgt acc gcc acc ctg cgc ccg aac 672
   Lys Ala Trp Ser Thr Trp Glu Gly Arg Thr Ala Thr Leu Arg Pro Asn
   210 215 220
   ccg ctg gtg gtc gac cgc ttc tcc gag ccg cag cgg gcg ctg tcg atc 720
   Pro Leu Val Val Asp Arg Phe Ser Glu Pro Gln Arg Ala Leu Ser Ile
   225 230 235 240
   gcc cgg atc gag tgc cac tac ttc atg aac aac gcc ttc ctc gaa ccg 768
   Ala Arg Ile Glu Cys His Tyr Phe Met Asn Asn Ala Phe Leu Glu Pro
   245 250 255
   gac cag ttg atc cgc gac ctg ccg aag atc gcc cac ctg cca gcg gtg 816
   Asp Gln Leu Ile Arg Asp Leu Pro Lys Ile Ala His Leu Pro Ala Val
   260 265 270
   atc gtg cac ggt cgc tat gac gtg atc tgt ccg ctg gac aac gcc tgg 864
   Ile Val His Gly Arg Tyr Asp Val Ile Cys Pro Leu Asp Asn Ala Trp
   275 280 285
   gcc ctg cac cag gcc tgg ccg aac agc gaa ctg aag gtg atc cgc gac 912
   Ala Leu His Gln Ala Trp Pro Asn Ser Glu Leu Lys Val Ile Arg Asp
   290 295 300
   gcc ggc cac gcc gcg tcc gag ccg ggc atc acc gat gcc ctg gtg cgg     960
   Ala Gly His Ala Ala Ser Glu Pro Gly Ile Thr Asp Ala Leu Val Arg
   305                 310                 315                 320
   gca gcc gac cag atg gcc cgg cgc ctg ctc gac ctg ccc ctg gaa gaa     1008
   Ala Ala Asp Gln Met Ala Arg Arg Leu Leu Asp Leu Pro Leu Glu Glu
                   325                 330                 335
   gca                                                                 1011
   Ala
<210> 22
   <211> 969
   <212> DNA
   <213> Pseudomonas putida
<400> 22
   atg cag act ttg tac ccg cag atc aaa ccc tac gtc cgg cac gat ctg     48
   Met Gln Thr Leu Tyr Pro Gln Ile Lys Pro Tyr Val Arg His Asp Leu
   1               5                   10                  15
   gcc gtc gat gaa acc cac acg ctg tat gtc gac gaa agt ggt tcc ccg     96
   Ala Val Asp Glu Thr His Thr Leu Tyr Val Asp Glu Ser Gly Ser Pro
               20                  25                  30
   caa ggt ttg ccc gtg gtc ttc atc cat ggc ggt ccc ggc gcc ggc tgc     144
   Gln Gly Leu Pro Val Val Phe Ile His Gly Gly Pro Gly Ala Gly Cys
           35                  40                  45
   gat gcc aat agc cgc tgc tat ttc gat ccg aac ctg tac cgc atc gtc     192
   Asp Ala Asn Ser Arg Cys Tyr Phe Asp Pro Asn Leu Tyr Arg Ile Val
       50                  55                  60
   acc ttt gac cag cgc ggc tgc ggg cgc tcc act ccg cgg gcc agc ctg     240
   Thr Phe Asp Gln Arg Gly Cys Gly Arg Ser Thr Pro Arg Ala Ser Leu
   65                  70                  75                  80
   gaa aac aac acc acc tgg gac ctg gtt gcc gac ctt gag cgc att cgc     288
   Glu Asn Asn Thr Thr Trp Asp Leu Val Ala Asp Leu Glu Arg Ile Arg
                   85                  90                  95
   gag cac ctg ggg att gaa aaa tgg gtg ctg ttc ggt ggt tcc tgg ggc    336
   Glu His Leu Gly Ile Glu Lys Trp Val Leu Phe Gly Gly Ser Trp Gly
               100                 105                 110
   tcg acc ctg gcc ctg gcc tat gca caa acc cac cct gat cgc gtg ctt    384
   Ser Thr Leu Ala Leu Ala Tyr Ala Gln Thr His Pro Asp Arg Val Leu
           115                 120                 125
   ggc ctg att gtg cgc ggc atc ttc ctg gcc cgc ccc cag gat atc cag    432
   Gly Leu Ile Val Arg Gly Ile Phe Leu Ala Arg Pro Gln Asp Ile Gln
       130                 135                 140
   tgg ttc tac cag gcc ggc gcg agc cgc ctg ttc ccg gac tac tgg cag    480
   Trp Phe Tyr Gln Ala Gly Ala Ser Arg Leu Phe Pro Asp Tyr Trp Gln
   145                 150                 155                 160
   gac tac atc gcg cca atc ccg gcg gaa gag cgc cac gac atg atc agc    528
   Asp Tyr Ile Ala Pro Ile Pro Ala Glu Glu Arg His Asp Met Ile Ser
                   165                 170                 175
   gcc tac cac aag cgc ctg acc ggc aat gac cag atc gcc cag atg cat    576
   Ala Tyr His Lys Arg Leu Thr Gly Asn Asp Gln Ile Ala Gln Met His
               180                 185                 190
   gcc gcc aag gcc tgg tcc acc tgg gaa ggc cgc atg ctc ggc ctg tgc    624
   Ala Ala Lys Ala Trp Ser Thr Trp Glu Gly Arg Met Leu Gly Leu Cys
           195                 200                 205
   ccc agc ccg cag ctg atc gag cgc ttc tcc gag ccc cag cgc gcg ttg    672
   Pro Ser Pro Gln Leu Ile Glu Arg Phe Ser Glu Pro Gln Arg Ala Leu
       210                 215                 220
   tcg att gcg cgc atc gag tgc cac tac ttc acc aat aac tcg ttc ctg    720
   Ser Ile Ala Arg Ile Glu Cys His Tyr Phe Thr Asn Asn Ser Phe Leu
   225                 230                 235                 240
   gag ccc aac cag ctg att cgc gat atg cac aag atc gcc cat ctg ccg    768
   Glu Pro Asn Gln Leu Ile Arg Asp Met His Lys Ile Ala His Leu Pro
                   245                 250                 255
   ggg atc atc gtg cat ggc cgc tac gat atg atc tgc ccg ctg gat aat    816
   Gly Ile Ile Val His Gly Arg Tyr Asp Met Ile Cys Pro Leu Asp Asn
   260 265 270
   gcc tgg gag ctg cac cag gcc tgg ccg aac agt gag ttg cag gtg atc 864
   Ala Trp Glu Leu His Gln Ala Trp Pro Asn Ser Glu Leu Gln Val Ile
   275 280 285
   cgc gag gcg ggc cac gcg gcg tcc gag ccg ggc atc acc gat gcg ctg 912
   Arg Glu Ala Gly His Ala Ala Ser Glu Pro Gly Ile Thr Asp Ala Leu
   290 295 300
   gtg cgt gcg gcg ggc gat atg gca cga cgc ctg ctt gat ctg ccc cct 960
   Val Arg Ala Ala Gly Asp Met Ala Arg Arg Leu Leu Asp Leu Pro Pro
   305 310 315 320
   gaa gaa gca 969
   Glu Glu Ala
<210> 23
   <211> 413
   <212> PRT
   <213> Corynebacterium glutamicum
<400> 23
   Met Thr Lys Thr Leu Gly Ser Leu Gln Leu Glu Glu Ile Thr Leu Thr
   1 5 10 15
   Leu Pro Leu Thr Glu Asp Val Ala Asp Glu Arg Thr Ile Asp Val Phe
   20 25 30
   Ala Arg Ile Ala Thr Arg Val Gly Gly Glu Asp Leu Pro Tyr Leu Val
   35 40 45
   Phe Leu Gln Gly Gly Pro Gly Asn Glu Ala Pro Arg Pro Ser Leu Asn
   50 55 60
   Pro Leu Asn Pro Asn Trp Leu Gly Val Ala Leu Glu Glu Tyr Arg Val
   65 70 75 80
   Val Met Leu Asp Gln Arg Gly Thr Gly Arg Ser Thr Pro Val Gly Asn
   85 90 95
   Asp Ile Leu Glu Lys Pro Thr Ala Glu Val Val Glu Tyr Leu Ser His
   100 105 110
   Leu Arg Ala Asp Gly Ile Val Arg Asp Ala Glu Ala Leu Arg Lys His
   115 120 125
   Leu Gly Val Asn Gln Trp Asn Leu Leu Gly Gln Ser Phe Gly Gly Phe
   130 135 140
   Thr Thr Leu His Tyr Leu Ser Arg His Ala Asp Ser Leu Asp Asn Val
   145 150 155 160
   Phe Ile Thr Gly Gly Leu Ser Ala Ile Asp Arg Pro Ala Glu Asp Val
   165 170 175
   Tyr Ala Asn Cys Tyr Asn Arg Met Arg Arg Asn Ser Glu Glu Phe Tyr
   180 185 190
   Arg Arg Phe Pro Gln Leu Arg Glu Thr Phe Arg Gly Leu Val Asn Arg
   195 200 205
   Ala Arg Ala Gly Glu Ile Val Leu Pro Thr Gly Glu Val Val Ser Glu
   210 215 220
   Thr Arg Leu Arg Ser Leu Gly His Leu Leu Gly Ser Asn Asp Gly Trp
   225 230 235 240
   Phe Asp Leu Tyr Asn Leu Leu Glu Leu Asp Pro Thr Ser Asn Ala Phe
   245 250 255
   Val His Asp Leu Ala Gly Leu Leu Pro Phe Gly Asn Arg Asn Pro Ile
   260 265 270
   Tyr Tyr Val Leu His Glu Ser Ser Tyr Ala Asp Gly Val Val Thr Asn
   275 280 285
   Trp Ala Ala Glu Arg Val Leu Pro Glu Asp Phe Arg Glu Asp Pro Thr
   290 295 300
   Leu Leu Thr Gly Glu His Val Phe Gln Glu Trp Thr Asp Thr Val Pro
   305 310 315 320
   Ser Leu Lys Pro Trp Lys Asp Val Ala Leu Ala Leu Ala Gln Gln Glu
   325 330 335
   Trp Pro Lys Leu Tyr Asp Ala Lys Ala Leu Glu Asn Ser Gln Ala Lys
   340 345 350
   Gly Ala Ala Ala Val Tyr Xaa Asn Asp Val Phe Val Pro Val Asp Tyr
   355 360 365
   Ser Leu Glu Thr Ala Gln His Leu Pro Gly Val Gln Leu Phe Ile Thr
   370 375 380
   Ser Gln His Glu His Asn Gly Leu Arg Ala Ser Ser Gly Ala Val Leu
   385 390 395 400
   Xaa His Leu Phe Asp Leu Ala His Gly Arg Glu Val Arg
   405 410
<210> 24
   <211> 1239
   <212> DNA
   <213> Corynebacterium glutamicum
<400> 24
   atg act aaa aca ctt ggt tcc ctt caa ctt gaa gaa att acc ttg acg 48
   Met Thr Lys Thr Leu Gly Ser Leu Gln Leu Glu Glu Ile Thr Leu Thr
   1 5 10 15
   ctc cct ctg act gaa gat gtg gcc gat gaa cgc acc att gat gtg ttc 96
   Leu Pro Leu Thr Glu Asp Val Ala Asp Glu Arg Thr Ile Asp Val Phe
   20 25 30
   gca cgc att gcc aca cgc gtc ggt ggg gaa gac ctt cca tat tta gta 144
   Ala Arg Ile Ala Thr Arg Val Gly Gly Glu Asp Leu Pro Tyr Leu Val
   35 40 45
   ttc ctg cag ggt ggg cct ggc aat gaa gct cca cgt cca agc ctt aat 192
   Phe Leu Gln Gly Gly Pro Gly Asn Glu Ala Pro Arg Pro Ser Leu Asn
   50 55 60
   ccc ctc aac ccc aat tgg ttg ggc gtg gcc ttg gag gaa tac cgc gtg 240
   Pro Leu Asn Pro Asn Trp Leu Gly Val Ala Leu Glu Glu Tyr Arg Val
    65                  70                  75                  80
   gtc atg ttg gat caa cgt ggc acc ggc cgt tcc acc cca gtg ggt aat     288
   Val Met Leu Asp Gln Arg Gly Thr Gly Arg Ser Thr Pro Val Gly Asn
                    85                  90                  95
   gat att ttg gaa aaa ccc aca gca gaa gta gtg gag tac tta tcc cac     336
   Asp Ile Leu Glu Lys Pro Thr Ala Glu Val Val Glu Tyr Leu Ser His
               100                 105                 110
   ctg cgc gca gat ggc att gtg cga gat gct gaa gcc ctg cgt aag cat     384
   Leu Arg Ala Asp Gly Ile Val Arg Asp Ala Glu Ala Leu Arg Lys His
           115                 120                 125
   ttg ggt gtg aat cag tgg aac ctt tta ggc cag tcc ttc gga ggt ttc     432
   Leu Gly Val Asn Gln Trp Asn Leu Leu Gly Gln Ser Phe Gly Gly Phe
       130                 135                 140
   acc acc ctg cat tac ttg tcc cgg cac gcc gat tcc ttg gac aac gtg     480
   Thr Thr Leu His Tyr Leu Ser Arg His Ala Asp Ser Leu Asp Asn Val
   145                 150                 155                 160
   ttt att acc ggc ggt ctc agc gct att gat cgc cca gca gaa gac gtg     528
   Phe Ile Thr Gly Gly Leu Ser Ala Ile Asp Arg Pro Ala Glu Asp Val
                   165                 170                 175
   tat gcc aac tgt tac aac cgc atg cgc cga aac tct gag gaa ttc tac     576
   Tyr Ala Asn Cys Tyr Asn Arg Met Arg Arg Asn Ser Glu Glu Phe Tyr
               180                 185                 190
   cgt cgc ttc ccg caa tta cgg gaa act ttc cga ggg ttg gtt aat cgt     624
   Arg Arg Phe Pro Gln Leu Arg Glu Thr Phe Arg Gly Leu Val Asn Arg
           195                 200                 205
   gct cgc gcc ggg gag att gtg ctt ccc acc ggc gaa gtt gtg tca gaa     672
   Ala Arg Ala Gly Glu Ile Val Leu Pro Thr Gly Glu Val Val Ser Glu
       210                 215                 220
   acc agg ctg cga tcc ctt ggt cac ttg ttg ggt agc aat gac ggc tgg     720
   Thr Arg Leu Arg Ser Leu Gly His Leu Leu Gly Ser Asn Asp Gly Trp
   225                 230                 235                 240
   ttt gat ctg tac aac ctg ctg gaa tta gat ccc acc tcc aac gct ttt   768
   Phe Asp Leu Tyr Asn Leu Leu Glu Leu Asp Pro Thr Ser Asn Ala Phe
                   245                 250                 255
   gtc cat gac ctg gca gga ctt ttg cct ttc ggc aac cgc aac cca att   816
   Val His Asp Leu Ala Gly Leu Leu Pro Phe Gly Asn Arg Asn Pro Ile
               260                 265                 270
   tat tac gtg ctc cat gag tcc tct tac gcc gac ggt gtg gtg aca aat   864
   Tyr Tyr Val Leu His Glu Ser Ser Tyr Ala Asp Gly Val Val Thr Asn
           275                 280                 285
   tgg gca gca gag cgt gtg ctt cca gag gat ttc cgc gag gat cca aca   912
   Trp Ala Ala Glu Arg Val Leu Pro Glu Asp Phe Arg Glu Asp Pro Thr
       290                 295                 300
   ctg ctc acc ggt gag cac gtg ttc cag gag tgg aca gac acc gtg ccg   960
   Leu Leu Thr Gly Glu His Val Phe Gln Glu Trp Thr Asp Thr Val Pro
   305                 310                 315                 320
   tcg ctc aag ccg tgg aag gac gtt gcc ctg gca ttg gct cag cag gaa   1008
   Ser Leu Lys Pro Trp Lys Asp Val Ala Leu Ala Leu Ala Gln Gln Glu
                   325                 330                 335
   tgg ccc aag ctt tat gat gcg aag gca ttg gaa aac tca cag gcc aag   1056
   Trp Pro Lys Leu Tyr Asp Ala Lys Ala Leu Glu Asn Ser Gln Ala Lys
               340                 345                 350
   ggc gct gca gca gtg tat ghc aat gac gtt ttc gtc cca gtg gat tac   1104
   Gly Ala Ala Ala Val Tyr Xaa Asn Asp Val Phe Val Pro Val Asp Tyr
           355                 360                 365
   tct ctg gaa acc gca caa cac ctg ccc ggt gtg cag ctg ttt atc acc   1152
   Ser Leu Glu Thr Ala Gln His Leu Pro Gly Val Gln Leu Phe Ile Thr
       370                 375                 380
   agc cag cat gaa cac aat gga ctt cgt gcc agc tca ggc gca gta ctg   1200
   Ser Gln His Glu His Asn Gly Leu Arg Ala Ser Ser Gly Ala Val Leu
   385                 390                 395                 400
   rag cac ctt ttc gat ctg gcc cac ggc cga gag gta cgc               1239
   Xaa His Leu Phe Asp Leu Ala His Gly Arg Glu Val Arg
                   405                 410
<210> 25
   <211> 27
   <212> DNA
   <213> Artificial Sequence
<220>
   <223> Description of Artificial Sequence: Synthetic DNA
<400> 25
   ttaaccatgg agagaaaaac agtattg 27
<210> 26
   <211> 30
   <212> DNA
   <213> Artificial Sequence
<220>
   <223> Description of Artificial Sequence: Synthetic DNA
<400> 28
   atatggatcc tactggcagc acatactttg 30
<210> 27
   <211> 21
   <212> DNA
   <213> Artificial Sequence
<220>
   <223> Description of Artificial Sequence: Synthetic DNA
<4.00> 27
   caccgcagac ggaggataca c 21
<210> 28
   <211> 22
   <212> DNA
   <213> Artificial Sequence
<220>
   <223> Description of Artificial Sequence: Synthetic DNA
<400> 28
   cggacgtcac ccaataatcg tg 22
<210> 29
   <211> 1428
   <212> DNA
   <213> Bacillus pumilus NRRL B-12025
<400> 29
   gtg ctt tca ttg agt aaa aaa act gta ctt gtc att gct gac tta gga    48
   Val Leu Ser Leu Ser Lys Lys Thr Val Leu Val Ile Ala Asp Leu Gly
    1               5                  10                  15
   ggg tgc ccg ccc cat atg ttt tat gaa agc gtg gcg gca tca tac cat    96
   Gly Cys Pro Pro His Met Phe Tyr Glu Ser Val Ala Ala Ser Tyr His
               20                  25                  30
   atc gtt tct tat atc cca aga ccc ttt gcg att aca aag gga cat gcc    144
   Ile Val Ser Tyr Ile Pro Arg Pro Phe Ala Ile Thr Lys Gly His Ala
           35                  40                  45
   gag cta atc gaa aaa tac tcc att gcc gtc atc aaa gac cgt gat tat    192
   Glu Leu Ile Glu Lys Tyr Ser Ile Ala Val Ile Lys Asp Arg Asp Tyr
       50                  55                  60
   ttt gag aca cac cct tct ttt gaa cac cct gat tct att tac tgg gca    240
   Phe Glu Thr His Pro Ser Phe Glu His Pro Asp Ser Ile Tyr Trp Ala
   65                  70                  75                  80
   cat gat gat tat cca aaa tca gaa gaa gaa gtt gtg gaa gac ttc att    288
   His Asp Asp Tyr Pro Lys Ser Glu Glu Glu Val Val Glu Asp Phe Ile
                   85                  90                  95
   cga gta gct tcc ttt ttc aaa gca gat gca atc acg acc aat aat gaa    336
   Arg Val Ala Ser Phe Phe Lys Ala Asp Ala Ile Thr Thr Asn Asn Glu
   100 105 110
   tta ttc att gca ccg atg gca aag gcc gct gaa cgt ctt ggg cta cga 384
   Leu Phe Ile Ala Pro Met Ala Lys Ala Ala Glu Arg Leu Gly Leu Arg
   115 120 125
   ggt gcc ggt gtc aag gca gcc gaa atg gcg cgt gat aaa agc caa atg 432
   Gly Ala Gly Val Lys Ala Ala Glu Met Ala Arg Asp Lys Ser Gln Met
   130 135 140
   agg gct gca ttc aat gcc tct ggc gtc aaa gcg gtg aaa act cag cct 480
   Arg Ala Ala Phe Asn Ala Ser Gly Val Lys Ala Val Lys Thr Gln Pro
   145 150 155 160
   gtc acg act tta tct gat ttc caa caa gcc att gag tct atc gga aca 528
   Val Thr Thr Leu Ser Asp Phe Gln Gln Ala Ile Glu Ser Ile Gly Thr
   165 170 175
   ccg ctc att tta aag cct aca tat tta gcc agt tct att ggc gtc acc 576
   Pro Leu Ile Leu Lys Pro Thr Tyr Leu Ala Ser Ser Ile Gly Val Thr
   180 185 190
   ttg ttt cat gac aaa gcc gga agt gat gac ttg ttt tta caa gta caa 624
   Leu Phe His Asp Lys Ala Gly Ser Asp Asp Leu Phe Leu Gln Val Gln
   195 200 205
   tcg tat ttg gaa acc ata cca gtc cca gac gct gtc acg tat gaa gca 672
   Ser Tyr Leu Glu Thr Ile Pro Val Pro Asp Ala Val Thr Tyr Glu Ala
   210 215 220
   ccg ttt gtc gct gaa aca tat tta gag ggt gct tac gaa gat tgg tat 720
   Pro Phe Val Ala Glu Thr Tyr Leu Glu Gly Ala Tyr Glu Asp Trp Tyr
   225 230 235 240
   gaa gac gaa gga tat gct gat tat gtc agt gta gaa ggg ctg gtc gta 768
   Glu Asp Glu Gly Tyr Ala Asp Tyr Val Ser Val Glu Gly Leu Val Val
   245 250 255
   gag ggc gaa tat ctc cct ttt gtc ata cat gat aaa acc cct caa atc 816
   Glu Gly Glu Tyr Leu Pro Phe Val Ile His Asp Lys Thr Pro Gln Ile
   260 265 270
   ggc ttt aca gaa acg gct cat atc act ccg acg atc tta gac aat gaa    864
   Gly Phe Thr Glu Thr Ala His Ile Thr Pro Thr Ile Leu Asp Asn Glu
               275                 280                 285
   gcc aag caa atc atc att gaa gca gca agg aag gca aat gaa ggg cta    912
   Ala Lys Gln Ile Ile Ile Glu Ala Ala Arg Lys Ala Asn Glu Gly Leu
           290                 295                 300
   ggt ctt gaa cat tgt gca acc cat aca gaa atc aaa ctc atg aaa aat    960
   Gly Leu Glu His Cys Ala Thr His Thr Glu Ile Lys Leu Met Lys Asn
   305                 310                 315                 320
   cga gaa act gga ctg atc gag gca gcg gct cga ttc gct ggc tgg aat   1008
   Arg Glu Thr Gly Leu Ile Glu Ala Ala Ala Arg Phe Ala Gly Trp Asn
                   325                 330                 335
   atg atc ccg aat att aaa aaa gtc ttt ggc gtc gat atg gcg aag cta   1056
   Met Ile Pro Asn Ile Lys Lys Val Phe Gly Val Asp Met Ala Lys Leu
               340                 345                 350
   ttg att gat gta tta gtt gat ggt aaa aag gct gta ctg cca aaa cag   1104
   Leu Ile Asp Val Leu Val Asp Gly Lys Lys Ala Val Leu Pro Lys Gln
           355                 360                 365
   ctg ctt tct gga cat aca ttt tat gta gcg gac tgc cac ctg tac cct   1152
   Leu Leu Ser Gly His Thr Phe Tyr Val Ala Asp Cys His Leu Tyr Pro
       370                 375                 380
   cag cat ttt aaa gag agt ggg ctt atc ccg cct gaa gcc aca cat att   1200
   Gln His Phe Lys Glu Ser Gly Leu Ile Pro Pro Glu Ala Thr His Ile
   385                 390                 395                 400
   acc att gat cat gtg tct att ccg cag gaa gca ttc gtt gga gat act   1248
   Thr Ile Asp His Val Ser Ile Pro Gln Glu Ala Phe Val Gly Asp Thr
                   405                 410                 415
   gcg att gtc agt caa tca ttc cct gcc aaa ggg act att gtg gat ctt   1296
   Ala Ile Val Ser Gln Ser Phe Pro Ala Lys Gly Thr Ile Val Asp Leu
               420                 425                 430
   gaa tta ttt gaa gct ttt aat gga atc gta tct ctt gaa tta aaa gga   1344
   Glu Leu Phe Glu Ala Phe Asn Gly Ile Val Ser Leu Glu Leu Lys Gly
   435 440 445
   tca tcc tca caa gat gtt gcc gcg tcc atc cgc aac att cag aaa cag 1392
   Ser Ser Ser Gln Asp Val Ala Ala Ser Ile Arg Asn Ile Gln Lys Gln
   450 455 460
   gca acg att cag tta atg gat gaa tta gtg aag gga 1428
   Ala Thr Ile Gln Leu Met Asp Glu Leu Val Lys Gly
   465 470 475
<210> 30
   <211> 1416
   <212> DNA
   <213> Bacillus subtilis ATCC 15245 and Bacillus subtilis IAM 1033
<400> 30
   atg gag aga aaa aca gta ttg gtc atc gct gat ctt gga ggc tgc ccg 48
   Met Glu Arg Lys Thr Val Leu Val Ile Ala Asp Leu Gly Gly Cys Pro
   1 5 10 15
   ccg cac atg ttt tat aaa agc gct gct gaa aaa tat aac ctg gtc agc 96
   Pro His Met Phe Tyr Lys Ser Ala Ala Glu Lys Tyr Asn Leu Val Ser
   20 25 30
   ttt att cca aga cct ttt gca att aca gcc tcc cat gca gca ttg att 144
   Phe Ile Pro Arg Pro Phe Ala Ile Thr Ala Ser His Ala Ala Leu Ile
   35 40 45
   gaa aaa tac tcg gtc gcg gtc ata aaa gat aaa gac tat ttt aag agt 192
   Glu Lys Tyr Ser Val Ala Val Ile Lys Asp Lys Asp Tyr Phe Lys Ser
   50 55 60
   tta gct gat ttt gag cat cct gat tcc att tat tgg gcg cat gag gat 240
   Leu Ala Asp Phe Glu His Pro Asp Ser Ile Tyr Trp Ala His Glu Asp
   65 70 75 80
   cat aac aag cct gag gaa gag gtc gtc gag caa atc gtc aag gtt gcc 288
   His Asn Lys Pro Glu Glu Glu Val Val Glu Gln Ile Val Lys Val Ala
   85 90 95
   gaa atg ttt ggg gcg gat gcc atc aca aca aac aat gaa tta ttc att 336
   Glu Met Phe Gly Ala Asp Ala Ile Thr Thr Asn Asn Glu Leu Phe Ile
               100                 105                 110
   gct ccg atg gcg aaa gcc tgt gaa cgt ctg ggc ctg aga ggt gcc ggc     384
   Ala Pro Met Ala Lys Ala Cys Glu Arg Leu Gly Leu Arg Gly Ala Gly
           115                 120                 125
   gtg cag gca gcc gaa aat gcc aga gat aaa aat aaa atg agg gac gct     432
   Val Gln Ala Ala Glu Asn Ala Arg Asp Lys Asn Lys Met Arg Asp Ala
       130                 135                 140
   ttt aat aag gcc gga gtc aaa tcg atc aaa aac aaa cga gtc aca act     480
   Phe Asn Lys Ala Gly Val Lys Ser Ile Lys Asn Lys Arg Val Thr Thr
   145                 150                 155                 160
   ctt gaa gat ttc cgt gct gct ctt gaa gag atc ggc aca cct ctt atc     528
   Leu Glu Asp Phe Arg Ala Ala Leu Glu Glu Ile Gly Thr Pro Leu Ile
                   165                 170                 175
   tta aag cct aca tac tta gcg agt tca atc ggt gta acg ctg att acg     576
   Leu Lys Pro Thr Tyr Leu Ala Ser Ser Ile Gly Val Thr Leu Ile Thr
               180                 185                 190
   gac act gag acg gca gaa gat gaa ttt aac aga gtc aat gac tat ctg     624
   Asp Thr Glu Thr Ala Glu Asp Glu Phe Asn Arg Val Asn Asp Tyr Leu
           195                 200                 205
   aaa tca att aac gtg cca aag gcg gtt acg ttt gaa gcg ccg ttt atc     672
   Lys Ser Ile Asn Val Pro Lys Ala Val Thr Phe Glu Ala Pro Phe Ile
       210                 215                 220
   gct gaa gaa ttt tta cag ggt gag tac gga gac tgg tat caa aca gaa     720
   Ala Glu Glu Phe Leu Gln Gly Glu Tyr Gly Asp Trp Tyr Gln Thr Glu
   225                 230                 235                 240
   ggg tac tcc gac tat atc agt ata gaa ggc atc atg gct gac ggt gag     768
   Gly Tyr Ser Asp Tyr Ile Ser Ile Glu Gly Ile Met Ala Asp Gly Glu
                   245                 250                 255
   tat ttc ccg atc gcc att cat gat aaa acg ccg caa atc ggg ttt aca     816
   Tyr Phe Pro Ile Ala Ile His Asp Lys Thr Pro Gln Ile Gly Phe Thr
               260                 265                 270
   gag aca tcc cac att acg ccg tcc att ctg gat gaa gag gca aaa aag 864
   Glu Thr Ser His Ile Thr Pro Ser Ile Leu Asp Glu Glu Ala Lys Lys
   275 280 285
   aaa att gtc gaa gct gcc aaa aag gca aat gaa ggg ctt ggc ctg caa 912
   Lys Ile Val Glu Ala Ala Lys Lys Ala Asn Glu Gly Leu Gly Leu Gln
   290 295 300
   aat tgc gca aca cat aca gag atc aag cta atg aaa aac aga gaa ccg 960
   Asn Cys Ala Thr His Thr Glu Ile Lys Leu Met Lys Asn Arg Glu Pro
   305 310 315 320
   ggt tta ata gag tcg gca gcc aga ttc gca ggc tgg aat atg att cct 1008
   Gly Leu Ile Glu Ser Ala Ala Arg Phe Ala Gly Trp Asn Met Ile Pro
   325 330 335
   aat att aaa aag gtc ttt ggc ctt gat atg gcg caa tta tta tta gat 1056
   Asn Ile Lys Lys Val Phe Gly Leu Asp Met Ala Gln Leu Leu Leu Asp
   340 345 350
   gtc ctc tgt ttc gga aaa gac gcc gat ctg ccg gac gga tta ttg gat 1104
   Val Leu Cys Phe Gly Lys Asp Ala Asp Leu Pro Asp Gly Leu Leu Asp
   355 360 365
   caa gag cct tat tat gtt gcc gac tgc cat ttg tac ccg cag cat ttc 1152
   Gln Glu Pro Tyr Tyr Val Ala Asp Cys His Leu Tyr Pro Gln His Phe
   370 375 380
   aaa caa aat ggc cag att cca gaa acc gct gag gat ttg gtc att gaa 1200
   Lys Gln Asn Gly Gln Ile Pro Glu Thr Ala Glu Asp Leu Val Ile Glu
   385 390 395 400
   gcg atc gat att ccg gac ggg ctt tta aaa ggg gat act gaa atc gtt 1248
   Ala Ile Asp Ile Pro Asp Gly Leu Leu Lys Gly Asp Thr Glu Ile Val
   405 410 415
   tca ttt tca gcc gca gca cca ggc act tca gtt gat ttg aca ttg ttt 1296
   Ser Phe Ser Ala Ala Ala Pro Gly Thr Ser Val Asp Leu Thr Leu Phe
   420 425 430
   gaa gct ttc aat tcc att gct gca ttt gaa ctg aaa ggc agt aat tca 1344
   Glu Ala Phe Asn Ser Ile Ala Ala Phe Glu Leu Lys Gly Ser Asn Ser
   435 440 445
   cag gat gtg gct gaa tca atc aga caa att cag cag cat gca aag ctg 1392
   Gln Asp Val Ala Glu Ser Ile Arg Gln Ile Gln Gln His Ala Lys Leu
   450 455 460
   acg gca aag tat gtg ctg cca gta 1416
   Thr Ala Lys Tyr Val Leu Pro Val
   465 470
<210> 31
   <211> 23
   <212> DNA
   <213> Artificial Sequence
<220>
   <223> Description of Artificial Sequence: Synthetic DNA
<400> 31
   ccgatggcra aagcstgtra acg 23
<210> 32
   <211> 21
   <212> DNA
   <213> Artificial Sequence
<220>
   <223> Description of Artificial Sequence: Synthetic DNA
<400> 32
   cggcagatcr gcdtcttttc c 21
<210> 33
   <211> 26
   <212> DNA
   <213> Artificial Sequence
<220>
   <223> Description of Artificial Sequence: Synthetic DNA
<400> 33
   gctaggtctt gaacattgtg caaccc 26
<210> 34
   <211> 23
   <212> DNA
   <213> Artificial Sequence
<220>
   <223> Description of Artificial Sequence: Synthetic DNA
<400> 34
   ggtgttccga tagactcaat ggc 23
<210> 35
   <211> 476
   <212> PRT
   <213> Bacillus pumilus NRRL B-12025
<400> 35
   Val Leu Ser Leu Ser Lys Lys Thr Val Leu Val Ile Ala Asp Leu Gly
   1               5                   10                  15
   Gly Cys Pro Pro His Met Phe Tyr Glu Ser Val Ala Ala Ser Tyr His
               20                  25                  30
   Ile Val Ser Tyr Ile Pro Arg Pro Phe Ala Ile Thr Lys Gly His Ala
           35                  40                  45
   Glu Leu Ile Glu Lys Tyr Ser Ile Ala Val Ile Lys Asp Arg Asp Tyr
       50                  55                  60
   Phe Glu Thr His Pro Ser Phe Glu His Pro Asp Ser Ile Tyr Trp Ala
   65                  70                  75                  80
   His Asp Asp Tyr Pro Lys Ser Glu Glu Glu Val Val Glu Asp Phe Ile
                   85                  90                  95
   Arg Val Ala Ser Phe Phe Lys Ala Asp Ala Ile Thr Thr Asn Asn Glu
               100                 105                 110
   Leu Phe Ile Ala Pro Met Ala Lys Ala Ala Glu Arg Leu Gly Leu Arg
   115 120 125
   Gly Ala Gly Val Lys Ala Ala Glu Met Ala Arg Asp Lys Ser Gln Met
   130 135 140
   Arg Ala Ala Phe Asn Ala Ser Gly Val Lys Ala Val Lys Thr Gln Pro
   145 150 155 160
   Val Thr Thr Leu Ser Asp Phe Gln Gln Ala Ile Glu Ser Ile Gly Thr
   165 170 175
   Pro Leu Ile Leu Lys Pro Thr Tyr Leu Ala Ser Ser Ile Gly Val Thr
   180 185 190
   Leu Phe His Asp Lys Ala Gly Ser Asp Asp Leu Phe Leu Gln Val Gln
   195 200 205
   Ser Tyr Leu Glu Thr Ile Pro Val Pro Asp Ala Val Thr Tyr Glu Ala
   210 215 220
   Pro Phe Val Ala Glu Thr Tyr Leu Glu Gly Ala Tyr Glu Asp Trp Tyr
   225 230 235 240
   Glu Asp Glu Gly Tyr Ala Asp Tyr Val Ser Val Glu Gly Leu Val Val
   245 250 255
   Glu Gly Glu Tyr Leu Pro Phe Val Ile His Asp Lys Thr Pro Gln Ile
   260 265 270
   Gly Phe Thr Glu Thr Ala His Ile Thr Pro Thr Ile Leu Asp Asn Glu
   275 280 285
   Ala Lys Gln Ile Ile Ile Glu Ala Ala Arg Lys Ala Asn Glu Gly Leu
   290 295 300
   Gly Leu Glu His Cys Ala Thr His Thr Glu Ile Lys Leu Met Lys Asn
   305 310 315 320
   Arg Glu Thr Gly Leu Ile Glu Ala Ala Ala Arg Phe Ala Gly Trp Asn
   325 330 335
   Met Ile Pro Asn Ile Lys Lys Val Phe Gly Val Asp Met Ala Lys Leu
   340 345 350
   Leu Ile Asp Val Leu Val Asp Gly Lys Lys Ala Val Leu Pro Lys Gln
   355 360 365
   Leu Leu Ser Gly His Thr Phe Tyr Val Ala Asp Cys His Leu Tyr Pro
   370 375 380
   Gln His Phe Lys Glu Ser Gly Leu Ile Pro Pro Glu Ala Thr His Ile
   385 390 395 400
   Thr Ile Asp His Val Ser Ile Pro Gln Glu Ala Phe Val Gly Asp Thr
   405 410 415
   Ala Ile Val Ser Gln Ser Phe Pro Ala Lys Gly Thr Ile Val Asp Leu
   420 425 430
   Glu Leu Phe Glu Ala Phe Asn Gly Ile Val Ser Leu Glu Leu Lys Gly
   435 440 445
   Ser Ser Ser Gln Asp Val Ala Ala Ser Ile Arg Asn Ile Gln Lys Gln
   450 455 460
   Ala Thr Ile Gln Leu Met Asp Glu Leu Val Lys Gly
   465 470 475
<210> 36
   <211> 44
   <212> DNA
   <213> Artificial Sequence
<220>
   <223> Description of Artificial Sequence: Synthetic DNA
<400> 36
   catgccatgg agaaaaaaac tgtacttgtc attgctgact tagg 44
<210> 37
   <211> 38
   <212> DNA
   <213> Artificial Sequence
<220>
   <223> Description of Artificial Sequence: Synthetic DNA
<400> 37
   cgcggatccc ttcactaatt catccattaa ctgaatcg 38

## Description

### Background of the Invention

The present invention relates to a process for producing a dipeptide using a microorganism producing a dipeptide.

Dipeptides are compounds that are important as foods, pharmaceuticals, cosmetics and the like.

Known methods for producing dipeptides include methods which comprise causing certain kinds of microorganisms or proline iminopeptidase to act on L-amino acid esters and L-amino acids (see WO03/010189 pamphlet and WO03/010307 pamphlet) and a method which comprises causing L-amino acid amide hydrolase to act on L-amino acid amides and L-amino acids (see WO03/010187 pamphlet).

According to the above methods, better production cost and efficiency are achieved by using, as an enzyme source, the cells of a microorganism which produces L-amino acid amide hydrolase or proline iminopeptidase rather than isolated L-amino acid amide hydrolase or proline iminopeptidase. However, use of microbial cells as an enzyme source involves the problem that the formed dipeptide is decomposed by peptidase possessed by the microorganism. Microorganisms have various dipeptidase activities as evidenced, for example, by the fact that more than 20 genes that are presumed to be peptidases of Escherichia coli, a typical microorganism, are registered with the database of DNA Data Bank of Japan (DDBJ).

Therefore, when a dipeptide is produced by using a culture of a microorganism having the ability to produce a dipeptide or a treated matter thereof as an enzyme source, it is considered that the reaction to decompose the dipeptide occurs simultaneously with the reaction to form the dipeptide, leading to remarkably poor yield of the dipeptide. In WO03/010307 pamphlet, solution of this problem is attempted by the addition of a metal enzyme inhibitor. However, productivity of the dipeptide is not sufficient.

An object of the present invention is to provide a process for producing a dipeptide using a microorganism producing a dipeptide.

### Summary of the Invention

The present invention relates to a process for producing a dipeptide using a culture of a microorganism or a treated matter thereof, as specified in the present claims.

### Brief Description of the Drawings

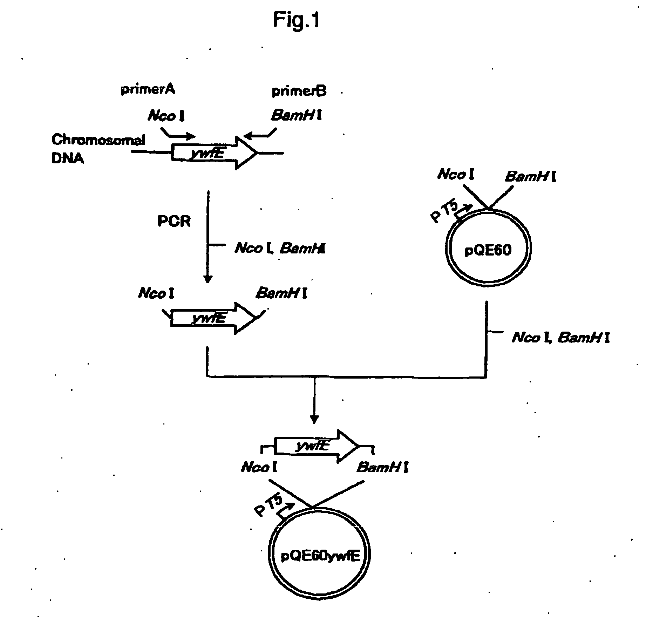

Fig. 1 shows the steps for constructing His-tagged ywfE expression vector pQE60ywfE.
Explanation of Symbol
PT5: T5 promoter

### Detailed Description of the Invention

### 1. Microorganisms Used in the Process for Producing a Dipeptide

There is not any specific restriction as to the microorganism used in the process for producing a dipeptide of the present invention, so long as it has the ability to produce a dipeptide from one or more kinds of substances selected from the group consisting of L-amino acid amides, L-amino acid esters and amino acids.

Suitable microorganisms include those belonging to the genera Achromobacter, Acinetobacter, Aeromonas, Agrobacterium, Alcaligenes, Arthrobacter, Beijerinckia, Brevibacterium, Clavibacter, Chryseobacterium, Escherichia, Enterobacter, Erwinia, Flavobacterium, Kluyvera, Microbacterium, Micrococcus, Mycoplana, Pantoea. Propionibacterium, Listonella, Rhizobium, Rhodococcus, Salmonella, Sarcina, Serratia, Staphylococcus, Stenotrophomonas, Streptomyces, Vibrio, Xanthomonas, Bullera, Candida, Cryptococcus, Filobasidium, Geotrichum, Pachysolen, Rhodosporidium, Rhodotorula, Saccharomyces, Sporobolomyces, Tremella, Torulaspora, Torulopsis, Gluconacetobacter, Acetobacter, Gluconobacter, Asaia, Zucharibacter, Actinomadura, Kitasatosporia, Micromonospora, Nocardia, Oerskovia, Saccharothrix, Streptoverticillium, Hafnia, Lactobacillus, Neisseria, Thermoplasma, Corynebacterium, Pseudomonas, Bacillus, Trichosporon and Sterigmatomyces.

Examples of one or more kinds of substances selected from the group consisting of amino acid amides, amino acid esters and amino acids are one or more kinds of amino acids; one or more kinds of amino acid esters and one or more kinds of amino acids; and one or more kinds of amino acid amides and one or more kinds of amino acids.

### (1) Microorganisms Having the Ability to Produce a Dipeptide from One or More Kinds of Amino Acids

The microorganisms having the ability to produce a dipeptide from one or more kinds of amino acids may be any microorganisms having such ability and include microorganisms producing a protein selected from the group consisting of non-ribosomal peptide synthetase (hereinafter referred to as NRPS), D-alanyl-D-alanine (D-Ala-D-Ala) ligase and bacilysin synthetase. Examples of the microorganisms producing NRPS include procaryotes such as microorganisms of the genus Bacillus, microorganisms producing BacA, BacB and BacC (GenBank AF007865), microorganisms producing TycA, TycB and TycC (GenBank. AF004835), microorganisms producing PcbAB (GenBank M57425), and microorganisms producing a protein having an amino acid sequence which has at least 80% or more, preferably at least 90% or more, more preferably at least 95% or more, even more preferably at least 98% or more and further preferably at least 99% or more homology to the amino acid sequence of any protein selected from BacA, BacB, BacC, TycA, TycB, TycC and PcbAB and having NRPS activity.

Examples of the microorganisms producing D-Ala-D-Ala ligase include procaryotes forming peptidoglycans, microorganisms producing DdlA (GenBank accession No. M58467), microorganisms producing DdlB (GenBank accession No. AE000118), microorganisms producing DdlC (GenBank accession No. D88151), and microorganisms producing a protein consisting of an amino acid sequence wherein one or more amino acid residues are deleted, substituted or added in the amino acid sequence of any protein selected from DdlA, DdlB and DdlC and having D-Ala-D-Ala ligase activity.

Examples of the microorganisms producing bacilysin synthetase include microorganisms belonging to the genus Bacillus, preferably, Bacillus subtilis, Bacillus amyloliquefaciens, Bacillus coagulans, Bacillus licheniformis, Bacillus megaterium and Bacillus pumilus, more preferably, Bacillus subtilis 168 (ATCC 23857), Bacillus subtilis ATCC 15245, Bacillus subtilis ATCC 6633, Bacillus subtilis IAM 1213, Bacillus subtilis IAM 1107, Bacillus subtilis IAM 1214, Bacillus subtilis ATCC 9466, Bacillus subtilis IAM 1033, Bacillus subtilis ATCC 21555 and Bacillus amyloliquefaciens IFO 3022, and microorganisms producing a protein selected from the following [1] to [4]:
[1] a protein having the amino acid sequence shown in any of SEQ ID NOS: 1 to 7 and 35;
[2] a protein consisting of an amino acid sequence wherein one or more amino acid residues are deleted, substituted or added in the amino acid sequence shown in any of SEQ ID NOS: 1 to 7 and 35 and having the activity to synthesize a dipeptide;
[3] a protein consisting of an amino acid sequence which has at least 65% or more, preferably at least 80% or more, more preferably at least 90% or more, even more preferably at least 95% or more, further preferably at least 98% or more and most preferably at least 99% or more homology to the amino acid sequence shown in any of SEQ ID NOS: 1 to 7 and 35 and having the activity to synthesize a dipeptide; and
[4] a protein comprising an amino acid sequence which has 80% or more, preferably at least 90% or more, more preferably at least 95% or more, even more preferably at least 98% or more and further preferably at least 99% or more homology to the amino acid sequence shown in SEQ ID NO: 15 and having the activity to synthesize a dipeptide.

The amino acid sequence shown in SEQ ID NO: 15 is a region conserved among the proteins having the amino acid sequences shown in SEQ ID NOS: 1 to 7 and is also a region corresponding to the consensus sequence of proteins having Ala-Ala ligase activity derived from various microorganisms.

Therefore, microorganisms producing a protein comprising an amino acid sequence which has at least 80% or more, preferably 90% or more, more preferably 95% or more, even more preferably at least 98% or more and further preferably at least 99% or more homology to the amino acid sequence shown in SEQ ID NO: 15 and having the activity to synthesize a dipeptide are also included in the dipeptide-producing microorganisms.

In order that the protein comprising an amino acid sequence which has at least 80% or more, preferably 90% or more, more preferably at least 95% or more, even more preferably at least 98% or more and further preferably at least 99% or more homology to the amino acid sequence shown in SEQ ID NO: 15 may have the activity to synthesize a dipeptide, it is desirable that the homology of its amino acid sequence to the amino acid sequence shown in any of SEQ ID NOS: 1 to 7 is at least 65% or more, preferably at least 80% or more, more preferably at least 90% or more, even more preferably at least 95% or more, further preferably at least 98% or more and most preferably at least 99% or more.

### (2) Microorganisms Having the Ability to Produce a Dipeptide from One or More Kinds of Amino Acid Esters and One or More Kinds of Amino Acids

The microorganisms having the ability to produce a dipeptide from one or more kinds of amino acid esters and one or more kinds of amino acids may be any microorganisms having such ability and include those belonging to the genera Achromobacter, Acinetobacter, Aeromonas, Agrobacterium, Alcaligenes, Arthrobacter, Beijerinckia, Brevibacterium, Clavibacter, Chryseobacterium, Escherichia, Enterobacter, Erwinia, Flavobacterium, Kluyvera, Microbacterium, Micrococcus, Mycoplana, Pantoea, Propionibacterium, Listonella, Rhizobium, Rhodococcus, Salmonella, Sarcina, Serratia, Staphylococcus, Stenotrophomonas, Streptomyces, Vibrio, Xanthomonas, Bullera, Candida, Cryptococcus, Filobasidium, Geotrichum, Pachysolen, Rhodosporidium, Rhodotorula, Saccharomyces, Sporobolomyces, Tremella, Torulaspora, Torulopsis, Gluconacetobacter, Acetobacter, Gluconobacter, Asaia, Zucharibacter, Actinomadura, Kitasatosporia, Micromonospora, Nocardia, Oerskovia, Saccharothrix, Streptoverticillium, Hafnia, Lactobacillus, Neisseria, Thermoplasma, Corynebacterium, Pseudomonas and Bacillus.

Specific examples of the above microorganisms include Achromobacter delmarvae (FERM BP-6988), Acinetobacter johnsonii (ATCC 9036), Aeromonas salmonicida (ATCC 14174), Agrobacterium tumefaciens (IFO 3058). Alcaligenes faecalis (ATCC 8750), Arthrobacter citreus (ATCC 11624), Beijerinckia indica (ATCC 9037), Brevibacterium roseum (ATCC 13825), Clavibacter michiganense (ATCC 7429), Chryseobacterium meningosepticum (ATCC 13253), Escherichia coli (ATCC 13071), Enterobacter aerogenes (ATCC 13048), Erwinia amylovora (IFO 12687), Flavobacterium resinovorum (ATCC 12524), Kluyvera citrophila (FERM BP-6564), Microbacterium imperiale (ATCC 8365). Micrococcus luteus (ATCC 11880), Mycoplana bullata (ATCC 4278), Pantoea ananatis (ATCC 23822), Propionibacterium shermanii (FERM BP-8100), Listonella anguillarum (ATCC 19264), Rhizobium radiobacter (ATCC 4720), Rhodococcus rhodochrous (ATCC 21198), Salmonella typhimurium (FERM BP-6566), Sarcina lutea (FERM BP-6562), Serratia grimesii (ATCC 14460), Staphylococcus aureus (ATCC 12600), Stenotrophomonas maltophilia (ATCC 13270), Streptomyces lavendulae (ATCC 11924), Vibrio tyrogenes (FERM BP-5848), Xanthomonas maltophilia (FERM BP-5568), Bullera alba (FERM BP-8099), Candida krusei (IFO 0011), Cryptococcus terreus (IFO 0727), Filobasidium capsuligenum (IFO 1119), Geotrichum amycelicum (ATCC 56046), Pachysolen tannophilus (IFO 1007), Rhodosporidium diobovatum (IFO 1829), Rhodotorula minuta (IFO 0879), Saccharomyces unisporus (IFO 0724), Sporobolomyces salmonicolor (IFO 1038), Tremella foliacea (IFO 9297), Torulaspora delbrueckii (IFO 1083), Torulopsis ingeniosa (FERM BP-8098), Gluconacetobacter liquefaciens (IFO 12388). Acetobacter orleanensis (IFO 3223), Acetobacter pasteurianus (ATCC 9325). Gluconobacter oxydans (ATCC 621). Gluconobacter oxydans (IFO 3171), Gluconacetobacter hansenii (JCM 7643), Asaia ethanolifaciens (FERM BP-6751), Zucharibacter floricola (FREM BP-6752). Actinomadura madurae (ATCC 19425). Kitasatosporia griseola (IFO 14371), Micromonospora chersina (ATCC 53710), Nocardia globerula (ATCC 21602), Oerskovia turbata (FERM BP-8122), Saccharothrix australiensis (IFO 14444). Streptoverticillium mobaraensis (IFO 13819), Streptomyces plicatus [Biochem. Biophys. Res. Commun., 184, 1250 (1992)], Corynebacterium variabilis [J. Appl. Microbiol., 90, 449 (2001)], Arthrobacter nicotianae [FEMS Microbiol. Lett., 78, 191 (1999)], Escherichia coli (Japanese Published Unexamined Patent Application No. 113887/90). Flavobacterium meningosepticum [Arch. Biochem. Biophys., 336, 35 (1996)]. Hafnia alvei [J. Biochem., 119, 468 (1996)], Lactobacillus delbrueckii [Microbiology, 140, 527 (1994)], Bacillus coagulans [J. Bacteriol., 174, 7919 (1994)], Aeromonas sobria [J. Biochem., 116, 818 (1994)], Xanthomonas campestris (Japanese Published Unexamined Patent Application No. 121860/97), Neisseria gonorrhoeae [Mol. Microbiol., 9, 1203 (1993)], Propionibacterium freudenreichii [Appl. Environ. Microbiol., 64, 4736 (1998)], Serratia marcescens [J. Biochem., 122, 601 (1997)], Thermoplasma acidophilum [FEBS Lett., 398, 101 (1996)], Pseudomonas aeruginosa [Nature, 406, 959 (2000)], Bacillus subtilis (ATCC 6633), Bacillus coagulans EK01 [J. Bacteriol., 174, 7919 (1992)], Corynebacterium glutamicum (ATCC 13286), Pseudomonas putida (FERM BP-8101), Pseudomonas putida (ATCC 12633) and Pseudomonas putida (FERM BP-8123).

The microorganisms having the ability to produce a dipeptide from one or more kinds of L-amino acid esters and one or more kinds of amino acids also include microorganisms having the ability to produce proline iminopeptidase. Examples of such microorganisms are those belonging to the genera Streptomyces, Arthrobacter, Escherichia, Flavobacterium, Hafnia, Lactobacillus, Aeromonas, Xanthomonas, Neisseria, Propionibacterium, Serratia, Thermoplasma, Corynebacterium, Pseudomonas and Bacillus.

Specific examples of the microorganisms are Streptomyces plicatus [Biochem. Biophys. Res. Commun., 184, 1250 (1992)], Arthrobacter nicotianae [FEMS Microbiol. Lett., 78, 191 (1999)], Escherichia coli (Japanese Published Unexamined Patent Application No. 113887/90), Flavobacterium meningosepticum [Arch. Biochem. Biophys., 336, 35 (1996)], Hafnia alvei [J. Biochem., 119, 468 (1996)], Lactobacillus delbrueckii [Microbiology, 140, 527 (1994)], Bacillus coagulans [J. Bacteriol., 174, 7919 (1994)], Aeromonas sobria [J. Biochem., 116, 818 (1994)], Xanthomonas campestris (Japanese Published Unexamined Patent Application No. 121860/97), Neisseria gonorrhoeae [Mol. Microbiol., 9, 1203 (1993)], Propionibacterium freudenreichii [Appl. Environ. Microbiol., 64, 4736 (1998)]. Serratia marcescens [J. Biochem., 122, 601 (1997)], Thermoplasma acidophilum [FEBS Lett., 398, 101 (1996)], Pseudomonas aeruginosa [Nature, 406, 959 (2000)], Bacillus subtilis (ATCC 6633), Bacillus coagulans EK01 [J. Bacteriol., 174, 7919 (1992)], Corynebacterium variabilis [J. Appl. Microbiol., 90, 449 (2001)], Corynebacterium glutamicum (ATCC 13286), Pseudomonas putida (FERM BP-8101), Pseudomonas putida (ATCC 12633). Pseudomonas putida (FERM BP-8123) and microorganisms having the ability to produce a protein having the amino acid sequence shown in any of SEQ ID NOS: 17 to 19.

Further, the microorganisms having the ability to produce proline iminopeptidase include microorganisms having the ability to produce a protein of the following [1] or [2]:
[1] proline iminopeptidase derived from Streptomyces plicatus [Biochem. Biophys. Res. Commun., 184, 1250 (1992)], Arthrobacter nicotianae [FEMS Microbiol. Lett., 78, 191 (1999)], Escherichia coli (Japanese Published Unexamined Patent Application No. 113887/90), Flavobacterium meningosepticum [Arch. Biochem. Biophys., 336, 35 (1996)], Hafnia alvei [J. Biochem., 119, 468 (1996)], Lactobacillus delbrueckii [Microbiology. 140, 527 (1994)], Bacillus coagulans [J. Bacteriol., 174, 7919 (1994)], Aeromonas sobria [J. Biochem., 116, 818 (1994)]. Xanthomonas campestris (Japanese Published Unexamined Patent Application No. 121860/97), Neisseria gonorrhoeae [Mol. Microbiol., 9, 1203 (1993)], Propionibacterium freudenreichii [Appl. Environ. Microbiol., 64, 4736 (1998)], Serratia marcescens [J. Biochem., 122, 601 (1997)], Thermoplasma acidophilum [FEBS Lett.. 398, 101 (1996)] and Pseudomonas aeruginosa [Nature, 406, 959 (2000)], and a protein consisting of an amino acid sequence wherein one or more amino acid residues are deleted, substituted or added in the amino acid sequence shown in any of SEQ ID NOS: 17 to 19 and having proline iminopeptidase activity; and
[2] a protein consisting of an amino acid sequence which has at least 80% or more, preferably at least 90% or more, more preferably at least 95% or more, even more preferably at least 98% or more and further preferably at least 99% or more homology to the amino acid sequence of any of the proline iminopeptidase and the protein of the above [1] and having proline iminopeptidase activity.

### (3) Microorganisms Having the Ability to Produce a Dipeptide from One or More Kinds of L-Amino Acid Amides and One or More Kinds of Amino Acids

The microorganisms having the ability to produce a dipeptide from one or more kinds of L-amino acid amides and one or more kinds of amino acids may be any microorganisms having such ability and include those belonging to the genera Bacillus, Corynebacterium, Erwinia, Rhodococcus, Chryseobacterium, Micrococcus, Pseudamonas, Cryptococcus, Trichosporon, Rhodosporidium, Sporobolomyces, Tremella, Torulaspora, Sterigmatomyces and Rhodotorula.

Specific examples of the microorganisms are Bacillus megaterium (FERM BP-8090), Corynebacterium glutamicum (ATCC 13286), Erwinia carotovora (FERM BP-8089), Rhodococcus rhodochrous (ATCC 19149), Chryseobacterium meningosepticum (ATCC 13253), Micrococcus luteus (ATCC 9341), Pseudomonas saccharophila (ATCC 15946), Cryptococcus albidus (IFO 0378), Trichosporon gracile (ATCC 24660), Rhodosporidium diobovatum (ATCC 22264), Sporobolomyces salmonicolor (IFO 1038), Tremella foliacea (IFO 9297), Torulaspora delbrueckii (IFO 1083), Sterigmatomyces elviae (IFO 1843) and Rhodotorula ingeniosa (ATCC 22993).

The microorganisms having the ability to produce a dipeptide from one or more kinds of L-amino acid amides and one or more kinds of amino acids also include microorganisms having the ability to produce L-amino acid amide hydrolase, and examples of the microorganisms are Corynebacterium glutamicum (ATCC 13286) and those having the ability to produce a protein having the amino acid sequence shown in SEQ ID NO: 23.

Further, the microorganisms having the ability to produce L-amino acid amide hydrolase include microorganisms having the ability to produce a protein of the following [1] or [2]:
[1] a protein consisting of an amino acid sequence wherein one or more amino acid residues are deleted, substituted or added in the amino acid sequence shown in SEQ ID NO: 23 and having L-amino acid amide hydrolase activity; and
[2] a protein consisting of an amino acid sequence which has at least 80% or more, preferably at least 90% or more, more preferably at least 95% or more, even more preferably at least 98% or more and further preferably at least 99% or more homology to the amino acid sequence shown in SEQ ID NO: 23 and having L-amino acid amide hydrolase activity.

The above protein consisting of an amino acid sequence wherein one or more amino acid residues are deleted, substituted or added and having the activity to synthesize a dipeptide can be obtained, for example, by introducing a site-directed mutation into DNA encoding a protein selected from a protein consisting of the amino acid sequence shown in any of SEQ ID NOS: 1 to 7 and 35, a protein consisting of the amino acid sequence shown in any of SEQ ID NOS: 17 to 19 and having proline iminopeptidase activity, and a protein consisting of the amino acid sequence shown in SEQ ID NO: 23 and having L-amino acid amide hydrolase activity by site-directed mutagenesis described in Molecular Cloning, A Laboratory Manual, Second Edition, Cold Spring Harbor Laboratory Press (1989) (hereinafter referred to as Molecular Cloning, Second Edition); Current Protocols in Molecular Biology, John Wiley & Sons (1987-1997) (hereinafter referred to as Current Protocols in Molecular Biology): Nucleic Acids Research, 10, 6487 (1982); Proc. Natl. Acad. Sci- USA, 79, 6409 (1982); Gene, 34, 315 (1985); Nucleic Acids Research, 13, 4431 (1985); Proc. Natl. Acad. Sci. USA, 82, 488 (1985), etc.

The number of amino acid residues which are deleted, substituted or added is not specifically limited so long as it is within the range where deletion, substitution or addition is possible by known methods such as the above site-directed mutagenesis. The suitable number is 1 to dozens, preferably 1 to 20, more preferably 1 to 10, further preferably 1 to 5.

The expression one or more amino acid residues are deleted, substituted or added in the amino acid sequence of any of a protein consisting of the amino acid sequence shown in any of SEQ ID NOS: 1 to 7 and 35, a protein consisting of the amino acid sequence shown in any of SEQ ID NOS: 17 to 19 and having proline iminopeptidase activity, and a protein consisting of the amino acid sequence shown in SEQ ID NO: 23 and having L-amino acid amide hydrolase activity" means that the amino acid sequence may contain deletion, substitution or addition of a single or plural amino acid residues at an arbitrary position therein.

Deletion, substitution and addition may be simultaneously contained in one sequence, and amino acids to be substituted or added may be either natural or not. Examples of the natural amino acids are L-alanine, L-asparagine, L-aspartic acid, L-glutamine, L-glutamic acid, glycine, L-arginine, L-histidine, L-isoleucine, L-leucine, L-lysine, L-methionine, L-phenylalanine, L-proline, L-serine, L-threonine, L-tryptophan, L-tyrosine, L-valine and L-cysteine.

The following are examples of the amino acids capable of mutual substitution. The amino acids in the same group can be mutually substituted.
- Group A:: leucine, isoleucine, norleucine, valine, norvaline, alanine, 2-aminobutanoic acid, methionine, O-methylserine, t-butylglycine, t-butylalanine, cyclohexylalanine
- Group B:: aspartic acid, glutamic acid, isoaspartic acid, isoglutamic acid, 2-aminoadipic acid, 2-aminosuberic acid
- Group C:: asparagine, glutamine
- Group D:: lysine, arginine, ornithine, 2,4-diaminobutanoic acid, 2,3-diaminopropionic acid
- Group E:: proline, 3-hydroxyproline, 4-hydroxyproline
- Group F:: serine, threonine, homoserine
- Group G:: phenylalanine, tyrosine

There is no specific restriction as to the position where the above deletion, substitution or addition of one or more amino acid residues is introduced, so long as a protein having an amino acid sequence carrying the introduced mutation has the dipeptide-synthesizing activity. Suitable examples include amino acid residues which are not conserved in all of the amino acid sequences shown in SEQ ID NOS: 1 to 7 and 35 when the sequences are compared using known alignment software. An example of known alignment software is alignment analysis software contained in gene analysis software Genetyx (Software Development Co., Ltd.). As analysis parameters for the analysis software, default values can be used.

The above proteins consisting of an amino acid sequence wherein one or more amino acid residues are deleted, substituted or added and having the activity to synthesize a dipeptide include proteins consisting of an amino acid sequence which has at least 65% or more, preferably at least 80% or more, more preferably at least 90% or more, even more preferably at least 95% or more, further preferably at least 98% or more and most further preferably at least 99% or more homology to the amino acid sequence shown in any of SEQ ID NOS: 1 to 7 and 35, the amino acid sequence shown in any of SEQ ID NOS: 17 to 19 or the amino acid sequence shown in SEQ ID NO: 23.

The above homology among amino acid sequences and nucleotide sequences can be determined by using algorithm BLAST by Karlin and Altschul [Proc. Natl. Acad. Sci. USA, 90, 5873 (1993)] and FASTA [Methods Enzymol., 183, 63 (1990)]. On the basis of the algorithm BLAST, programs such as BLASTN and BLASTX have been developed [J. Mol. Biol., 215, 403 (1990)]. When a nucleotide sequence is analyzed by BLASTN on the basis of BLAST, the parameters, for instance, are as follows: score=100 and wordlength=12. When an amino acid sequence is analyzed by BLASTX on the basis of BLAST, the parameters, for instance, are as follows: score=50 and wordlength=3. When BLAST and Gapped BLAST programs are used, default parameters of each program are used. The specific techniques for these analyses are known (http://www.ncbi.nlm.nih.gov.).

### (4) Microorganisms Transformed with DNA Encoding a Protein Having the Activity to Synthesize a Dipeptide

The microorganisms transformed with DNA encoding a protein having the activity to synthesize a dipeptide include those carrying a recombinant DNA obtained by ligating the DNA to a vector DNA.

Examples of the microorganisms are those carrying a recombinant DNA obtained by ligating, to a vector DNA, DNA encoding a protein having the activity to synthesize a dipeptide from one or more kinds of amino acids, DNA encoding a protein having proline iminopeptidase activity or DNA encoding a protein having L-amino acid amide hydrolase activity.

Examples of the microorganisms include those belonging to the genera Escherichia, Bacillus, Corynebacterium, Pseudomonas and Saccharomyces.

The DNAs encoding a protein having the activity to synthesize a dipeptide from one or more kinds of amino acids include DNAs encoding NRPS, D-Ala-D-Ala ligase or bacilysin synthetase.

Examples of the DNAs encoding NRPS include DNAs encoding a protein selected from the group consisting of BacA, BacB, BacC, TycA, TycB, TycC and PcbAB.

Examples of the DNAs encoding D-Ala-D-Ala ligase include DNAs encoding a protein selected from the group consisting of DDlA, DdlB and DdlC.

Examples of the DNAs encoding bacilysin synthetase include DNAs encoding a protein of any of the following [1] to [4]:
[1] a protein having the amino acid sequence shown in any of SEQ ID NOS: 1 to 7 and 35;
[2] a protein consisting of an amino acid sequence wherein one or more amino acid residues are deleted, substituted or added in the amino acid sequence shown in any of SEQ ID NOS: 1 to 7 and 35 and having the activity to synthesize a dipeptide:
[3] a protein consisting of an amino acid sequence which has at least 80% or more, preferably at least 90% or more, more preferably at least 95% or more, even more preferably at least 98% or more and further preferably at least 99% or more homology to the amino acid sequence shown in any of SEQ ID NOS: 1 to 7 and 35 and having the activity to synthesize a dipeptide: and
[4] a protein comprising an amino acid sequence which has at least 80% or more, preferably at least 90% or more, more preferably at least 95% or more, even more preferably at least 98% or more and further preferably at least 99% or more homology to the amino acid sequence shown in SEQ ID NO: 15 and having the activity to synthesize a dipeptide:
   and DNAs of the following [5] to [7]:
[5] DNA having the nucleotide sequence shown in any of SEQ ID NOS: 8 to 14. 29 and 30;
[6] DNA which hybridizes with DNA having the nucleotide sequence shown in any of SEQ ID NOS: 8 to 14, 29 and 30 under stringent conditions and which encodes a protein having the activity to synthesize a dipeptide: and
[7] DNA comprising a nucleotide sequence which has at least 80% or more, preferably at least 90% or more, more preferably at least 95% or more, even more preferably at least 98% or more and further preferably at least 99% or more homology to the nucleotide sequence shown in SEQ ID NO: 16 and encoding a protein having the activity to synthesize a dipeptide.

Examples of the DNAs encoding a protein having proline iminopeptidase activity include DNAs encoding proline iminopeptidase or a protein of any of the following [1] to [3]:
[1] proline iminopeptidase described in any of Biochem. Biophys. Res. Commun., 184, 1250 (1992); FEMS Microbiol. Lett., 78, 191 (1999): Japanese Published Unexamined Patent Application No. 113887/90; Arch. Biochem. Biophys., 336, 35 (1996); J. Biochem., 119, 468 (1996); Microbiology, 140, 527 (1994); J. Bacteriol., 174, 7919 (1994); J. Biochem., 116, 818 (1994); Japanese Published Unexamined Patent Application No. 121860/97; Mol. Microbiol., 9, 1203 (1993); Appl. Environ. Microbiol., 64. 4736 (1998); J. Biochem., 122, 601 (1997); FEBS Lett., 398, 101 (1996); and Nature, 406, 959 (2000), and a protein having the amino acid sequence shown in any of SEQ ID NOS: 17 to 19;
[2] a protein consisting of an amino acid sequence wherein one or more amino acid residues are deleted, substituted or added in the amino acid sequence of any of the proline iminopeptidase or the protein of the above [1] and having proline iminopeptidase activity; and
[3] a protein consisting of an amino acid sequence which has at least 80% or more, preferably at least 90% or more, more preferably at least 95% or more, even more preferably at least 98% or more and further preferably at least 99% or more homology to the amino acid sequence of any of the proline iminopeptidase or the protein of the above [1] and having proline iminopeptidase activity;
   and DNAs of the following [4] and [5]:
[4] DNA encoding proline iminopeptidase described in any of Biochem. Biophys. Res. Commun., 184, 1250 (1992); FEMS Microbiol. Lett., 78, 191 (1999); Japanese Published Unexamined Patent Application No. 113887/90; Arch. Biochem. Biophys.. 336, 35 (1996): J. Biochem., 119, 468 (1996); Microbiology, 140, 527 (1994); J. Bacteriol., 174, 7919 (1994); J. Biochem., 116, 818 (1994); Japanese Published Unexamined Patent Application No. 121860/97; Mol. Microbiol., 9, 1203 (1993); Appl. Environ. Microbiol., 64, 4736 (1998); J. Biochem., 122, 601 (1997); FEBS Lett., 398, 101 (1996); and Nature. 406, 959 (2000), and DNA having the nucleotide sequence shown in any of SEQ ID NOS: 20 to 22; and
[5] DNA which hybridizes with any DNA of the above [4] under stringent conditions and which encodes a protein having proline iminopeptidase activity.

Examples of the DNAs encoding a protein having L-amino acid amide hydrolase activity include DNAs encoding a protein of any of the following [1] to [3]:
[1] a protein having the amino acid sequence shown in SEQ ID NO: 23;
[2] a protein consisting of an amino acid sequence wherein one or more amino acid residues are deleted, substituted or added in the amino acid sequence shown in SEQ ID NO: 23 and having L-amino acid amide hydrolase activity; and
[3] a protein consisting of an amino acid sequence which has at least 80% or more, preferably at least 90% or more, more preferably at least 95% or more, even more preferably at least 98% or more and further preferably at least 99% or more homology to the amino acid sequence shown in SEQ ID NO: 23 and having L-amino acid amide hydrolase activity:
   and DNAs of the following [4] and [5]:
[4] DNA having the nucleotide sequence shown in SEQ ID NO: 24 and encoding L-amino acid amide hydrolase; and
[5] DNA which hybridizes with DNA consisting of the nucleotide sequence shown in SEQ ID NO: 24 and encoding L-amino acid amide hydrolase under stringent conditions and which encodes a protein having L-amino acid amide hydrolase activity.

The term "hybridizing" as used herein refers to a process in which polynucleotides hybridize to the recited nucleic acid sequence or parts thereof. Therefore, said nucleic acid sequences may be useful as probes in Northern or Southern Blot analysis of RNA or DNA preparations, respectively, or can be used as oligonucleotide primers in PCR analysis dependent on their respective size. Preferably, said hybridizing polynucleotides comprise at least 10, more preferably at least 15 nucleotides while a hybridizing polynucleotide of the present to be used as a probe preferably comprises at least 100. more preferably at least 200, or most preferably at least 500 nucleotides.

It is well known in the art how to perform hybridization experiments with nucleic acid molecules, i.e. the person skilled in the art knows what hybridization conditions s/he has to use in accordance with the present invention. Such hybridization conditions are referred to in standard text books such as Sambrook et al.: Molecular Cloning: A Laboratory Manual; Cold Spring Harbor Laboratory Press, 2nd edition 1989 and 3rd edition 2001; Gerhardt et al.; Methods for General and Molecular Bacteriology; ASH Press, 1994; Lefkovits ; Immunology Methods Manual: The Comprehensive Sourcebook of Techniques; Academic Press, 1997; Golemis; Protein-Protein Interactions: A Molecular Cloning Manual; Cold Spring Harbor Laboratory Press, 2002 and other standard laboratory manuals known by the person skilled in the art or as recited above. Preferred in accordance with the present Inventions are stringent hybridization conditions.

"Stringent hybridization conditions" refer, e.g. to an overnight incubation at 42°C in a solution comprising 50% formamide, 5 x SSC (750 mM NaCl, 75 mM sodium citrate), 50 mM sodium phosphate (pH 7.6), 5x Denhardt's solution, 10% dextran sulfate, and 20µg/ml denatured, sheared salmon sperm DNA, followed e.g. by washing the filters in 0.2 x SSC at about 65°C. Also contemplated are nucleic acid molecules that hybridize at low stringency hybridization conditions. Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37°C in a solution comprising 6 x SSPE (20 x SSPE = 3 mol/l NaCl: 0.2 mol/l NaH₂PO₄; 0.02mol/l EDTA, pH 7.4), 0.5% SDS, 30% formamide, 100µg/ml salmon sperm blocking DNA; followed by washes at 50°C with 1 x SSPE, 0.1% SDS. In addition, to achieve even lower stringency, washes performed following stringent hybridization can be done at higher salt concentrations (e.g. 5 x SSC). It is of note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of hybridization conditions described above, is due to problems with compatibility.

The hybridizable DNA under the above described stringent hybridization conditions includes DNA having at least 80% or more, preferably at least 90% or more, more preferably at least 95% or more, even more preferably at least 98% or more, further preferably at least 99% or more homology to the nucleotide sequence of any of the DNAs described above as calculated by use of programs such as. BLAST and FASTA described above based on the above parameters.

The homology among nucleotide sequences can be determined by using a program such as BLAST or FASTA described above.

It is possible to confirm that the DNA hybridizing with the above DNA under stringent conditions is DNA encoding a protein having the activity to synthesize a dipeptide in the following manner. That is, a recombinant DNA expressing the DNA is prepared and the recombinant DNA is introduced into a host cell to obtain a microorganism to be used as an enzyme source. Then, 1) the enzyme source and one or more kinds of amino acids are allowed to be present in an aqueous medium, followed by HPLC analysis or the like to know whether a dipeptide is formed and accumulated in the aqueous medium, 2) the enzyme source, one or more kinds of amino acid esters and one or more kinds of amino acids are allowed to be present in an aqueous medium, followed by HPLC analysis or the like to know whether a dipeptide is formed and accumulated in the aqueous medium, or 3) the enzyme source, one or more kinds of amino acid amides and one or more kinds of amino acids are allowed to be present in an aqueous medium, followed by HPLC analysis or the like to know whether a dipeptide is formed and accumulated in the aqueous medium.

### 2. Process for Preparing the Microorganisms Used in the Process for Producing a Dipeptide

The microorganisms used in the process for producing a dipeptide of the present invention include the above-mentioned strains available from various public organizations, strains which are obtained by subjecting the above strains which have the ability to produce a dipeptide to known mutagenesis and also recombinant strains in which the ability to produce a dipeptide is intensified by genetic engineering techniques. The recombinant strains include microorganisms in which bacilysin synthetase activity, proline iminopeptidase activity or L-amino acid amide hydrolase activity is enhanced, and examples of the recombinant microorganisms include Escherichia coli NM522/pQE60ywfE, Escherichia coli JM109/pUCAAH (WO03/010187). Escherichia coli JM109/pQEAAH (WO03/010187). Escherichia coli JM109/pUCPPPEPI (WO03/010307) and Escherichia coli JM109/pUCPGPEPI (WO03/010307).

### (1) Process for Preparing the Recombinant Strains

### (a) Acquisition of DNA Encoding a Protein Having the Activity to Synthesize a Dipeptide

The above DNA encoding a protein having the activity to synthesize a dipeptide can be obtained utilizing the nucleotide sequence information on the DNA, for example, by the method described below.

The following illustrates examples of the methods to obtain DNA encoding bacilysin synthetase. That is, the DNA encoding bacilysin synthetase can be obtained, for example, by Southern hybridization of a chromosomal DNA library from a microorganism belonging to the genus Bacillus using a probe designed based on the nucleotide sequence shown in any of SEQ ID NOS: 8 to 14, 29 and 30, or by PCR [PCR Protocols. Academic Press (1990)] using primer DNAs designed based on the nucleotide sequence shown in any of SEQ ID NOS: 8 to 14, 29 and 30 and, as a template, the chromosomal DNA of a microorganism belonging to the genus Bacillus.

The DNA encoding bacilysin synthetase can also be obtained by conducting a search through various gene databases for a sequence having at least 65% or more, preferably at least 80% or more, more preferably at least 90% or more, even more preferably at least 95% or more, further preferably at least 98% or more, most further preferably at least 99% or more homology to the nucleotide sequence of DNA encoding the amino acid sequence shown in any of SEQ ID NOS: 1 to 7, 15 and 35, and obtaining the desired DNA, based on the nucleotide sequence obtained by the search, from a chromosomal DNA or cDNA library of an organism having the nucleotide sequence according to the above-described method.

The obtained DNA, as such or after cleavage with appropriate restriction enzymes, is inserted into a vector by a conventional method to obtain a recombinant DNA. A plasmid DNA is extracted from a transformant obtained by introducing the recombinant DNA into Escherichia coli. Then, the nucleotide sequence of the DNA can be determined by a conventional sequencing method such as the dideoxy method [Proc. Natl. Acad. Sci., USA, 74, 5463 (1977)] or by using a nucleotide sequencer such as 373A DNA Sequencer (Parkin-Elmer Corp.).

In cases where the obtained DNA is found to be a partial DNA by the analysis of nucleotide sequence, the full length DNA can be obtained by Southern hybridization of a chromosomal DNA library using the partial DNA as a probe.

It is also possible to prepare the desired DNA by chemical synthesis using a DNA synthesizer (e.g., Model 8905, PerSeptive Biosystems) based on the determined nucleotide sequence of the DNA.

Examples of the DNAs that can be obtained by the above-described method are DNAs having the nucleotide sequences shown in SEQ ID NOS: 1 to 7, 29 and 30.

Examples of the vectors for inserting the DNA include pBluescript II KS(+) (Stratagene), pDIRECT [Nucleic Acids Res.. 18, 6069 (1990)], pCR-Script Amp SK(+) (Stratagene), pT7 Blue (Novagen, Inc.), pCR II (Invitrogen Corp.) and pCR-TRAP (Genhunter Corp.).

Examples of Escherichia coli include Escherichia coli XL1-Blue, Escherichia coli XL2-Blue. Escherichia coli DH1, Escherichia coli MC1000, Escherichia coli KY3276, Escherichia coli W1485, Escherichia coli JM101, Escherichia coli JM109, Escherichia coli HB101, Escherichia coli No. 49, Escherichia coli W3110, Escherichia coli NY49, Escherichia coli MP347, Escherichia coli NM522 and Escherichia coli ME8415.

Introduction of the recombinant DNA can be carried out by any of the methods for introducing DNA into the above host cells, for example, the method using calcium ion [Proc. Natl. Acad. Sci. USA, 69, 2110 (1972)], the protoplast method (Japanese Published Unexamined Patent Application No. 248394/88) and electroporation [Nucleic Acids Res., 16, 6127 (1988)].

An example of the microorganism carrying the DNA encoding a protein having the dipeptide-synthesizing activity obtained by the above method is the above-described Escherichia coli NM522/pQE60ywfE, which is a microorganism carrying a recombinant DNA comprising DNA having the nucleotide sequence shown in SEQ ID NO: 8.

### (b) Acquisition of the Microorganism which Produces a Protein Having the Dipeptide-synthesizing Activity

The microorganism which produces a protein having the dipeptide-synthesizing activity can be obtained by introducing the DNA obtained by the method described in the above (a) into a host cell using the methods described in Molecular Cloning, Second Edition, Current Protocols in Molecular Biology, etc., for example, in the following manner.

On the basis of the DNA obtained by the method described in the above (a), a DNA fragment of an appropriate length comprising a region encoding the protein having the dipeptide-synthesizing activity is prepared according to need. The productivity of the protein can be enhanced by replacing a nucleotide in the nucleotide sequence of the region encoding the protein so as to make a codon most suitable for the expression in a host cell.

The DNA fragment is inserted downstream of a promoter in an appropriate expression vector to prepare a recombinant DNA.

A transformant producing the protein having the dipeptide-synthesizing activity can be obtained by introducing the recombinant DNA into a host cell suited for the expression vector.

As the host cell, any microorganisms that are capable of expressing the desired gene can be used.

The expression vectors that can be employed are those capable of autonomous replication or integration into the chromosome in the above host cells and comprising a promoter at a position appropriate for the transcription of the DNA encoding a protein having the dipeptide-synthesizing activity.

When a procaryote such as a bacterium is used as the host cell, it is preferred that the recombinant DNA comprising the DNA encoding a protein having the dipeptide-synthesizing activity is a recombinant DNA which is capable of autonomous replication in the procaryote and which comprises a promoter, a ribosome binding sequence, the DNA encoding a protein having the dipeptide-synthesizing activity, and a transcription termination sequence. The recombinant DNA may further comprise a gene regulating the promoter.

Examples of suitable expression vectors are pBTrp2, pBTac1 and pBTac2 (products of Boehringer Mannheim GmbH), pHelix1 (Roche Diagnostics Corp.), pKK233-2 (Amersham Pharmacia Biotech), pSE280 (Invitrogen Corp.), pGEMEX-1 (Promega Corp.), pQE-8 (Qiagen, Inc.), pET-3 (Novagen, Inc.), pKYP 10. (Japanese Published Unexamined Patent Application No. 110600/83), pKYP200 [Agric. Biol. Chem., 48, 669 (1984)], pLSA1 [Agric. Biol. Chem., 53, 277 (1989)], pGEL1 [Proc. Natl. Acad. Sci. USA, 82, 4306 (1985)], pBluescript II SK(+), pBluescript II KS(-) (Stratagene), pTrS30 [prepared from Escherichia coli JM109/pTrS30 (FERM BP-5407)], pTrS32 [prepared from Escherichia coli JM109/pTrS32 (FERM BP-5408)], pPAC31 (WO98/12343), pUC19 [Gene, 33, 103 (1985)], pSTV28 (Takara Bio Inc.), pUC118 (Takara Bio Inc.) and pPAl (Japanese Published Unexamined Patent Application No. 233798/88).

As the promoter, any promoters capable of functioning in host cells such as Escherichia coli can be used. For example, promoters derived from Escherichia coli or phage, such as trp promoter (Pₜᵣₚ), lac promoter (P_{lac}), P_{L} promoter, P_{R} promoter and P_{SE} promoter, SPO1 promoter, SPO2 promoter and penP promoter can be used. Artificially designed and modified promoters such as a promoter in which two Pₜᵣₚs are combined in tandem, tac promoter, lacT7 promoter and letI promoter, etc. can also be used.

Also useful are promoters such as xylA promoter for the expression in bacteria belonging to the genus Bacillus [Appl. Microbiol. Biotechnol., 35, 594-599 (1991)] and P54-6 promoter for the expression in bacteria belonging to the genus Corynebacterium [Appl. Microbiol. Biotechnol., 53, 674-679 (2000)].

It is preferred to use a plasmid in which the distance between the Shine-Dalgarno sequence (ribosome binding sequence) and the initiation codon is adjusted to an appropriate length (e.g., 6 to 18 nucleotides).

In the recombinant DNA wherein the DNA encoding a protein having the dipeptide-synthesizing activity is ligated to an expression vector, the transcription termination sequence is not essential, but it is preferred to place the transcription termination sequence immediately downstream of the structural gene.

An example of such recombinant DNA is pPE43 described below.

Examples of procaryotes which are preferably used as host cells include microorganisms belonging to the genera Escherichia, Bacillus, Pseudomonas and Corynebacterium. Specific examples of microorganisms belonging to these genera are Escherichia coli XL1-Blue, Escherichia coli XL2-Blue, Escherichia coli DHl, Escherichia coli DH5α, Escherichia coli MC1000, Escherichia coli KY3276, Escherichia coli W1485, Escherichia coli JM101, Escherichia coli JM109. Escherichia coli HB101, Escherichia coli No. 49, Escherichia coli W3110, Escherichia coli NY49, Escherichia coli MP347, Escherichia coli NM522, Bacillus subtilis (ATCC 33712), Bacillus megaterium, Bacillus sp. (FERM BP-6030), Bacillus amyloliquefaciens, Bacillus coagulans, Bacillus licheniformis. Bacillus pumilus, Pseudomonas putida. Corynebacterium glutamicum (ATCC 13032) and Corynebacterium glutamicum (ATCC 14297).

Introduction of the recombinant DNA can be carried out by any of the methods for introducing DNA into the above host cells, for example, the method using calcium ion [Proc. Natl. Acad. Sci. USA, 69, 2110 (1972)], the protoplast method (Japanese Published Unexamined Patent Application No. 248394/88) and electroporation [Nucleic Acids Res., 16, 6127 (1988)].

When a strain belonging to the genus Saccharomyces is used as the host cell, YEp13 (ATCC 37115), YBp24 (ATCC 37051), YCp50 (ATCC 37419), pHS19, pHS15, etc. can be used as the expression vector.

As the promoter, any promoters capable of functioning in strains belonging to the genus Saccharomyces can be used. Suitable promoters include PHO5 promoter, PGK promoter, GAP promoter, ADH promoter, gal 1 promoter, gal 10 promoter, heat shock polypeptide promoter, MFα1 promoter and CUP 1 promoter.

Examples of suitable host cells are strains belonging to the genus Saccharomyces, specifically. Saccharomyces cerevisiae.

Introduction of the recombinant DNA can be carried out by any of the methods for introducing DNA into yeast, for example, electroporation [Methods Enzymol., 194, 182 (1990)], the spheroplast method [Proc. Natl. Acad. Sci. USA, 81, 4889 (1984)] and the lithium acetate method [J. Bacteriol., 153, 163 (1983)].

### 3. Production of the Enzyme Source Used in the Process for Producing a Dipeptide of the Present Invention

The enzyme source used in the process for producing a dipeptide of the present invention can be obtained by culturing the microorganism obtained in the above 2 in a natural or synthetic medium suitable for efficient culturing of the microorganism which contains carbon sources, nitrogen sources, inorganic salts, etc. which can be assimilated by the microorganism to obtain a culture of the microorganism or a treated matter of the culture, and then treating the culture or treated matter thereof at a temperature as indicated below for a time as indicated below (hereinafter also referred to simply as heat treatment).

As the carbon sources, any carbon sources that can be assimilated by the microorganism can be used. Examples of suitable carbon sources include carbohydrates such as glucose, fructose, sucrose, molasses containing them, starch and starch hydrolyzate; organic acids such as acetic acid and propionic acid; and alcohols such as ethanol and propanol.

As the nitrogen sources, ammonia, ammonium salts of organic or inorganic acids such as ammonium chloride, ammonium sulfate, ammonium acetate and ammonium phosphate, and other nitrogen-containing compounds can be used as well as peptone, meat extract, yeast extract, corn steep liquor, casein hydrolyzate, soybean cake, soybean cake hydrolyzate, and various fermented microbial cells and digested products thereof.

Examples of the inorganic salts include potassium dihydrogenphosphate, dipotassium hydrogenphosphate, magnesium phosphate, magnesium sulfate, sodium chloride, ferrous sulfate, manganese sulfate, copper sulfate and calcium carbonate.

Culturing is usually carried out under aerobic conditions, for example, by shaking culture or submerged spinner culture under aeration. The culturing temperature is preferably 15 to 40° C. and the culturing period is usually 5 hours to 7 days. The pH is maintained at 3.0 to 9.0 during the culturing. The pH adjustment is carried out by using an organic or inorganic acid, an alkali solution, urea, calcium carbonate, ammonia, etc.

If necessary, antibiotics such as ampicillin and tetracycline may be added to the medium during the culturing.

When a microorganism transformed with an expression vector comprising an inducible promoter is used in the process for producing a dipeptide of the present invention, an inducer may be added to the medium, if necessary. For example, in the case of a microorganism transformed with an expression vector comprising lac promoter, isopropyl-β-D-thiogalactopyranoside or the like may be added to the medium; and in the case of a microorganism transformed with an expression vector comprising trp promoter, indoleacrylic acid or the like may be added.

The enzyme source used in the production process of the present invention can be produced by applying heat treatment to the culture of the microorganism or treated matter thereof obtained above at a temperature from 50 to 65°C for 5 to 20 minutes, preferably from 50 to 65°C for 10 to 15 minutes, so long as it does not deprive the microorganism used as the enzyme source of the activity to produce a dipeptide and it can reduce the activity of the microorganism to decompose the dipeptide. Those skilled in the art can easily select heat treatment conditions suitable for the microorganism used in the process for producing a dipeptide of the present invention by simple experiments.

The heat treatment of the culture of the microorganism or treated matter thereof can be carried out in such a state that the culture or treated matter thereof is allowed to be present in an aqueous medium. An example of the aqueous medium is an aqueous medium used for the reaction to form a dipeptide described below. There is no restriction as to when the heat treatment is to be carried out so long as it is before contacting the substrate such as amino acids, etc. with the enzyme source. For example, in the case of using the treated matter of the culture of the microorganism as the enzyme source, the treated matter of the culture may be produced after giving heat treatment to the culture, or the treated matter of the culture may be produced prior to the heat treatment.

### 4. Process for Producing a Dipeptide

The production process of the present invention contemplates a process for producing a dipeptide which comprises allowing an enzyme source and one or more kinds, preferably one or two kinds of substances selected from the group consisting of amino acid amides, amino acid esters and amino acids to be simultaneously present in an aqueous medium, said enzyme source being the culture of a microorganism or treated matter thereof subjected to heat treatment which is obtained in the above 3, allowing the dipeptide to form and accumulate in the medium and recovering the dipeptide from the medium.

Specifically, the production processes of the present invention include the following (i) to (iii):
(i) a process for producing a dipeptide, which comprises: allowing an enzyme source and one or more kinds, preferably one or two kinds of amino acids to be present in an aqueous medium, said enzyme source being the culture of a microorganism or treated matter thereof subjected to heat treatment which is obtained in the above 3; allowing the dipeptide to form and accumulate in the medium; and recovering the dipeptide from the medium;
(ii) a process for producing a dipeptide, which comprises: allowing an enzyme source, one or more kinds, preferably one kind of amino acid ester and one or more kinds, preferably one kind of amino acid to be present in an aqueous medium, said enzyme source being the culture of a microorganism or treated matter thereof subjected to heat treatment which is obtained in the above 3; allowing the dipeptide to form and accumulate in the medium; and recovering the dipeptide from the medium; and
(iii) a process for producing a dipeptide, which comprises: allowing an enzyme source, one or more kinds, preferably one kind of amino acid amide and one or more kinds, preferably one kind of amino acid to be present in an aqueous medium, said enzyme source being the culture of a microorganism or treated matter thereof subjected to heat treatment which is obtained in the above 3: allowing the dipeptide to form and accumulate in the medium; and recovering the dipeptide from the medium.

One or more kinds, preferably one or two kinds of amino acids used as substrates in the above production process (i) are amino acids, preferably amino acids selected from the group consisting of L-amino acids. Glycine (Gly) and β-alanine (β-Ala), and can be used in any combination. Examples of L-amino acids are L-alanine (L-Ala), L-glutamine (L-Gln), L-glutamic acid (L-Glu), L-valine (L-Val), L-leucine (L-Leu). L-isoleucine (L-Ile), L-proline (L-Pro), L-phenylalanine (L-Phe), L-tryptophan (L-Trp), L-methionine (L-Met), L-serine (L-Ser), L-threonine (L-Thr), L-cysteine (L-Cys), L-asparagine (L-Asn), L-tyrosine (L-Tyr), L-lysine (L-Lys), L-arginine (L-Arg), L-histidine (L-His), L-aspartic acid (L-Asp), L-a-aminobutyric acid (L-a-AB), L-azaserine, L-theanine, L-4-hydroxyproline (L-4-HYP), L-3-hydroxyproline (L-3-HYP), L-ornithine (L-Orn), L-citrulline (L-Cit) and L-6-diazo-5-oxo-norleucine.

The amino acids which are more preferably used in the above process (i) include the following: a combination of one kind of amino acid selected from the group consisting of L-Ala, Gly, L-Met, L-Ser, L-Thr and β-Ala, and one kind of amino acid selected from the group consisting of L-Ala, L-Gln, L-Glu, Gly, L-Val, L-Leu, L-Ile, L-Pro, L-Phe, L-Trp, L-Met, L-Ser, L-Thr, L-Cys, L-Asn, L-Tyr, L-Lys, L-Arg, L-His, L-Asp, L-a-AB, B-Ala, L-azaserine, L-theanine, L-4-HYP, L-3-HYP, L-Orn, L-Cit and L-6-diazo-5-oxo-norleucine; a combination of L-Gln and L-Phe; and a combination of L-a-AB and L-Gln, L-Arg or L-a-AB. Further preferred amino acids are: a combination of L-Ala and one kind of amino acid selected from the group consisting of L-Ala, L-Gln, Gly, L-Val, L-Leu, L-Ile, L-Phe, L-Trp, L-Met, L-Ser, L-Thr, L-Cys, L-Asn, L-Tyr, L-Lys. L-Arg, L-His, L-a-AB, L-azaserine, L-Cit and L-theanine: a combination of Glyand one kind of amino acid selected from the group consisting of L-Gln. Gly, L-Phe, L-Trp, L-Met, L-Ser, L-Thr, L-Cys, L-Tyr. L-Lys, L-Arg, L-a-AB and L-Cit; a combination of L-Met and one kind of amino acid selected from the group consisting of L-Phe, L-Met, L-Ser, L-Thr, L-Cys, L-Tyr, L-Lys and L-His; a combination of L-Ser and one kind of amino acid selected from the group consisting of L-Gln, L-Phe, L-Ser, L-Thr, L-Tyr, L-His and L-a-AB; a combination of L-Thr and one kind of amino acid selected from the group consisting of L-Gln. L-Phe, L-Leu, L-Thr and L-a-AB: a combination of L-Gln and L-Phe; a combination of B-Ala and one kind of amino acid selected from the group consisting of L-Phe, L-Met, L-His and L-Cit; and a combination of L-a-AB and L-Gln, L-Arg or L-a-AB.

In the above production process (i), the amino acid used as a substrate is added to the aqueous medium at the start or in the course of reaction to give a concentration of 0.1 to 500 g/l, preferably 0.2 to 200 g/l.

The dipeptides produced by the above process (i) include the dipeptides represented by the following formula (I):

R¹-R² (I)

(wherein R¹ and R², which may be the same or different, each representing an amino acid). Preferred dipeptides are those represented by the above formula (I) wherein R¹ and R² , which may be the same or different, each represent an amino acid selected from the group consisting of L-Ala, L-Gln, L-Glu, Gly, L-Val, L-Leu, L-Ile, L-Pro, L-Phe, L-Trp, L-Met, L-Ser, L-Thr, L-Cys, L-Asn, L-Tyr, L-Lys, L-Arg, L-His, L-Asp, L-a-AB, β-Ala, L-azaserine. L-theanine, L-4-HYP, L-3-HYP, L-Orn and L-6-diazo-5-oxo-norleucine. More preferred are dipeptides wherein R¹ is L-Ala, Gly, L-Met, L-Ser, L-Thr or β-Ala, and R² is L-Ala, L-Gln, L-Glu. Gly, L-Val, L-Leu, L-Ile, L-Pro, L-Phe, L-Trp, L-Met, L-Ser, L-Thr, L-Cys, L-Asn, L-Tyr, L-Lys, L-Arg, L-His, L-Asp, L-a-AB, β-Ala, L-azaserine, L-theanine, L-4-HYP, L-3-HYP, L-Orn or L-6-diazo-5-oxo-norleucine. Further preferred dipeptides are: dipeptides wherein R¹ is L-Ala and R² is L-Ala, L-Gln, Gly, L-Val, L-Leu, L-Ile, L-Phe, L-Trp, L-Met, L-Ser, L-Thr, L-Cys, L-Asn, L-Tyr, L-Lys, L-Arg, L-His, L-a-AB, L-azaserine or L-theanine; dipeptides wherein R¹ is Gly and R² is L-Gln, Gly, L-Trp, L-Met, L-Ser, L-Thr, L-Cys, L-Tyr, L-Lys. L-Arg or L-a-AB; dipeptides wherein R¹ is L-Met and R² is L-Phe, L-Met, L-Cys. L-Tyr, L-Lys or L-His; dipeptides wherein R¹ is L-Ser and R² is L-Gln, Gly, L-Phe, L-Met, L-Ser, L-Thr, L-Tyr, L-His or L-a-AB: dipeptides wherein R¹ is L-Thr and R² is L-Gln, Gly, L-Phe, L-Met, L-Ser, L-Thr or L-a-AB; dipeptides wherein R¹ is L-Gln and R² is L-Phe or L-a-AB; a dipeptide wherein R¹ is L-Phe and R² is L-Gln: a dipeptide wherein R¹ is L-Trp and R² is Gly: dipeptides wherein R¹ is L-Cys and R² is L-Ala, L-Gln, Gly or L-Met; dipeptides wherein R¹ is L-Lys and R² is L-Ala, Gly or L-Met; a dipeptide wherein R¹ is L-Arg and R² is L-a-AB; a dipeptide wherein R¹ is L-His and R² is L-Met; and dipeptides wherein R¹ is L-a-AB and R² is L-Ala, L-Gln, Gly, L-Ser, L-Thr, L-Arg or L-a-AB.

Further, in the above process, compounds which can be metabolized by the microorganism of the present invention to produce ATP, for example, sugars such as glucose, alcohols such as ethanol, and organic acids such as acetic acid may be added, as ATP source, to the aqueous medium according to need.

One or more kinds of amino acid esters and one or more kinds of amino acids used as substrates in the above production process (ii) may be any of amino acid esters and amino acids that can be used as substrates by the microorganism used as an enzyme source of the process of the present invention to form a dipeptide, and they can be used in any combination. Preferably, a combination of one kind of L-amino acid ester and one kind of amino acid is used, and it is preferred to use L-amino acid and Gly as the amino acid. More preferred combinations of one kind of amino acid ester and one kind of amino acid include combinations of one kind of amino acid ester selected from the group consisting of L-alanine ester, glycine ester, L-valine ester, L-isoleucine ester, L-methionine ester, L-phenylalanine ester, L-serine ester, L-threonine ester, L-glutamine ester, L-tyrosine ester, L-arginine ester, L-aspartic acid-a-ester, L-aspartic acid-β-ester, L-leucine ester, L-asparagine ester, L-lysine ester, L-aspartic acid-a,β-dimethyl ester and L-glutamine-Y-ester, and one kind of amino acid selected from the group consisting of L-Gln. L-Asn, Gly, L-Ala, L-Leu, L-Met, L-Pro, L-Phe, L-Trp, L-Ser, L-Thr, L-Tyr, L-Lys, L-Arg, L-His and L-Glu.

In the above process (ii), the amino acid ester and the amino acid used as substrates are added to the aqueous medium at the start or in the course of reaction to give a concentration of 0.1 to 500 g/l, preferably 0.2 to 200 g/l, respectively.

One or more kinds of amino acid amides and one or more kinds of amino acids used as substrates in the above production process (iii) may be any of amino acid amides and amino acids that can be used as substrates by the microorganism used as an enzyme source of the process of the present invention to form a dipeptide, and they can be used in any combination. Preferably, a combination of one kind of amino acid amide and one kind of amino acid is used, and it is preferred to use L-amino acid and Gly as the amino acid. Examples of combinations of one kind of amino acid amide and one kind of amino acid include combinations of one kind of amino acid amide selected from the group consisting of L-alanine amide, glycine amide and L-aspartic acid amide, and one kind of amino acid selected from the group consisting of L-Gln, L-Asn, Gly, L-Ala, L-Val, L-Leu, L-Ile, L-Met, L-Pro, L-Phe, L-Trp, L-Ser, L-Thr, L-Tyr, L-Lys, L-Arg, L-His and L-Glu.

In the above process (iii), the amino acid amide and the amino acid used as substrates are added to the aqueous medium at the start or in the course of reaction to give a concentration of 0.1 to 500 g/l, preferably 0.2 to 200 g/l, respectively.

The aqueous medium used in the production processes of the present invention may comprise any components and may have any composition so far as the dipeptide-forming reaction is not inhibited. Suitable aqueous media include water and buffers such as phosphate buffer, carbonate buffer, acetate buffer, borate buffer, citrate buffer and Tris buffer. The aqueous medium may comprise alcohols such as methanol and ethanol, esters such as ethyl acetate, ketones such as acetone, and amides such as acetamide.

The dipeptide-forming reaction is carried out in the aqueous medium at pH 5 to 11, preferably pH 6 to 10, at 20 to 60°C, preferably 25 to 45°C, for 2 to 150 hours, preferably 6 to 120 hours.

If necessary, a surfactant or an organic solvent may further be added to the aqueous medium.

Any surfactant that promotes the formation of a dipeptide can be used. Suitable surfactants include nonionic surfactants such as polyoxyethylene octadecylamine (e.g., Nymeen S-215, NOF Corporation), cationic surfactants such as cetyltrimethylammonium bromide and alkyldimethylbenzylammonium chloride (e.g., Cation F2-40E, NOF Corporation), anionic surfactants such as lauroyl sarcosinate, and tertiary amines such as alkyldimethylamine (e.g., Tertiary Amine FB, NOF Corporation), which may be used alone or in combination. The surfactant is usually used at a concentration of 0.1 to 50 g/l. As the organic solvent, xylene, toluene, aliphatic alcohols, acetone, ethyl acetate, etc. may be used usually at a concentration of 0.1 to 50 ml/l.

The treated matters of the culture include the treated matter comprising living cells such as concentrated culture, dried culture, cells obtained by centrifuging the culture, and products obtained by treating the cells by various means such as drying, freeze-drying, treatment with a surfactant, treatment with a solvent, enzymatic treatment and immobilization. The treated matters of the culture of the present invention also include crude extracts of protein obtained by removing insoluble matters and the like from the treated matters obtained by treating the above cells by means such as treatment with ultrasonication and mechanical friction.

The amount of the culture or a treated matter of the culture used as the enzyme source to be added varies according to its specific activity, etc., but is, for example. 5 to 1000 mg, preferably 10 to 400 mg per mg of the amino acid, amino acid ester or amino acid amide used as a substrate.

Recovery of the dipeptide formed and accumulated in the aqueous medium can be carried out by ordinary methods using active carbon, ion-exchange resins, etc. or by means such as extraction with an organic solvent, crystallization, thin layer chromatography and high performance liquid chromatography.

Further, the above production processes (11) and (iii) can be carried out according to the descriptions in WO03/010189 or WO03/010187.

### 5. Experimental Examples of the Process for Producing a Microorganism Which Produces a Dipeptide from One or More Kinds of Amino Acids

Experimental examples of the process for producing a microorganism which produces a dipeptide from one or more kinds of amino acids are shown below.

### Experimental Example 1

### Construction of a Plasmid Expressing ywfE Gene Derived from Bacillus subtilis

A ywfE gene fragment of Bacillus subtilis was obtained in the following manner.

By using a DNA synthesizer (Model 8905, PerSeptive Biosystems. Inc.), DNAs having the nucleotide sequences shown in SEQ ID NOS: 25 and 26 (hereinafter referred to as primer A and primer B. respectively) were synthesized. Primer A has a nucleotide sequence containing a region wherein the initiation codon of ywfE gene (atg) is substituted by the NcoI recognition sequence (ccatgg). Primer B has a nucleotide sequence containing a region wherein the termination codon of ywfE gene is substituted by the BamHI recognition sequence (ggatcc).

PCR was carried out using the chromosomal DNA of Bacillus subtilis as a template and the above primer A and primer B as a set of primers. That is, PCR was carried out by 30 cycles, one cycle consisting of reaction at 94°C for one minute, reaction at 55°C for 2 minutes and reaction at 72°C for 3 minutes, using 40 µl of a reaction mixture comprising 0.1 µg of the chromosomal DNA, 0.5 µ mol/l each of the primers, 2.5 units of Pfu DNA polymerase, 4 µl of buffer for Pfu DNA polymerase (10 x) and 200 µ mol/l each of dNTPs.

One-tenth of the resulting reaction mixture was subjected to agarose gel electrophoresis to confirm that a ca. 1.4 kb fragment corresponding to the ywfE gene fragment was amplified. Then, the remaining reaction mixture was mixed with an equal amount of phenol/chloroform saturated with TE. The resulting mixture was centrifuged, and the obtained upper layer was mixed with a two-fold volume of cold ethanol and allowed to stand at -80°C for 30 minutes. The resulting solution was centrifuged, and the obtained DNA precipitate was dissolved in 20 µl of TE.

The thus obtained solution (5 µl) was subjected to reaction to cleave the amplified DNA with restriction enzymes NcoI and BamHI. DNA fragments were separated by agarose gel electrophoresis, and a 1.4 kb DNA fragment containing ywfE gene was recovered using GENECLEAN II Kit(BIO 101).

C-Terminal His-tagged recombinant expression vector pQB60 (Qiagen, Inc.) (0.2 µg) was cleaved with restriction enzymes NcoI and BamHI. DNA fragments were separated by agarose gel electrophoresis, and a 3.4 kb DNA fragment was recovered in the same manner as above.

The 1.4 kb DNA fragment containing ywfE gene and the 3.4 kb DNA fragment obtained above were subjected to ligation reaction using a ligation kit (Takara Bio Inc.) at 16°C for 16 hours.

Escherichia coli NM522 (Stratagene) was transformed using the ligation reaction mixture according to the method using calcium ion [Proc. Natl. Acad. Sci. USA, 69, 2110 (1972)], spread on LB agar medium containing 50 µ g/ml ampicillin, and cultured overnight at 30°C.

A plasmid was extracted from a colony of the transformant that grew on the medium according to a known method, whereby pQE60ywfE, which is a C-terminal His-tagged type of ywfE gene expression vector, was obtained. The structure of the vector was confirmed by digestion with restriction enzymes (Fig. 1).

### Experimental Example 2

### Acquisition of a ywfE Gene Product

Escherichia coli NM522/pQE60ywfE carrying pQE60ywfE was inoculated into 8 ml of LB medium containing 50 µg/ml ampicillin in a test tube, and cultured at 28°C for 17 hours. The resulting culture was inoculated into 50 ml of LB medium containing 50 µg/ml ampicillin in a 250-ml Erlenmeyer flask, and cultured at 30°C for 3 hours. Then, isopropyl-β-D-thlogalactopyranoside (IPTG) was added to give a final concentration of 1 mmol/l, followed by further culturing at 30°C for 4 hours. The resulting culture was centrifuged to obtain wet cells, and a His-tagged recombinant enzyme was purified from the wet cells using HisTrap (His-tagged protein purification kit, Amersham Pharmacia Biotech) according to the instructions attached thereto.

### Experimental Example 3

### Production of Dipeptides Using the His-Tagged Recombinant Enzyme (1)

(i) A reaction mixture (0.1 ml) comprising 0.04 mg of the purified His-tagged recombinant enzyme obtained in Experimental Example 2, 100 mmol/l Tris-HCl (pH 8.0). 60 mmol/l magnesium chloride. 60 mmol/l ATP, 30 mmol/l L-Ala and 30 mmol/l L-Gln was prepared, and reaction was carried out at 37°C for 16 hours .
   After the completion of reaction, the reaction product was derivatized by the dinitrophenol method and then analyzed by HPLC. The HPLC analysis was carried out using, as a separation column, Lichrosorb-RP-18 column (Kanto Kagaku) and, as an eluting solution, 1% (v/v) phosphoric acid and 25% (v/v) acetonitrile at a flow rate of 0.7 ml/min. As a result, it was confirmed that 3.7 g/l L-Ala-L-Gln and 0.3 g/l L-alanyl-L-alanine (L-Ala-L-Ala) were formed and accumulated in the reaction mixture.
(ii) Reactions were carried out under the same conditions as in the above (i) using reaction mixtures having the same composition as that of the reaction mixture of the above (1) except that 0.01 mg of the enzyme was used and L-Phe, L-Met. L-Leu and L-Val, respectively, were used in place of L-Gln.
   After the completion of reactions, the reaction products were analyzed in the same manner as in the above (1), whereby it was confirmed that the following dipeptides were formed and accumulated in the respective reaction mixtures: 7.0 g/l L-alany1-L-phenylalanine (L-Ala-L-Phe) alone; 7.0 g/l L-alanyl-L-methionine (L-Ala-L-Met) and 0.03 g/l L-Ala-L-Ala; 5.0 g/l L-alanyl-L-leucine (L-Ala-L-Leu) and 0.2 g/l L-Ala-L-Ala; and 1.6 g/l L-alanyl-L-valine (L-Ala-L-Val) and 0.3 g/l L-Ala-L-Ala.
(iii) Reactions were carried out under the same conditions as in the above (i) using reaction mixtures having the same composition as that of the reaction mixture of the above (i) except that 0.01 mg of the enzyme was used, Gly was used in place of L-Ala, and L-Phe and L-Met, respectively, were used in place of L-Gln.
   After the completion of reactions, the reaction products were analyzed in the same manner as in the above (1), whereby it was confirmed that 5.2 g/l glycyl-L-phenylalanine (Gly-L-Phe) and 1.1 g/l glycyl-L-methionine (Gly-L-Met) were formed and accumulated in the respective reaction mixtures.
   When ATP was excluded from the compositions of the above reaction mixtures, no dipeptide was formed.
   The above results revealed that the ywfE gene product has the activity to produce, in the presence of ATP, the following dipeptides: L-Ala-L-Gln plus L-Ala-L.-Ala, L-Ala-L-Phe, L-Ala-L-Met plus L-Ala-L-Ala, L-Ala-L-Leu plus L-Ala-L-Ala, or L-Ala-L-Val plus L-Ala-L-Ala from L-Ala plus L-Gln, L-Phe, L-Met, L-Leu or L-Val; and Gly-L-Phe or Gly-L-Met from Gly plus L-Phe or L-Met.

### Experimental Example 4

### Production of Dipeptides Using the His-Tagged Recombinant Enzyme (2)

A reaction mixture (0.1 ml) comprising 0.04 mg of the purified His-tagged recombinant enzyme obtained in Experimental Example 2. 100 mmol/l Tris-HCl (pH 8.0), 60 mmol/l magnesium chloride and 60 mmol/l ATP was prepared. To this mixture combinations of various L-amino acids were added respectively, Gly and β-Ala selected from the amino acids shown in the first row of Table 1 and in the leftmost column of Table 1 to give a concentration of 30 mmol/l each, and the resulting mixtures were subjected to reaction at 37°C for 16 hours. After the completion of reactions, the reaction products were analyzed by HPLC, whereby it was confirmed that the dipeptides shown in Table 1 were formed.

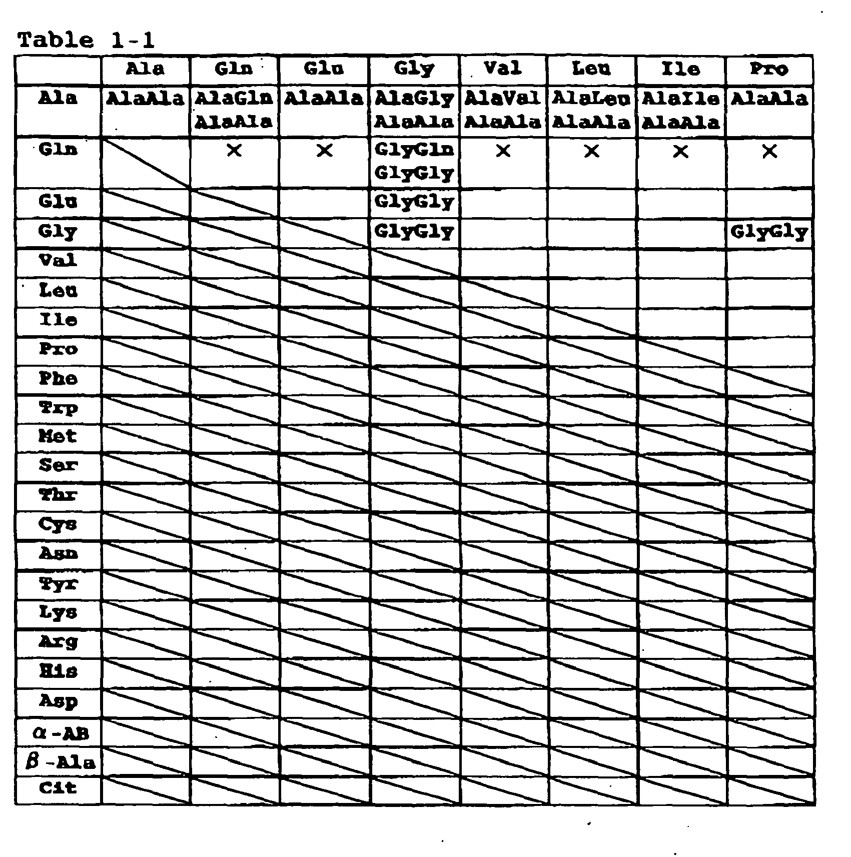

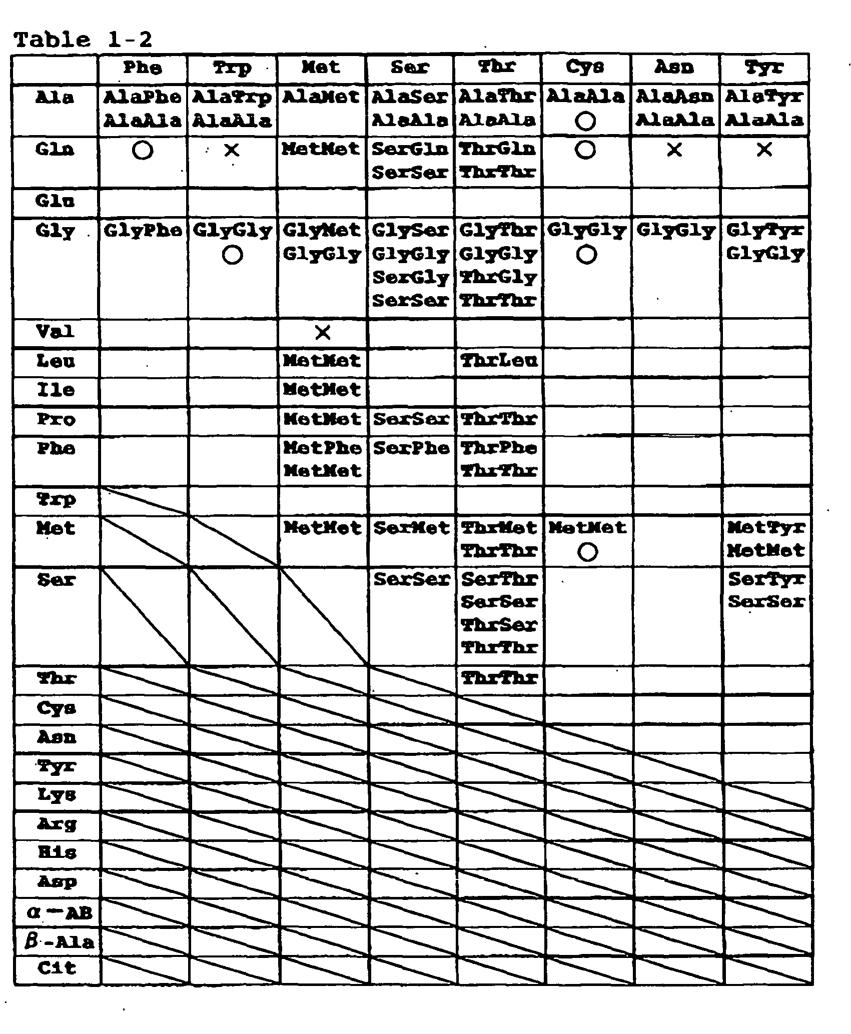

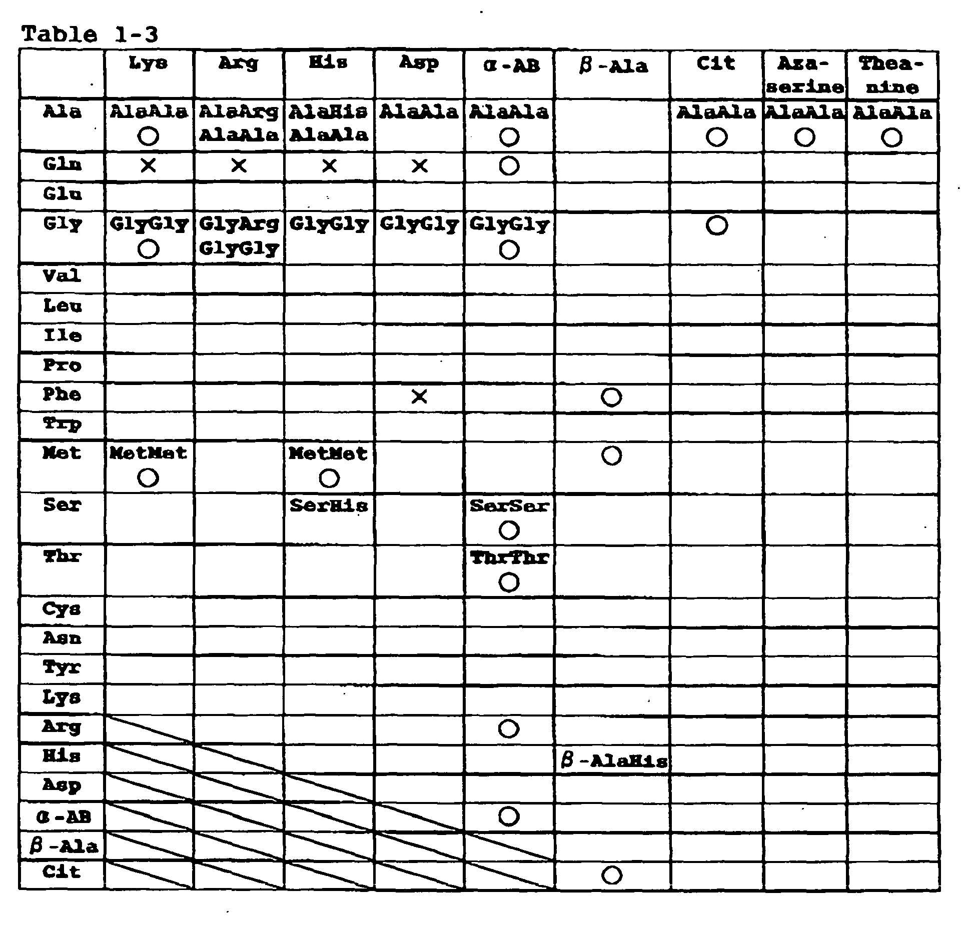

The dipeptides formed by the reaction using, as substrates, two (or one) kinds of L-amino acids, Gly and β-Ala shown in the first row and the leftmost column of Table 1 are shown in the respective cells of the table. In the table, ○ means that a dipeptide was formed though its sequence was unidentified; × means that formation of a dipeptide was not confirmed: and a blank means that reaction was not carried out.

### Experimental Example 5

### Production of a Dipeptide Using the Strain Expressing the His-Tagged Recombinant Enzyme

Escherichia coli NM522/pQR60ywfE obtained in Experimental Example 1 was inoculated into 8 ml of LB medium containing 50 µg/ml ampicillin in a test tube, and cultured at 28° C for 17 hours. The resulting culture was inoculated into 50 ml of LB medium containing 50 µg/ml ampicillin in a 250-ml Erlenmeyer flask, and cultured at 30°C for 3 hours. Then, IPTG was added to give a' final concentration of 1 mmol/1, followed by further culturing at 30°C for 4 hours. The resulting culture was centrifuged to obtain wet cells.

A reaction mixture (20 ml, pH 7.2) comprising 200 g/l wet cells, 50 g/l glucose. 5 g/l phytic acid (diluted to neutrality with 33% conc. sodium hydroxide solution), 15 g/l potassium dihydrogenphosphate, 5 g/l magnesium sulfate heptahydrate, 4 g/l Nymeen S-215, 10 ml/l xylene, 200 mmol/l L-Ala and 200 mmol/l L-Gln was put in a 50-ml beaker, and the reaction was carried out at 32°C at 900 rpm for 2 hours. During the reaction, the pH of the reaction mixture was maintained at 7.2 by using 2 mol/l potassium hydroxide.

The reaction product was analyzed by the same method as in Experimental Example 3, whereby it was confirmed that 25 mg/l L-Ala-L-Gln was accumulated.

### Experimental Example 6

### Cloning of Genes Corresponding to the ywfE Gene from Various Microorganisms of the Genus Bacillus and Analysis Thereof

On the basis of the nucleotide sequence shown in SEQ ID NO: 8, genes corresponding to the ywfE gene which exist in Bacillus subtilis ATCC 15245, ATCC 6633, IAM 1213, IAM 1107, IAM 1214, ATCC 9466, IAM 1033 and ATCC 21555, Bacillus amyloliquefaciens IFO 3022 and Bacillus pumilus NRRL B-12025 were obtained in the following manner.

That is, Bacillus subtilis ATCC 15245, ATCC 6633, IAM 1213, IAM 1107, IAM 1214, ATCC 9466, IAM 1033 and ATCC 21555. Bacillus amyloliquefaciens IFO 3022 and Bacillus pumilus NRRL B-12025 were respectively inoculated into LB medium and subjected to static culture overnight at 30°C. After the culturing, the chromosomal DNAs of the respective microorganisms were isolated and purified according to the method using saturated phenol described in Current Protocols in Molecular Biology.

By using a DNA synthesizer (Model 8905, PerSeptive Biosystems, Inc.), DHAs having the nucleotide sequences shown in SEQ ID NOS: 27 and 28 (hereinafter referred to as primer C and primer D, respectively) were synthesized. Primer C has a sequence containing a region upstream of the initiation codon of ywfE gene of the chromosomal DNA of Bacillus subtilis 168, and primer D has a sequence complementary to a sequence containing a region downstream of the termination codon of ywfE gene.

PCR was carried out using each of the chromosomal DNAs of Bacillus subtilis ATCC 15245. ATCC 6633, IAM 1213, IAM 1107, IAM 1214, ATCC 9466, IAM 1033 and ATCC 21555 and Bacillus amyloliquefaciens IFO 3022 as a template and the above primer C and primer D as a set of primers. That is, PCR was carried out by 30 cycles, one cycle consisting of reaction at 94°C for one minute, reaction at 55°C for 2 minutes and reaction at 72°C for 3 minutes, using 40 µl of a reaction mixture comprising 0.1 µg of the chromosomal DNA, 0.5 µmol/l each of the primers, 2.5 units of Pfu DNA polymerase, 4 µl of buffer for Pfu DNA polymerase (10 x) and 200 µmol/l each of dNTPs.

One-tenth of each of the resulting reaction mixtures was subjected to agarose gel electrophoresis to confirm that a ca. 1.4 kb fragment corresponding to the ywfE gene fragment was amplified. Then, the remaining reaction mixture was mixed with an equal amount of phenol/chloroform saturated with TE. The resulting solution was centrifuged, and the obtained upper layer was mixed with a two-fold volume of cold ethanol and allowed to stand at -80°C for 30 minutes. The resulting solution was centrifuged, and the obtained DNA precipitate was dissolved in 20 µl of TE.

Each of the thus obtained 1.4 kb DNA fragments derived from the chromosomal DNAs of the respective strains and pCR-blunt (Invitrogen Corp.) were subjected to ligation reaction using a ligation kit at 16°C for 16 hours.

Escherichia coli NM522 was transformed using each ligation reaction mixture according to the method using calcium ion, spread on LB agar medium containing 50 µg/ml ampicillin, and cultured overnight at 30°C.

A plasmid was extracted from a colony of each transformant that grew on the medium according to a known method and the structure of each plasmid was analyzed using restriction enzymes. As a result, it was confirmed that the following plasmids containing a gene corresponding to the ywfE gene were obtained: pYWFE1 (derived from ATCC 15245. DNA having the nucleotide sequence shown in SEQ ID NO: 30), pYWFE2 (derived from ATCC 6633, DNA having the nucleotide sequence shown in SEQ ID NO: 9), pYWFE3 (derived from IAM 1213, DNA having the nucleotide sequence shown in SEQ ID NO: 10), pYWFE4 (derived from IAM 1107, DNA having the nucleotide sequence shown in SEQ ID NO: 11), pYWFE5 (derived from IAM 1214, DNA having the nucleotide sequence shown in SEQ ID NO: 12), pYWFE6 (derived from ATCC 9466, DNA having the nucleotide sequence shown in SEQ ID NO: 8), pYWFE7 (derived from IAM 1033, DNA having the nucleotide sequence shown in SEQ ID NO: 30), pYWFE8 (derived from ATCC 21555, DNA having the nucleotide sequence shown in SEQ ID NO: 13) and pYWFE9 (derived from IFO 3022, DNA having the nucleotide sequence shown in SEQ ID NO: 14).

On the other hand, a gene corresponding to ywfE gene derived from Bacillus pumilus NRRL B-12025 (DNA having the nucleotide sequence shown in SEQ ID NO: 29) was obtained in the following manner.

PCR was carried out using the chromosomal DNA of the NRRL B-12025 strain prepared above as a template and DNAs respectively consisting of the nucleotide sequences shown in SEQ ID NOS: 31 and 32 as a set of primers. That is, PCR was carried out by 30 cycles, one cycle consisting of reaction at 98°C for 5 seconds, reaction at 55°C for 30 seconds and reaction at 72°C for one minute, using 50 µl of a reaction mixture comprising 0.1 µg of the chromosomal DMA, 0-5 µmol/l each of the primers, 2.5 units of Z-taq polymerase (TaKara Bio Inc.), 5 µl of buffer for Z-taq polymerase (10 x) (Takara Bio Inc.) and 200 µmol/l each of dNTPs.

One-tenth of the resulting reaction mixture was subjected to agarose gel electrophoresis to confirm that a ca. 0.8 kb fragment was amplified. Then, the remaining reaction mixture was mixed with an equal amount of phenol/chloroform saturated with TE. The resulting mixture was centrifuged, and the obtained upper layer was mixed with a two-fold volume of cold ethanol and allowed to stand at -80°C for 30 minutes. The resulting solution was centrifuged, and the obtained DNA precipitate was dissolved in 20 µl of TE.

The thus obtained 0.8 kb fragment derived from the chromosomal DNA and pGEM T-easy (Promega Corp.) were subjected to ligation reaction using a ligation kit at 16°C for 16 hours.

Escherichia coli DH5α was transformed using the reaction mixture according to the method using calcium ions, spread on LB agar medium containing 50 µg/ml ampicillin, and cultured overnight at 30°C.

A plasmid was extracted from the transformant obtained above and the nucleotide sequence of the ca. 0.8 kb DNA insert was determined, whereby a sequence from nucleotides 358 to 1160 in the nucleotide sequence shown in SEQ ID NO: 29 was confirmed.

The above plasmid was cleaved with EcoRI and then subjected to agarose gel electrophoresis to separate a DNA fragment. The DNA fragment was purified using GENECLEAN II Kit, and about 0.5 µg of the purified DNA fragment was DIG-labeled using DIG-High Prime DNA Labeling & Detection Starter Kit I (Roche Diagnostics Corp.) according to the instructions attached thereto.

Southern analysis of the chromosomal DNA of the NRRL B-12025 strain was carried out using the DIG-labeled DNA obtained above.

The chromosomal DNA of the NRRL B-12025 strain was completely digested with BamHI, EcoRI, HindIII, KpnI, PstI, SacI, SalI and SphI, respectively, and subjected to agarose gel electrophoresis to separate DNA fragments, followed by transfer to nylon membrane plus charge (Roche Diagnostics Corp.) according to an ordinary method.

After the DNA fragments were fixed on the nylon membrane by UV irradiation, Southern hybridization was carried out using the above probe DNA and the nylon membrane.

The hybridization was carried out by contacting the nylon membrane with the probe DNA at 65°C for 16 hours, washing the nylon membrane twice with a solution consisting of 0-1% SDS and 2 x SSC at room temperature for 5 minutes, and further washing the membrane twice with a solution consisting of 0.1% SDS and 0.5 x SSC at 65°C for 15 minutes. The other operations and conditions and detection of the hybridized DNA were carried out according to the instructions attached to the above-mentioned DIG-High Prime DNA Labeling & Detection Starter Kit I.

As a result, color development was observed at around 3.5 kbp of the fragments completely digested with HindIII and PstI.

Subsequently, the chromosomal DNA of the NRRL B-12025 strain was completely digested with HindIII and PstI, respectively, and subjected to agarose gel electrophoresis to separate DNA fragments. From the respective restriction enzyme-digested DNAs, 3-4 kbp fragments were purified using GENECLEAN II Kit, followed by autocyclization using the ligation kit.

On the basis of the nucleotide sequence of the 0.8 kb DNA fragment determined above, the nucleotide sequences shown in SBQ ID NOS: 33 and 34 were designed and synthesized, and they were used in PCR using the cyclized DNA obtained above as a template. PCR was carried out by 30 cycles, one cycle consisting of reaction at 98°C for 5 seconds, reaction at 55°C for 30 seconds and reaction at 72°C for 3 minutes and 30 seconds, using 50 µl of a reaction mixture comprising 10 ng of the cyclized DNA, 0.5 µmol/l each of the primers, 2.5 units of pyrobest polymerase (Takara Bio Inc.), 5 µl of buffer for pyrobest polymerase (10 x) (Takara Bio Inc.) and 200 µmol/l each of dNTPs.

One-tenth of the resulting reaction mixture was subjected to agarose gel electrophoresis to confirm that a ca. 3.0 kb fragment was amplified. Then, the remaining reaction mixture was mixed with an equal amount of phenol/chloroform saturated with TE. The resulting mixture was centrifuged, and the obtained upper layer was mixed with a two-fold volume of cold ethanol and allowed to stand at -80°C for 30 minutes. The resulting solution was centrifuged, and the obtained DNA precipitate was dissolved in 20 µl of TE.

The thus obtained DNA fragment and Zero Blunt PCR Cloning Kit (Invitrogen Corp.) were subjected to ligation reaction using a ligation kit.

Escherichia coli NM522 was transformed using the reaction mixture according to the method using calcium ion, spread on LB agar medium containing 50 µg/ml ampicillin, and cultured overnight at 30°C.

A plasmid was extracted from a colony of the transformant that grew on the medium according to a known method and the structure of the plasmid was analyzed using restriction enzymes. As a result, it was confirmed that plasmid pYWFE10 (derived from NRRL B-12025, DNA having the nucleotide sequence shown in SEQ ID NO: 29) containing a gene corresponding to the ywfE gene was obtained.

The nucleotide sequences of the genes corresponding to the ywfE gene which are respectively contained in the plasmids pYWFE1 to pYWFE10 obtained above were determined using 373A DNA Sequencer.

The amino acid sequences of the proteins encoded by the genes respectively contained in pYWFE1, pYWFE6 and pYWFB7 were identical with the amino acid sequence of the protein encoded by the ywfE gene, whereas those of the proteins encoded by the genes respectively contained in pYWFE2, pYWFR3, pYWPE4, pYWFE5, pYWFE8, pYWFE9 and pYWFE10 were different from the amino acid sequence of the protein encoded by the ywfE gene.

The amino acid sequences of the proteins encoded by the genes corresponding to the ywfE gene which are contained in pYWFE2, pYWFE3, pYWFE4, pYWFE5, pYWFE8, pYWFE9 and pYWFE10, and pYWFE1 and pYWFE7 are shown in SEQ ID NOS: 2 to 7, 35 and 1, respectively, and the nucleotide sequences of these genes are shown in SEQ ID NOS: 9 to 14, 29, 8 and 30, respectively.

### Experimental Example 7

### Purification of C-Terminal His-Tagged Recombinant Dipeptide Synthetase

PCR was carried out using each of the chromosomal DNAs of Bacillus subtilis ATCC 15245, ATCC 6633, IAM 1213, IAM 1107, IAM 1214, ATCC 9466, IAM 1033 and ATCC 21555 and Bacillus amyloliquefaciens IFO 3022 as a template and primer A and primer B described in Experimental Example 1 as a set of primers. That is, PCR was carried out by 30 cycles, one cycle consisting of reaction at 94°C for one minute, reaction at 55°C for 2 minutes and reaction at 72°C for 3 minutes, using 40 µl of a reaction mixture comprising 0.1 µg of the chromosomal DNA, 0.5 µmol/l each of the primers, 2.5 units of Pfu DNA polymerase, 4 µl of buffer for Pfu DNA polymerase (10 x) and 200 µmol/l each of dNTPs.

When the chromosomal DNA of Bacillus pumilus NRRL B-12025 was used as a template. PCR was carried out using DNAs respectively having the nucleotide sequences shown in SEQ ID NOS: 36 and 37 as a set of primers under the same conditions as above.

One-tenth of each of the resulting reaction mixtures was subjected to agarose gel electrophoresis to confirm that a ca. 1.4 kb DNA fragment corresponding to the ywfE fragment was amplified. Then, the remaining reaction mixture was mixed with an equal amount of phenol/chloroform saturated with TE. The resulting mixture was centrifuged, and the obtained upper layer was mixed with a two-fold volume of cold ethanol and allowed to stand at -80°C for 30 minutes. The resulting solution was centrifuged, and the obtained DNA precipitate was dissolved in 20 µl of TE.

Each of the thus obtained solutions (5 µl) was subjected to reaction to cleave the amplified DNA with restriction enzymes NcoI and BamHI. DNA fragments were separated by agarose gel electrophoresis, and a 1.4 kb DNA fragment containing a gene corresponding to the ywfE gene was recovered using GENECLEAN II Kit.

Subsequently, 0.2 µg of the C-terminal His-tagged recombinant expression vector pQE60 was cleaved with restriction enzymes NcoI and BamHI. DNA fragments were separated by agarose gel electrophoresis, and a 3.4 kb DNA fragment was recovered in the same manner as above.

Bach of the 1.4 kb DNA fragments containing a gene corresponding to the ywfE gene of Bacillus subtilis 168 and the 3.4 kb DNA fragment obtained above were subjected to ligation reaction using a ligation kit at 16°C for 16 hours. Escherichia coli NH522 was transformed using each ligation reaction mixture according to the method using calcium ions, spread on LB agar medium containing 50 µg/ml ampicillin, and cultured overnight at 30°C.

A plasmid was extracted from a colony of each transformant that grew on the medium according to a known method and the structure of each plasmid was analyzed using restriction enzymes. As a result, it was confirmed that the following C-terminal His-tagged gene expression vectors were obtained: pQE60ywfE1 (a vector containing the gene derived from ATCC 15245), pQE60ywfE2 (a vector containing the gene derived from ATCC 6633), pQE60ywfE3 (a vector containing the gene derived from IAM 1213), pQE60ywfE4 (a vector containing the gene derived from IAM 1107), pQE60ywfE5 (a vector containing the gene derived from IAM 1214), pQE60ywfE6 (a vector containing the gene derived from ATCC 9466), pQE60ywfE7 (a vector containing the gene derived from IAM 1033), pQE60ywfE8 (a vector containing the gene derived from ATCC 21555), pQE60ywfE9 (a vector containing the gene derived from IFO 3022) and pQE60ywfE10 (a vector containing the gene derived from NRRL B-12025).

Escherichia coli NM522/pQE60ywfE1 to NM522/pQE60ywfE10 strains obtained above were respectively inoculated into 8 ml of LB medium containing 50 µg/ml ampicillin in a test tube, and cultured at 28°C for 17 hours. Each of the resulting cultures was inoculated into 50 ml of LB medium containing 50 µg/ml ampicillin in a 250-ml Erlenmeyer flask, and cultured at 30°C for 3 hours. Then. IPTG was added to give a final concentration of 1 mmol/l, followed by further culturing at 30°C for 4 hours. The resulting culture was centrifuged to obtain wet cells, and His-tagged recombinant enzymes were purified from the respective wet cells using HisTrap according to the instructions attached thereto.

### Experimental Example 8

### Production of Dipeptides Using Purified Enzymes

Reaction mixtures (0.1 ml each) comprising 0.04 mg of the respective recombinant enzymes obtained in Experimental Example 7, 100 mmol/l Tris-HCl (pH 8.0), 60 mmol/l magnesium chloride, 60 mmol/l ATP, 30 mmol/l L-Ala and 30 mmol/l L-Gln were prepared, and reactions were carried out at 37°C for 16 hours.

After the completion of reactions, the reaction mixtures were analyzed by the method described in Experimental Example 3, whereby it was confirmed that 3.0 to 3.5 g/l L-Ala-L-Gln and 0.25 to 0.3 g/l L-Ala-L-Ala were formed and accumulated.

When ATP was excluded from the compositions of the above reaction mixtures, L-Ala-L-Gln or L-Ala-L-Ala was not formed at all.

The above results revealed that all of the products of the genes obtained in Experimental Example 7 have the activity to produce L-Ala-L-Gln and L-Ala-L-Ala from L-Ala and L-Gln in the presence of ATP.

The present invention is illustrated more in detail in the following examples. These examples are not to be construed as limiting the scope of the invention.

### Example 1

### Production of L-Alanyl-L-Glutamine (L-Ala-L-Gln)

A liquid medium (30 ml, pH 7.0) containing 5 g/l glucose, 5 g/l ammonium sulfate, 1 g/l potassium dihydrogenphosphate, 3 g/l dipotassium hydrogenphosphate. 0.5 g/l magnesium sulfate, 10 g/l yeast extract and 10 g/l peptone was put in a 300-ml Erlenmeyer flask and sterilized at 115°C for 15 minutes. Corynebacterium glutamicum ATCC 13286 was cultured at 30ºC for 24 hours using a slant agar medium having the same composition as that of the liquid medium (containing 20 g/l agar, pH 7.0), and one platinum loop of the cultured cells was inoculated into the liquid medium and cultured with shaking at 120 reciprocation/min. at 30ºC for 17 hours. After the completion of culturing, the culture was centrifuged, and 100 mmol/l borate buffer (pH 9.0) was added to the resulting wet cells to give a concentration of 100 g/l. One-ml aliquots of the thus prepared cell suspension were subjected to: 1) no heat treatment; 2) heat treatment at 50ºC for 10 minutes; or 3) heat treatment at 55ºC for 10 minutes. The cell suspensions were then added, respectively, to one ml of a reaction solution [20 mmol/l EDTA, 200 mmol/l L-alanine ethyl ester hydrochloride, 400 mmol/l L-glutamine, 100 mmol/l borate buffer, pH 9.0] to bring total volume to 2 ml, followed by incubation at 30ºC for 30 minutes. The reaction mixture was heated at 90ºC for 15 minutes and then centrifuged, and the amount of L-Ala-L-Gln in the resulting supernatant was determined by HPLC.

HPLC analysis was carried out after the reaction product was derivatized by the dinitrophenol method using, as a separation column, Lichrosorb-RP-18 column (Kanto Kagaku) and, as an eluting solution, a solution comprising 1% (v/v) phosphoric acid and 25% (v/v) acetonitrile at a flow rate of 0.7 ml/min. The results are shown in Table 2.

**Table 2**

| Heat treatment conditions | Amount of L-Ala-L-Gln formed (relative value) |
|---|---|
| No heat treatment | 100 |
| At 50ºC for 10 min. | 353 |
| At 55ºC for 10 min. | 329 |

The amount of L-Ala-L-Gln formed was increased to more than 3-fold by carrying out heat treatment.

### Example 2

### Activity of Dipeptide-Producing Strain to Decompose L-Ala-L-Gln

Corynebacterium glutamicum ATCC 13286 was cultured and a cell suspension was obtained in the same manner as in Example 1. One-ml aliquots of the cell suspension were subjected to: 1) no heat treatment; 2) heat treatment at 50ºC for 15 minutes; or 3) heat treatment at 65ºC for 15 minutes. The cell suspensions were then added, respectively, to one ml of a reaction solution [20 mmol/l EDTA, 2 g/l L-Ala-L-Gln, 100 mmol/l borate buffer, pH 9.0] to bring total volume to 2 ml, followed by incubation at 30ºC for 30 minutes. The reaction mixture was heated at 90ºC for 15 minutes and then centrifuged, and the amount of L-Ala-L-Gln in the resulting supernatant was determined by HPLC in the same manner as in Example 1.

As a result, the amount of L-Ala-L-Gln decomposed by the 30-minute reaction was, when no heat treatment is designated as 100. 84 for the heat treatment at 55ºC for 15 minutes and 50 for the heat treatment at 65ºC for 15 minutes.

The above results show that the amount of L-Ala-L-Gln formed was increased because the activity to decompose L-Ala-L-Gln was suppressed by the heat treatment.

### Example 3

### Activity of Non-Dipeptide-Producing Strain to Decompose L-Ala-L-Gln

Escherichia coli JM109 was cultured and a cell suspension was obtained in the same manner as in Example 1. One-ml aliquots of the cell suspension were subjected to: 1) no heat treatment; 2) heat treatment at 55ºC for 15 minutes; or 3) heat treatment at 65ºC for 15 minutes. The cell suspensions were then added, respectively, to one ml of a reaction solution [20 mmol/l EDTA, 2 g/l L-Ala-L-Gln, 100 mmol/l borate buffer, pH 9.0] to bring total volume to 2 ml, followed by incubation at 30ºC for 30 minutes. The reaction mixture was heated at 90QC for 15 minutes and then centrifuged, and the amount of L-Ala-L-Gln in the resulting supernatant was determined by HPLC in the same manner as in Example 1.

As a result, the amount of L-Ala-L-Gln decomposed by the 30-minute reaction was, when no heat treatment is designated as 100, 0 for the heat treatment at 55ºC for 15 minutes and 7 for the heat treatment at 65ºC for 15 minutes.

The results of Examples 1 and 2 and the above results revealed that the amount of a dipeptide formed is remarkably improved by using, as an enzyme source, a heat-treated product of a culture of a microorganism transformed with DNA encoding a protein concerned with formation of a dipeptide, for example, DNA encoding a protein consisting of the amino acid sequence shown in any of SEQ ID NOS: 1 to 7. 17 to 19 and 23, thereby imparted with the ability to produce a dipeptide, or that of a treated matter of the culture.

### SEQUENCE LISTING FREE TEXT

SEQ ID NO: 25 - Description of Artificial Sequence: Synthetic DNA
SEQ ID NO: 26 - Description of Artificial Sequence: Synthetic DNA
SEQ ID NO: 27 - Description of Artificial Sequence: Synthetic DNA
SEQ ID NO: 28 - Description of Artificial Sequence: Synthetic DNA
SEQ ID NO: 31 - Description of Artificial Sequence: Synthetic DNA
SEQ ID NO: 32 - Description of Artificial Sequence: Synthetic DNA
SEQ ID NO: 33 - Description of Artificial Sequence: Synthetic DNA
SEQ ID NO: 34 - Description of Artificial Sequence: Synthetic DNA
SEQ ID NO: 36 - Description of Artificial Sequence: Synthetic DNA
SEQ ID NO: 37 - Description of Artificial Sequence: Synthetic DNA

## Claims

1. A process for producing a dipeptide, which comprises:
allowing an enzyme source and one or more kinds of substances selected from the group consisting of amino acid amides, amino acid esters and amino acids to be present in an aqueous medium, said enzyme source being a culture of a microorganism having the ability to produce a dipeptide or a treated matter of the culture, and having been subjected to heat treatment at a temperature of 50° to 65°C for 5 to 20 minutes; so as to produce and accumulate the dipeptide in the aqueous medium; and
recovering the dipeptide from the aqueous medium.

2. The process according to claim 1, wherein the microorganism having the ability to produce a dipeptide is a microorganism belonging to the genus Achromobacter, Acinetobacter, Aeromonas, Agrobacterium, Alcaligenes, Arthrobacter, Beiierinckia, Brevibacterium, Clavibacter, Chaseobacterium, Escherichia, Enterobacter, Erwinia, Flavobacterium, Kluyvera, Microbacterium, Micrococcus, Mycoplana, Pantoea, Propionibacterium, Listonella, Rhizobium, Rhodococcus, Salmonella, Sarcina, Serratia, Staphylococcus, Stenotrophomonas, Streptomyces, Vibrio, Xanthomonas, Bullera, Candida, Cryptococcus, Filobasidium, Geotrichum, Pachysolen, Rhodosporidium, Rhodotorula, Saccharomyces, Sporobolomyces, Tremella, Torulaspora, Torulopsis, Gluconacetobacter, Acetobacter, Gluconobacter, Asaia, Zucharibacter, Actinomadura, Kitasatosporia, Micromonospora, Nocardia, Oerskovia, Saccharothrix, Streptoverticillium, Hafnia, Lactobacillus, Neisseria, Thermoplasma, Corynebacterium, Pseudomonas, Bacillus, Trichosporon or Sterigmatomyces.

3. The process according to claim 1 or 2, wherein the microorganism having the ability to produce a dipeptide is a microorganism having the ability to produce a protein according to any of [1] to [4] below:
[1] a protein having the amino acid sequence shown in any of SEQ ID NOS: 1 to 7 and 35;
[2] a protein consisting of an amino acid sequence wherein one or more amino acid residues are deleted, substituted or added in the amino acid sequence shown in any of SEQ ID NOS: 1 to 7 and 35 and having the activity to synthesize a dipeptide;
[3] a protein having an amino acid sequence which has 65% or more homology to the amino acid sequence shown in any of SEQ ID NOS: 1 to 7 and 35 and having the activity to synthesize a dipeptide; and
[4] a protein comprising an amino acid sequence which has 80% or more homology to the amino acid sequence shown in SEQ ID NO: 15 and having the activity to synthesize a dipeptide.

4. The process according to claim 1 or 2, wherein the microorganism having the ability to produce a dipeptide is a microorganism carrying DNA according to any of [1] to [4] below:
[1] DNA encoding the protein according to any of [1] to [4] of Claim 3;
[2] DNA having the nucleotide sequence shown in any of SEQ ID NOS: 8 to 14, 29 and 30;
[3] DNA which hybridizes with DNA having the nucleotide sequence shown in any of SEQ ID NOS: 8 to 14, 29 and 30 under stringent conditions and which encodes a protein having the activity to synthesize a dipeptide; and
[4] DNA comprising a nucleotide sequence which has 80% or more homology to the nucleotide sequence shown in SEQ ID NO: 16 and encoding a protein having the activity to synthesize a dipeptide.

5. The process according to claim 1 or 2, wherein the microorganism having the ability to produce a dipeptide is a microorganism producing a protein having proline iminopeptidase activity or a protein having L-amino acid amide hydrolase activity.

6. The process according to Claim 5, wherein the protein having proline iminopeptidase activity is a protein according to any of [1] to [3] below:
[1] a protein having the amino acid sequence shown in any of SEQ ID NOS: 17 to 19;
[2] a protein consisting of an amino acid sequence wherein one or more amino acid residues are deleted, substituted or added in the amino acid sequence shown in any of SEQ ID NOS: 17 to 19 and having proline iminopeptidase activity; and
[3] a protein consisting of an amino acid sequence which has 80% or more homology to the amino acid sequence shown in any of SEQ ID NOS: 17 to 19 and having proline iminopeptidase activity.

7. The process according to Claim 5, wherein the microorganism producing a protein having proline iminopeptidase activity is a microorganism carrying DNA according to any of [1] to [3] below:
[1] DNA encoding the protein according to any of [1] to [3] of Claim 6;
[2] DNA having the nucleotide sequence shown in any of SEQ ID NOS: 20 to 22; and
[3] DNA which hybridizes with DNA having the nucleotide sequence shown in any of SEQ ID NOS: 20 to 22 under stringent conditions and which encodes a protein having proline iminopeptidase activity.

8. The process according to Claim 5, wherein the protein having L-amino acid amide hydrolase activity is a protein according to any of [1] to [3] below:
[1] a protein having the amino acid sequence shown in SEQ ID NO: 23;
[2] a protein consisting of an amino acid sequence wherein one or more amino acid residues are deleted, substituted or added in the amino acid sequence shown in SEQ ID NO: 23 and having L-amino acid amide hydrolase activity; and
[3] a protein consisting of an amino acid sequence which has 80% or more homology to the amino acid sequence shown in SEQ ID NO: 23 and having L-amino acid amide hydrolase activity.

9. The process according to Claim 5, wherein the microorganism producing a protein having L-amino acid amide hydrolase activity is a microorganism carrying DNA according to any of [1] to [3] below:
[1] DNA encoding the protein according to any of [1] to [3] of Claim 8;
[2] DNA having the nucleotide sequence shown in SEQ ID NO: 24; and
[3] DNA which hybridizes with DNA having the nucleotide sequence shown in SEQ ID NO: 24 under stringent conditions and which encodes a protein having L-amino acid amide hydrolase activity.

10. The process according to Claim 1, wherein the microorganism having the ability to produce a dipeptide is a microorganism carrying a recombinant DNA in which the DNA according to any of [1] to [4] of Claim 4, [1] to [3] of Claim 7 and [1] to [3] of Claim 9 is ligated to a vector DNA.

11. The process according to Claim 10, wherein the microorganism carrying a recombinant DNA is a microorganism belonging to the genus Escherichia, Bacillus, Pseudomonas, Corynebacterium or Saccharomyces.

12. The process according to any of Claims 1 to 11, wherein the amino acid is an amino acid selected from L-amino acids, glycine and β-alanine.

13. The process according to Claim 12, wherein the L-amino acid is one or more kinds of amino acids selected from the group consisting of L-alanine, L-glutamine, L-glutamic acid, L-valine, L-leucine, L-isoleucine, L-proline, L-phenylalanine, L-tryptophan, L-methionine, L-serine, L-threonine, L-cysteine, L-asparagine, L-tyrosine, L-lysine, L-arginine, L-histidine, L-aspartic acid, L-α-aminobutyric acid, L-azaserine, L-theanine, L-4-hydroxyproline, L-3-hydroxyproline, L-ornithine and L-6-diazo-5-oxo-norleucine.

14. The process according to any of Claims 1, 2, 5 to 7, 10 and 11, wherein the amino acid ester is one or more kinds of amino acid esters selected from the group consisting of L-alanine ester, glycine ester, L-valine ester, L-isoleucine ester, L-methionine ester, L-phenylalanine ester, L-serine ester, L-threonine ester, L-glutamine ester, L-tyrosine ester, L-arginine ester, L-aspartic acid-α-ester, L-aspartic acid-β-ester, L-leucine ester, L-asparagine ester, L-lysine ester, L-aspartic acid-α,β-dimethyl ester and L-glutamine-γ-ester, and the amino acid is one or more kinds of amino acids selected from the group consisting of L-glutamic acid, L-glutamine, L-asparagine, glycine, L-alanine, L-leucine, L-methionine, L-proline, L-phenylalanine, L-tryptophan, L-serine, L-threonine, L-tyrosine, L-lysine, L-arginine and L-histidine.

15. The process according to any of Claims 1, 2, 5 and 8 to 11, wherein the amino acid amide is one or more kinds of amino acid amides selected from the group consisting of L-alanine amide, glycine amide and L-aspartic acid-α-amide, and the amino acid is one or more kinds of amino acids selected from the group consisting of L-glutamic acid, L-asparagine, L-glutamine, glycine, L-alanine, L-valine, L-leucine, L-isoleucine, L-methionine, L-proline, L-phenylalanine, L-tryptophan, L-serine, L-threonine, L-tyrosine, L-lysine, L-arginine and L-histidine.

16. The process according to any of Claims 1 to 15, wherein the treated matter of the culture is concentrated culture, dried culture, cells obtained by centrifuging the culture, or a product obtained by subjecting the cells to drying, freeze-drying, treatment with a surfactant, treatment with a solvent, enzymatic treatment or immobilization.

## Patentansprüche

1. Verfahren zur Herstellung eines Dipeptids, umfassend:
Verfügbarmachen einer Enzymquelle und einer oder mehrerer Arten von Substanzen, ausgewählt aus der Gruppe bestehend aus Aminosäureamiden, Aminosäureestern und Aminosäuren, in einem wässrigen Medium, wobei die Enzymquelle eine Kultur eines Mikroorganismus, welcher die Fähigkeit besitzt, ein Dipeptid zu produzieren, oder behandeltes Material aus der Kultur ist, welches einer Hitzebehandlung bei Temperaturen von 50°C bis 65°C für 5 bis 20 Minuten ausgesetzt war, um das Dipeptid im wässrigen Medium zu produzieren und anzureichern, und die Gewinnung des Dipeptids aus dem wässrigen Medium.

2. Verfahren nach Anspruch 1, wobei der Mikroorganismus, welcher die Fähigkeit besitzt ein Dipeptid zu produzieren, ein Mikroorganismus ist, welcher zur Gattung Achromobacter, Acinetobacter, Aeromonas, Agrobacterium, Alcaligenes, Arthrobacter, Beijerinckia, Brevibacterium, Clavibacter, Chryseobacterium, Escherichia, Enterobacter, Erwinia, Flavobacterium, Kluyvera, Microbacterium, Micrococcus, Mycoplana, Pantoea, Propionibacterium, Listonella, Rhizobium, Rhodococcus, Salmonella, Sarcina, Serratia, Staphylococcus, Stenotrophomonas, Streptomyces, Vibrio, Xanthomonas, Bullera, Candida, Cryptococcus, Filobasidium, Geotrichum, Pachysolen, Rhodosporidium, Rhodotorula, Saccharomyces, Sporobolomyces, Tremella, Torulaspora, Torulopsis, Gluconacetobacter, Acetobacter, Gluconobacter, Asaia, Zucharibacter, Actinomadura, Kitasatosporia, Micromonospora, Nocardia, Oerskovia, Saccharothrix, Streptoverticillium, Hafnia, Lactobacillus, Neisseria, Thermoplasma, Corynebacterium, Pseudomonas, Bacillus, Trichosporon oder Sterigmatomyces gehört.

3. Verfahren nach Anspruch 1 oder 2, wobei der Mikroorganismus, welcher die Fähigkeit besitzt, ein Dipeptid zu produzieren, ein Mikroorganismus ist, welcher die Fähigkeit besitzt, ein Protein gemäss einem der nachstehenden Punkte (1) bis (4) zu produzieren:
(1) ein Protein, welches die Aminosäuresequenz, wie in einer der SEQ ID Nrn.: 1 bis 7 und 35 gezeigt, hat;
(2) ein Protein, welches aus einer Aminosäuresequenz besteht, in der ein oder mehrere Aminosäurereste in der Aminosäuresequenz, wie in einer der SEQ ID Nrn.: 1 bis 7 und 35 gezeigt, deletiert, substituiert oder hinzugefügt sind, und die Aktivität besitzt, ein Dipeptid zu synthetisieren;
(3) ein Protein, welches eine Aminosäuresequenz hat, die eine 65%-ige oder höhere Homologie zu der Aminosäuresequenz, wie in einer der SEQ ID Nrn.: 1 bis 7 und 35 gezeigt, aufweist und die Aktivität besitzt, ein Dipeptid zu synthetisieren; und
(4) ein Protein, welches eine Aminosäuresequenz umfasst, welche eine 80%-ige oder höhere Homologie zu der in SEQ ID Nr.: 15 gezeigten Aminosäuresequenz aufweist und die Aktivität besitzt, ein Dipeptid zu synthetisieren.

4. Verfahren nach Anspruch 1 oder 2, wobei der Mikroorganismus, welcher die Fähigkeit besitzt, ein Dipeptid zu produzieren, ein Mikroorganismus ist, welcher DNA gemäss einem der nachstehenden Punkte (1) bis (4) enthält:
(1) DNA, welche das Protein nach einem der Punkte (1) bis (4) in Anspruch 3 codiert;
(2) DNA, welche die Nucleotidsequenz, wie in einer der SEQ ID Nrn.: 8 bis 14, 29 und 30 gezeigt, besitzt;
(3) DNA, welche mit DNA, die eine Nucleotidsequenz, wie in einer der in SEQ ID Nrn.: 8 bis 14, 29 und 30 gezeigt, hat, unter stringenten Bedingungen hybridisiert und ein Protein codiert, das die Aktivität besitzt, ein Dipeptid zu synthetisieren; und
(4) DNA, umfassend eine Nucleotidsequenz, welche eine 80%-ige oder höhere Homologie zu der in SEQ ID Nr.: 16 gezeigten Nucleotidsequenz aufweist und ein Protein codiert, das die Aktivität besitzt, ein Dipeptid zu synthetisieren.

5. Verfahren nach Anspruch 1 oder 2, wobei der Mikroorganismus, welcher die Fähigkeit besitzt, ein Dipeptid zu produzieren, ein Mikroorganismus ist, welcher ein Protein produziert, welches eine Proliniminopeptidase-Aktivität besitzt, oder ein Protein, welches L-Aminosäureamidhydrolase-Akivität besitzt.

6. Verfahren nach Anspruch 5, wobei das Protein, welches Proliniminopeptidase-Aktivität besitzt, ein Protein gemäss einem der nachstehenden Punkte (1) bis (3) ist:
(1) ein Protein, welches die Aminosäuresequenz, wie in einer der SEQ ID Nrn.: 17 bis 19 gezeigt, besitzt;
(2) ein Protein, welches aus einer Aminosäuresequenz besteht, in der ein oder mehrere Aminosäurereste in der Aminosäuresequenz, wie in einer der SEQ ID Nrn.: 17 bis 19 gezeigt, deletiert, substituiert oder hinzugefügt sind, und Proliniminopeptidase-Aktivität besitzt; und
(3) ein Protein, welches aus einer Aminosäuresequenz besteht, welche eine 80%-ige oder höhere Homologie zu der Aminosäuresequenz, wie in einer der SEQ ID Nrn.: 17 bis 19 gezeigt, aufweist und Proliniminopeptidase-Aktivität besitzt.

7. Verfahren nach Anspruch 5, wobei der Mikroorganismus, welcher ein Protein produziert, das Proliniminopeptidase-Aktivität besitzt, ein Mikroorganismus ist, der DNA gemäss einem der nachstehenden Punkte (1) bis (3) enthält:
(1) DNA, welche das Protein gemäss einem der Punkte (1) bis (3) in Anspruch 6 codiert;
(2) DNA, welche die Nucleotidsequenz, wie in einer der SEQ ID Nrn.: 20 bis 22 gezeigt, besitzt; und
(3) DNA, welche mit DNA, die die Nucleotidsequenz, wie in einer der SEQ ID Nrn.: 20 bis 22 gezeigt, hat, unter stringenten Bedingungen hybridisiert und ein Protein codiert, das Proliniminopeptidase-Aktivität besitzt.

8. Verfahren nach Anspruch 5, wobei das Protein, welches L-Aminosäureamidhydrolase-Aktivität besitzt, ein Protein gemäss einem der nachstehenden Punkte (1) bis (3) ist:
(1) Protein, welches die in SEQ ID Nr.: 23 gezeigte Aminosäuresequenz besitzt;
(2) ein Protein, welches aus einer Aminosäuresequenz besteht, in der ein oder mehrere Aminosäurereste in der in der SEQ ID Nr. 23 gezeigten Aminosäuresequenz deletiert, substituiert oder hinzugefügt sind und L-Aminosäureamidhydrolase-Aktivität besitzt; und
(3) ein Protein, welches aus einer Aminosäuresequenz besteht, welche eine 80%-ige oder höhere Homologie zu der in SEQ ID Nr.: 23 gezeigten Aminosäuresequenz aufweist und L-Aminosäureamidhydrolase-Aktivität besitzt.

9. Verfahren nach Anspruch 5, wobei der Mikroorganismus, welcher ein Protein produziert, das L-Aminosäureamidhydrolase-Aktivität besitzt, ein Mikroorganismus ist, welcher DNA nach einem der nachstehenden Punkte (1) bis (3) enthält:
(1) DNA, welche das Protein gemäss einem der Punkte (1) bis (3) in Anspruch 8 codiert;
(2) DNA, welche die Nucleotidsequenz, wie in SEQ ID Nr.: 24 gezeigt, besitzt; und
(3) DNA, welche mit DNA, die die Nucleotidsequenz, wie in einer der SEQ ID Nr.: 24 gezeigt, hat, unter stringenten Bedingungen hybridisiert und welche ein Protein codiert, das L-Aminosäureamidhydrolase-Aktivität besitzt.

10. Verfahren nach Anspruch 1, wobei der Mikroorganismus, welcher die Fähigkeit besitzt, ein Dipeptid zu produzieren, ein Mikroorganismus ist, welcher eine rekombinante DNA enthält, in welcher die DNA nach einem der Punkte (1) bis (4) in Anspruch 4, der Punkte (1) bis (3) in Anspruch 7 und der Punkte (1) bis (3) in Anspruch 9 an eine Vektor-DNA ligiert ist.

11. Verfahren nach Anspruch 10, wobei der Mikroorganismus welcher eine rekombinante DNA enthält, ein Mikroorganismus ist, der zur Gattung Escherichia, Bacillus, Pseudomonas, Corynebacterium oder Saccharomyces gehört.

12. Verfahren nach einem der Ansprüche 1 bis 11, wobei die Aminosäure eine Aminosäure ist, die ausgewählt ist aus L-Aminosäuren, Glycin und β-Alanin.

13. Verfahren nach Anspruch 12, wobei die L-Aminosäure eine oder mehrere Arten von Aminosäuren ist, ausgewählt aus der Gruppe bestehend aus L-Alanin, L-Glutamin, L-Glutaminsäure, L-Valin, L-Leucin, L-Isoleucin, L-Prolin, L-Phenylalanin, L-Tryptophan, L-Methionin, L-Serin, L-Threonin, L-Cystein, L-Asparagin, L-Tyrosin, L-Lysin, L-Arginin, L-Histidin, L-Asparaginsäure, L-α-Aminobuttersäure, L-Azaserin, L-Theanin, L-4-Hydroxyprolin, L-3-Hydroxyprolin, L-Ornithin und L-6-Diazo-5-oxo-Norleucin.

14. Verfahren nach einem der Ansprüche 1, 2, 5 bis 7, 10 und 11, wobei der Aminosäureester eine oder mehrere Arten von Aminosäureestern ist, ausgewählt aus der Gruppe bestehend aus L-Alaninester, Glycinester, L-Valinester, L-Isoleucinester, L-Methioninester, L-Phenylalaninester, L-Serinester, L-Threoninester, L-Glutaminester, L-Tyrosinester, L-Argininester, L-Asparaginsäure-α-ester, L-Asparaginsäure-β-ester, L-Leucinester, L-Asparaginester, L-Lysinester, L-Asparaginsäure-α,β-dimethylester und L-Glutamin-γ-ester, und die Aminosäure eine oder mehrerer Arten von Aminosäuren ist, ausgewählt aus der Gruppe bestehend aus L-Glutaminsäure, L-Glutamin, L-Asparagin, Glycin, L-Alanin, L-Leucin, L-Methionin, L-Prolin, L-Phenylalanin, L-Tryptophan, L-Serin, L-Threonin, L-Tyrosin, L-Lysin, L-Arginin und L-Histidin.

15. Verfahren nach einem der Ansprüche 1, 2, 5 und 8 bis 11, wobei das Aminosäureamid eine oder mehrere Arten von Aminosäureamiden ist, ausgewählt aus der Gruppe bestehend aus L-Alaninamid, Glycinamid and L-Asparaginsäure-a-amid, und die Aminosäure eine oder mehrere Arten von Aminosäuren ist, ausgewählt aus der Gruppe bestehend aus L-Glutaminsäure, L-Asparagin, L-Glutamin, Glycin, L-Alanin, L-Valin, L-Leucin, L-Isoleucin, L-Methionin, L-Prolin, L-Phenylalanin, L-Tryptophan, L-Serin, L-Threonin, L-Tyrosin, L-Lysin, L-Arginin und L-Histidin.

16. Verfahren nach einem der Ansprüche 1 bis 15, wobei das behandelte Material der Kultur eine konzentrierte Kultur, eine getrocknete Kultur, Zellen, welche durch Zentrifugieren der Kultur erhalten wurden, oder ein Produkt, welches durch die Trocknung der Zellen, die Gefriertrocknung der Zellen, die Behandlung der Zellen mit einem Tensid, die Behandlung der Zellen mit einem Lösungsmittel, die enzymatische Behandlung der Zellen oder die Fixierung der Zellen erhalten wurde, ist.

## Revendications

1. -Procédé de production d'un dipeptide, qui comporte :
- le fait de mettre en présence, dans un milieu aqueux, une source d'enzyme et des substances d'un ou plusieurs types choisis parmi les acides aminés, les amides d'acides aminés et les esters d'acides aminés, laquelle source d'enzyme est une culture d'un microorganisme capable de produire un dipeptide ou un produit de traitement d'une telle culture et a subi un traitement de 5 à 20 minutes de chauffage à une température de 50 à 65 °C, de manière à ce que du dipeptide soit produit et s'accumule dans le milieu aqueux ;
- et le fait de récupérer ce dipeptide dans le milieu aqueux.

2. Procédé conforme à la revendication 1, dans lequel le microorganisme capable de produire un dipeptide est un microorganisme appartenant au genre Achromobacter, Acinetobacter, Aeromonas, Agrobacterium, Alcaligenes, Arthrobacter, Beijerinckia, Brevibacterium, Clavibacter, Chryseobacterium, Escherichia, Enterobacter, Erwinia, Flavobacterium, Kluyvera, Microbacterium, Micrococcus, Mycoplana, Pantoea, Propionibacterium, Listonella, Rhizobium, Rhodococcus, Salmonella, Sarcina, Serratia, Staphylococcus, Stenotrophomonas, Streptomyces, Vibrio, Xanthomonas, Bullera, Candida, Cryptococcus, Filobasidium, Geotrichum, Pachysolen, Rhodosporidium, Rhodotorula, Saccharomyces, Sporobolomyces, Tremella, Torulaspora, Torulopsis, Gluconacetobacter, Acetobacter, Gluconobacter, Asaia, Zucharibacter, Actinomadura, Kitasatosporia, Micromonospora, Nocardia, Oerskovia, Saccharothrix, Streptoverticillium, Hafnia, Lactobacillus, Neisseria, Thermoplasma, Corynebacterium, Pseudomonas, Bacillus, Trichosporon ou Sterigmatomyces.

3. Procédé conforme à la revendication 1 ou 2, dans lequel le microorganisme capable de produire un dipeptide est un microorganisme capable de produire une protéine décrite ci-dessous dans l'un des items [1] à [4] :
[1] une protéine dont la séquence d'acides aminés est l'une de celles présentées en tant que Séquences N° 1 à 7 et 35 ;
[2] une protéine dont la séquence d'acides aminés est l'une de celles présentées en tant que Séquences N° 1 à 7 et 35 où un ou plusieurs résidus d'acides aminés ont été retranchés, remplacés ou ajoutés, et qui possède une activité de synthèse de dipeptide ;
[3] une protéine dont la séquence d'acides aminés est homologue, pour au moins 65 %, à l'une de celles présentées en tant que Séquences N° 1 à 7 et 35, et qui possède une activité de synthèse de dipeptide;
[4] une protéine qui comprend une séquence d'acides aminés homologue, pour au moins 80 %, à celle présentée en tant que Séquence N° 15, et qui possède une activité de synthèse de dipeptide.

4. Procédé conforme à la revendication 1 ou 2, dans lequel le microorganisme capable de produire un dipeptide est un microorganisme qui héberge un ADN décrit ci-dessous dans l'un des items [1] à [4] :
[1] un ADN codant une protéine décrite dans l'un des items [1] à [4] de la revendication 3 ;
[2] un ADN dont la séquence de nucléotides est l'une de celles présentées en tant que Séquences N° 8 à 14, 29 et 30 ;
[3] un ADN qui s'hybride avec un ADN ayant l'une des séquences de nucléotides présentées en tant que Séquences N° 8 à 14, 29 et 30, dans des conditions stringentes, et qui code une protéine possédant une activité de synthèse de dipeptide ;
[4] un ADN qui comprend une séquence de nucléotides homologue, pour au moins 80 %, à celle présentée en tant que Séquence N° 16, et qui code une protéine possédant une activité de synthèse de dipeptide.

5. Procédé conforme à la revendication 1 ou 2, dans lequel le microorganisme capable de produire un dipeptide est un microorganisme qui produit une protéine possédant une activité de proline-iminopeptidase ou une protéine possédant une activité d'hydrolase pour les amides d'acides L-aminés.

6. Procédé conforme à la revendication 5, dans lequel la protéine possédant une activité de proline-iminopeptidase est une protéine décrite ci-dessous dans l'un des items [1] à [3] :
[1] une protéine dont la séquence d'acides aminés est l'une de celles présentées en tant que Séquences N° 17 à 19 ;
[2] une protéine dont la séquence d'acides aminés est l'une de celles présentées en tant que Séquences N° 17 à 19 où un ou plusieurs résidus d'acides aminés ont été retranchés, remplacés ou ajoutés, et qui possède une activité de proline-iminopeptidase ;
[3] une protéine dont la séquence d'acides aminés est homologue, pour au moins 80 %, à l'une de celles présentées en tant que Séquences N° 17 à 19, et qui possède une activité de proline-iminopeptidase.

7. Procédé conforme à la revendication 5, dans lequel le microorganisme qui produit une protéine possédant une activité de proline-iminopeptidase est un microorganisme qui héberge un ADN décrit ci-dessous dans l'un des items [1] à [3] :
[1] un ADN codant une protéine décrite dans l'un des items [1] à [3] de la revendication 6 ;
[2] un ADN dont la séquence de nucléotides est l'une de celles présentées en tant que Séquences N° 20 à 22 ;
[3] un ADN qui s'hybride avec un ADN ayant l'une des séquences de nucléotides présentées en tant que Séquences N° 20 à 22, dans des conditions stringentes, et qui code une protéine possédant une activité de proline-iminopeptidase.

8. Procédé conforme à la revendication 5, dans lequel la protéine possédant une activité d'hydrolase pour les amides d'acides L-aminés est une protéine décrite ci-dessous dans l'un des items [1] à [3] :
[1] une protéine dont la séquence d'acides aminés est celle présentée en tant que Séquence N° 23 ;
[2] une protéine dont la séquence d'acides aminés est celle présentée en tant que Séquence N° 23 où un ou plusieurs résidus d'acides aminés ont été retranchés, remplacés ou ajoutés, et qui possède une activité d'hydrolase pour les amides d'acides L-aminés ;
[3] une protéine dont la séquence d'acides aminés est homologue, pour au moins 80 %, à celle présentée en tant que Séquence N° 23, et qui possède une activité d'hydrolase pour les amides d'acides L-aminés.

9. Procédé conforme à la revendication 5, dans lequel le microorganisme qui produit une protéine possédant une activité d'hydrolase pour les amides d'acides L-aminés est un microorganisme qui héberge un ADN décrit ci-dessous dans l'un des items [1] à [3] :
[1] un ADN codant une protéine décrite dans l'un des items [1] à [3] de la revendication 8 ;
[2] un ADN dont la séquence de nucléotides est celle présentée en tant que Séquence N° 24 ;
[3] un ADN qui s'hybride avec un ADN ayant la séquence de nucléotides présentée en tant que Séquence N° 24, dans des conditions stringentes, et qui code une protéine possédant une activité d'hydrolase pour les amides d'acides L-aminés.

10. Procédé conforme à la revendication 1, dans lequel le microorganisme capable de produire un dipeptide est un microorganisme qui héberge un ADN recombiné dans lequel un ADN décrit dans l'un des items [1] à [4] de la revendication 4, [1] à [3] de la revendication 7 et [1] à [3] de la revendication 9 est ligaturé à un ADN vecteur.

11. Procédé conforme à la revendication 10, dans lequel le microorganisme hébergeant un ADN recombiné est un microorganisme qui appartient au genre Escherichia, Bacillus, Pseudomonas, Corynebacterium ou Saccharomyces.

12. Procédé conforme à l'une des revendications 1 à 11, dans lequel l'acide aminé est un acide aminé choisi parmi les acides L-aminés, la glycine et la β-alanine.

13. Procédé conforme à la revendication 12, dans lequel l'acide Laminé est un ou plusieurs acides aminés choisi(s) dans l'ensemble formé par les suivants : L-alanine, L-glutamine, acide L-glutamique, L-valine, L-leucine, L-isoleucine, L-proline, L-phénylalanine, L-tryptophane, L-méthionine, L-sérine, L-thréonine, L-cystéine, L-asparagine, L-tyrosine, L-lysine, L-arginine, L-histidine, acide L-aspartique, acide L-α-aminobutyrique, L-azasérine, L-théanine, L-4-hydroxyproline, L-3-hydroxyproline, L-ornithine et L-6-diazo-5-oxo-norleucine.

14. Procédé conforme à l'une des revendication 1, 2, 5 à 7, 10 et 11, dans lequel l'ester d'acide aminé est un ou plusieurs esters d'acide aminé choisi(s) dans l'ensemble formé par les suivants : ester de L-alanine, ester de glycine, ester de L-valine, ester de L-isoleucine, ester de L-méthionine, ester de L-phénylalanine, ester de L-sérine, ester de L-thréonine, ester de L-glutamine, ester de L-tyrosine, ester de L-arginine, α-ester d'acide L-aspartique, β-ester d'acide L-aspartique, ester de L-leucine, ester de L-asparagine, ester de L-lysine, α,β-diester diméthylique d'acide L-aspartique et γ-ester de L-glutamine, et l'acide aminé est un ou plusieurs acides aminés choisi(s) dans l'ensemble formé par les suivants : acide L-glutamique, L-glutamine, L-asparagine, glycine, L-alanine, L-leucine, L-méthionine, L-proline, L-phénylalanine, L-tryptophane, L-sérine, L-thréonine, L-tyrosine, L-lysine, L-arginine et L-histidine.

15. Procédé conforme à l'une des revendication 1, 2, 5 et 8 à 11, dans lequel l'amide d'acide aminé est un ou plusieurs amides d'acide aminé choisi(s) dans l'ensemble formé par l'amide de L-alanine, l'amide de glycine et l'α-amide d'acide L-aspartique, et l'acide aminé est un ou plusieurs acides aminés choisi(s) dans l'ensemble formé par les suivants : acide L-glutamique, L-glutamine, L-asparagine, glycine, L-alanine, L-valine, L-leucine, L-isoleucine, L-méthionine, L-proline, L-phénylalanine, L-tryptophane, L-sérine, L-thréonine, L-tyrosine, L-lysine, L-arginine et L-histidine.

16. Procédé conforme à l'une des revendications 1 à 15, dans lequel le produit de traitement de culture est une culture qui a été concentrée ou qui a été séchée, ou des cellules obtenues par centrifugation de la culture, ou encore un produit qu'on a obtenu en faisant sécher les cellules, en les lyophilisant, en les traitant avec un tensioactif ou avec un solvant, en leur faisant subir un traitement enzymatique ou en les immobilisant.
